(19) Europäisches Patentamt
European Patent Office
Office européen des brevets

(11) EP 4 144 732 A1

(12) EUROPEAN PATENT APPLICATION
published in accordance with Art. 153(4) EPC

(43) Date of publication:
**08.03.2023 Bulletin 2023/10**

(21) Application number: **20933967.0**

(22) Date of filing: **10.11.2020**

(51) International Patent Classification (IPC):
***C07D 417/14*** (2006.01) ***C07D 215/12*** (2006.01)
***C07D 215/02*** (2006.01) ***C07D 239/72*** (2006.01)
***C07D 277/62*** (2006.01) ***A61K 31/517*** (2006.01)
***A61P 35/00*** (2006.01)

(52) Cooperative Patent Classification (CPC):
**A61K 31/496; A61K 31/517; A61P 35/00; A61P 35/02; C07D 215/02; C07D 215/12; C07D 239/72; C07D 277/62; C07D 417/04; C07D 417/14;** Y02P 20/55

(86) International application number:
**PCT/CN2020/127861**

(87) International publication number:
**WO 2021/218110 (04.11.2021 Gazette 2021/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.04.2020 CN 202010358615**

(71) Applicants:
- **Shanghai Ringene BioPharma Co., Ltd.**
  **Shanghai 201210 (CN)**
- **Shanghai Ringene Therapeutics Co. Ltd**
  **Shanghai 201203 (CN)**

(72) Inventors:
- **WAN, Huixin**
  **Shanghai 201210 (CN)**
- **ZHA, Chuantao**
  **Shanghai 201210 (CN)**
- **MA, Jingui**
  **Shanghai 201210 (CN)**
- **SHEN, Jingkang**
  **Shanghai 201210 (CN)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

# (54) BENZOTHIAZOLYL BIARYL COMPOUND, AND PREPARATION METHOD AND USE

(57) A benzothiazolyl biaryl compound represented by general formula I, or a pharmaceutically acceptable salt thereof, or an enantiomer, a diastereoisomer, a tautomer, a torsional isomer, a solvate, a polymorph, or a prodrug thereof, a preparation method therefor, and a use thereof as a $KRAS^{G12C}$ inhibitor.

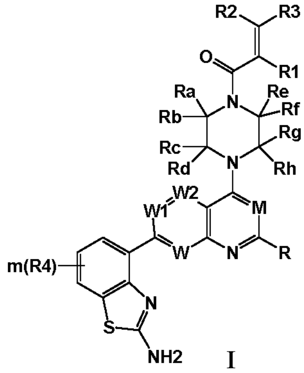

I

EP 4 144 732 A1

## Description

This application requires the priority of the Chinese patent application 202010358615.7 with the application date of April 29, 2020.This application refers to the full text of the above-mentioned Chinese patent application.

## Technical field

The invention belongs to the field of pharmaceutical chemistry, in particular, relates to a class of benzothiazolyl biaryl compounds, compounds with inhibition of mutant Ras protein activity, preparation methods and uses thereof.

## Background

RAS is the first oncogene identified in human tumors and was first identified in two murine sarcoma viruses.There are three members of the RAS gene family, namely Hras, Kras, and Nras. In human tumors, Kras mutation is the most common, accounting for about 85%.Previous studies have shown that the Kras mutation can cause cancer because of the missense mutation in codon 12, which changes the structure of the Kras protein and keeps it activated. The role of Ras in the transmission of signaling pathway is mainly to activate kinases that control gene transcription, thereby regulating cell differentiation and proliferation, and is closely related to the survival, proliferation, migration, metastasis and angiogenesis of tumor cells. According to statistics, $Kras^{G12C}$mutation exists in 11%-16% lung adenocarcinoma cases, and Kras mutationalso causesa part of pancreatic cancer, colorectal cancer, ovarian cancer and cholangiocarcinoma.However, more than 30 years have passed since the first discovery of Kras oncogenes. Targeted drugs for common proto-oncogenes such as EGFR and BCL have gone through several generations, but targeted drugs for Kras have not yet been successfully developed. For a long time, targeted drugs for KRas pathway mutant tumors mainly focus on farinyltransferase inhibitors and Raf-MEK pathway inhibitors, but receivedlittle effect. In recent years, the research and development of inhibitors targeted KRas specific gene mutations have become a hot spot. Some inhibitors have gradually moved from preclinical incubation to clinical research, such as $KRas^{G12C}$ inhibitors AMG510, MRTX1257, etc., and have shown certain curative effects in early clinical experiments. The first clinical data of the world's first $KRAS^{G12C}$inhibitor AMG 510 was finally officially released by the American Society of Clinical Oncology in June 2019. In this clinical study, Amgen AMG 510 showed that it can prevent Tumor growth in most non-small cell lung cancer and colorectal cancer patients with KRas mutations. Therefore, it has become a hot spot in the industry to discover and find targeted drugs with high specificity and excellent druggability for KRas specific mutant genes.

## SUMMARY OF THE INVENTION

The technical problem to be solved by the present invention is to overcome the problem of the lack of $KRAS^{G12C}$ inhibitors in the prior art, and to provide a class of benzothiazolyl biaryl compounds, preparation methods and uses thereof. The benzothiazolyl biaryl compounds provided by the present invention are a new class of $KRAS^{G12C}$ inhibitors that exhibit good inhibitory activity; they have good inhibitory activity against tumor cells and have good drug-forming properties, and have broad prospects for drug development.

The present invention solves the above-mentioned technical problems by the following technical solutions.

The invention provides a benzothiazolyl biaryl compound having fomula I, or a pharmaceutically acceptable salt thereof, or an enantiomer, a diastereoisomer, a tautomer, a torsional isomer, a solvate, a polymorph or a prodrug thereof,

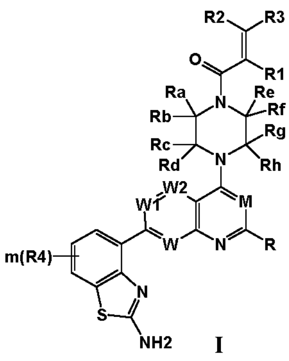

I

wherein M is N or CR5;

When M is N, R is independently halogen, cyano, hydroxyl, nitro, amino, $C_1$-$C_6$alkyl, $C_1$-$C_6$alkyl-S-, $C_1$-$C_6$alkyl-SO-, $C_1$-$C_6$alkyl-$SO_2$-, $C_1$-$C_6$haloalkyl, $C_1$-$C_6$alkyl-O-, $C_1$-$C_6$alkyl-NR'R"-, 3-12-membered cycloalkyl, 3-12-membered heterocycloalkyl, 3-12-membered cycloalkyl-O-, 3-12-membered cycloalkyl-NR'R"-, 3-12-membered heterocycloalkyl-O-, 3-12-membered heterocycloalkyl-NR'R"-, Q1-($C_1$-$C_4$alkylene)-O-, Q2-($C_1$-$C_4$alkylene)-NR'-, NR'R"-($C_1$-$C_4$alkylene)-O-, NR'R"-CO-($C_1$-$C_4$alkylene)-NR'-, NR'R"-Q3-, 5-12-membered aryl or 5-12-membered heteroaryl;

When M is CR5, R is independently hydrogen, halogen, cyano, hydroxyl, nitro, amino, $C_1$-$C_6$alkyl, $C_1$-$C_6$alkyl-S-, $C_1$-$C_6$alkyl-SO-, $C_1$-$C_6$alkyl-$SO_2$-, $C_1$-$C_6$haloalkyl, $C_1$-$C_6$alkyl-O-, $C_1$-$C_6$alkyl-NR'R "-,3-12-membered cycloalkyl, 3-12-membered heterocycloalkyl, 3-12-membered cycloalkyl-O-, 3-12-membered cycloalkyl-NR'R'-, 3-12-membered heterocycloalkyl-O-, 3-12-membered heterocycloalkyl-NR'R"-, Q4-($C_1$-$C_4$alkylene)-O-, QS-($C_1$-$C_4$alkylene)-NR'-, NR'R"-($C_1$-$C_4$alkylene)-O-, NR'R"-CO-($C_1$-$C_4$alkylene)-NR'-, NR'R"-Q6-, 5-12 membered aryl or 5-12 membered heteroaryl;

Q1, Q2, Q3, Q4, Q5 and Q6 are independently 3-7-membered heterocycloalkyl or 3-7-membered heterocycloalkyl substituted by one or more Rq; when there are multiple substituents, the same or different;

Rq is independently halogen, cyano, hydroxyl, amino, or $C_1$-$C_4$alkyl;

R5 is independently hydrogen, halogen, cyano, hydroxyl, nitro, amino, $C_1$-$C_6$alkyl, $C_1$-$C_6$alkyl-S-, $C_1$-$C_6$alkyl-SO-, $C_1$-$C_6$alkyl-$SO_2$-, $C_1$-$C_6$haloalkyl, $C_1$-$C_6$alkyl-O-, $C_1$-$C_6$alkyl-NR'R"-, 3-12-membered cycloalkyl, 3-12-membered heterocycloalkyl, 3-12-membered cycloalkyl-O-, 3-12-membered cycloalkyl-NR'R"-, 3-12-membered heterocycloalkyl-O-, 3-12-membered heterocycloalkyl-NR'R"-, 5-12-membered aryl or 5-12-membered heteroaryl;

R' and R" are independently hydrogen, $C_1$-$C_6$alkyl, 3-12-membered cycloalkyl, 3-12-membered heterocycloalkyl, 5-12-membered aryl or heteroaryl;

R1 is independently hydrogen, halogen, cyano, nitro, $C_1$-$C_6$alkyl, $C_1$-$C_6$alkyl-$SO_2$-, $C_1$-$C_6$alkyl-SO-, or $C_1$-$C_6$haloalkyl;

R2, R3 are independently hydrogen, halogen, cyano, nitro, $C_1$-$C_6$alkyl, $C_1$-$C_6$alkyl-$SO_2$-, $C_1$-$C_6$alkyl-SO-, $N(R_{2a})(R_{2b})$-$(CH_2)$x-;

$R_{2a}$ and $R_{2b}$ are each independently hydrogen or $C_1$-$C_6$alkyl, x is selected from any integer inbetween 0-5; the above-mentioned alkyl can be further substituted by deuterium, halogen, substituted or unsubstituted amino/cyclic amine group;

or, $R_{2a}$ and $R_{2b}$ together form a 5-10 membered nitrogen-containing heterocycloalkyl substituted by $C_1$-$C_6$alkyl;

R4 is independently hydrogen, halogen, cyano, nitro, $C_1$-$C_6$alkyl, $C_1$-$C_6$alkyl-$SO_2$-, $C_1$-$C_6$alkyl-SO-, $C_1$-$C_6$haloalkyl, or $C_1$-$C_6$alkyl-O-;

m is an integer independently selected from0-4;

W,W1,W2 are independently CR6 or N;

R6 is independently H, halogen, cyano, $C_1$-$C_6$alkyl, Ci-Cealkyl-O-, $C_1$-$C_6$haloalkyl, Ci-Cehaloalkyl-O-, hydroxyl substituted $C_1$-$C_6$alkyl, cyano substituted $C_1$-$C_6$alkyl, $C_2$-$C_6$alkenyl, $C_2$-$C_6$alkynyl, 3-8 membered cycloalkyl or 3-8-membered heterocycloalkyl;

Ra, Rb, Rc, Rd, Re, Rf, Rg, Rh are independently hydrogen, halogen, $C_1$-$C_6$alkyl, $C_1$-$C_6$alkyl-O-, $C_1$-$C_6$haloalkyl, cyano substituted $C_1$-$C_6$alkyl;

or Ra, Rb, Rc, Rd, Re, Rf, Rg and Rh form a saturated or partially unsaturated 3-8-membered ring system between the two;

or either Rg or Rh can form Cy together with M and the attached moiety; Cy is a saturated or partially unsaturated or unsaturated 3-10-membered ring system or a saturated or partially unsaturated or unsaturated 3-10-membered ring system substituted by one or more Rp; when there are multiple substituents, the same or different;

Rp is independently halogen, cyano, hydroxyl, amino, $C_1$-$C_4$alkyl; or, when two Rp are located on the same C-atom, they jointly form =O;

one or more hydrogen atoms on any of the abovementioned groups can be substituted by Rr substituents selected from the group consisting of: deuterium, halogen, hydroxyl, amino or cycloamino, cyano, nitro, sulfone or sulfoxide, $C_1$-$C_8$alkyl, 3-8-membered cycloalkyl or heterocycloalkyl, $C_1$-$C_8$alkoxy, $C_1$-$C_8$alkylamino, $C_2$-$C_6$alkenyl, $C_2$-$C_6$alkynyl, acyl or sulfonyl, urea or sulfonylurea, 5~8-membered aryl or heteroaryl; or when two Rr are located on the same C-atom, they jointly form =O;

the heteroaryl comprises 1-3 heteroatoms selected from the group consisting of: N, O, P and S; the heterocycloalkyl comprises 1-3 heteroatoms selected from the group consisting of: N, O, P and S;

the ring system comprises saturated or partially unsaturated ring system such as a spiro-ring, a bridged ring, a fused-ring and a fused ring.

In certain preferred embodiments of the present invention, certain groups in the benzothiazolyl biaryl compound as shown in Formula I, or a pharmaceutically acceptable salt thereof, or an enantiomer, a diastereoisomer, a tautomer, a torsional isomer, a solvate, a polymorph, or a prodrug thereof are defined as follows, the unmentioned groupsare as

described in any of the aspects of the present application (hereinafter referred to as in anembodiment of the present invention).

In an embodiment of the present invention,when M is N, R is halogen (such as chlorine), cyano, amino, $C_1$-$C_6$alkyl, $C_1$-$C_6$alkyl-O-, $C_1$-$C_6$alkylamino, NR'R"-($C_1$-$C_4$alkylene)-O-, NR'R"-CO-($C_1$-$C_4$alkylene)-NR'-, Q1-($C_1$-$C_4$alkylene)-O-, Q2-($C_1$-$C_4$alkylene)-NR'-, NR'R"-Q3-;

for example, R is halogen (e. g. chlorine), NR'R"-($C_1$-$C_4$alkylene)-O-, NR'R"-CO-($C_1$-$C_4$alkylene)-NR'-, Q1-($C_1$-$C_4$alkylene)-O-, Q2-($C_1$-$C_4$alkylene)-NR'-, NR'R"-Q3-;
foranother example, R is NR'R"-($C_1$-$C_4$alkylene)-O-, NR'R"-CO-($C_1$-$C_4$alkylene)-NR'-, Q1-($C_1$-$C_4$alkylene)-O-, Q2-($C_1$-$C_4$alkylene)-NR'-, NR'R"-Q3-;
for another example, R is NR'R"-($C_1$-$C_4$alkylene)-O-, Q1-($C_1$-$C_4$alkylene)-O-, Q2-($C_1$-$C_4$alkylene)-NR'-, NR'R"-Q3-;
also, for example, R is NR'R"-($C_1$-$C_4$alkylene)-O-, Q1-($C_1$-$C_4$alkylene)-O-, NR'R"-Q3-.

In an embodiment of the present invention,When M is CR5, R is hydrogen, halogen (such as chlorine), cyano, amino, $C_1$-$C_6$alkyl, $C_1$-$C_6$alkyl-O-, $C_1$-$C_6$alkylamino, NR'R"-($C_1$-$C_4$alkylene)-O-, NR'R"-CO-($C_1$-$C_4$alkylene)-NR'-, Q4-($C_1$-$C_4$alkylene)-O-, QS-($C_1$-$C_4$alkylene)-NR'-, NR'R"-Q6-;

forexample, R is hydrogen, halogen (e. g. chlorine), NR'R"-($C_1$-$C_4$alkylene)-O-, NR'R"-CO-($C_1$-$C_4$alkylene)-NR'-, Q4-($C_1$-$C_4$alkylene)-O-, QS-($C_1$-$C_4$alkylene)-NR'-, NR'R"-Q6-;
foranother example, R is NR'R"-($C_1$-$C_4$alkylene)-O-, NR'R"-CO-($C_1$-$C_4$alkylene)-NR'-, Q4-($C_1$-$C_4$alkylene)-O-, QS-($C_1$-$C_4$alkylene)-NR'-, NR'R"-Q6-.

In an embodiment of the present invention,Q1, Q2, Q3, Q4, Q5 and Q6 are independently 3-7-membered heterocycloalkyl substituted by one or more Rq;
for example, a 4-5-membered heterocycloalkyl substituted by one or more Rq; for another example, the heterocycloalkyl comprises one N.

In an embodiment of the present invention,Rq is independently halogen, hydroxyl, or $C_1$-$C_4$alkylgroup (e. g. methyl group).

In an embodiment of the present invention,R5 is independently cyano.

In an embodiment of the present invention,R' and R" are independently hydrogen, or $C_1$-$C_6$alkyl.

In an embodiment of the present invention, R1 is hydrogen, halogen (e. g., fluorine), cyano, or $C_1$-$C_6$alkyl (e. g., methyl); for example, hydrogen, halogen (e. g., fluorine); and also, for example, hydrogen.

In an embodiment of the present invention, R2 and R3 are independently hydrogen, $C_1$-$C_6$alkyl (e. g. methyl), halogen (e. g. fluorine); for example, hydrogen.

In an embodiment of the present invention, R4 is halogen; such as fluorine.

In an embodiment of the present invention, m is 0, 1 or 2; for example, 1.

In an embodiment of the present invention, W is independently CR6; for example, R6 is halogen (e. g. chlorine, fluorine).

In an embodiment of the present invention, W1 is independently CR6; for example, R6 is hydrogen.

In an embodiment of the present invention, W2 is independently CR6; for example, R6 is halogen (e. g. chlorine, fluorine).

In an embodiment of the present invention, R6 is hydrogen, halogen (e. g. chlorine, fluorine), cyano, $C_1$-$C_6$alkyl (e. g. methyl), $C_1$-$C_6$alkyl-O-(e. g. methyl-O-), hydroxyl substituted $C_1$-$C_6$alkyl (e. g. hydroxymethyl, hydroxyethyl), cyano substituted $C_1$-$C_6$alkyl (e. g. cyanomethyl), 3-8-membered cycloalkyl (e. g. cyclopropyl)), $C_2$-$C_6$alkenyl, $C_2$-$C_6$alkynyl, $C_1$-$C_6$haloalkyl, $C_1$-$C_6$haloalkyl-O-(e. g.-$OCH_2CHF_2$,-$OCH_2CF_3$);

for example, R6 is hydrogen, halogen (e. g. chlorine, fluorine), $C_1$-$C_4$alkyl (e. g. methyl), $C_1$-$C_4$haloalkyl-O-(e. g.-$OCH_2CHF_2$,-$OCH_2CF_3$);
for another example, R is hydrogen orhalogen (e. g. chlorine, fluorine).

In an embodiment of the present invention, W is CR6 or N;R6 is halogen (e. g. chlorine, fluorine), $C_1$-$C_4$alkyl (e. g. methyl), $C_1$-$C_4$haloalkyl-O-(e. g.-$OCH_2CHF_2$,-$OCH_2CF_3$);
for example, W is N,C(F),C(Cl),C(Me),C(OMe),C($OCH_2CHF_2$),C($OCH_2CF_3$); another example is C(F).

In an embodiment of the present invention, W2 is CR6 or N;R6 is halogen (e. g. chlorine, fluorine), $C_1$-$C_4$alkyl (e. g. methyl), $C_1$-$C_4$alkyl-O-(e. g. methyl-O-);
for example, W2 is N or CH, C(F), C(Cl), C(Me), C(OMe) ; another example is CH.

In an embodiment of the present invention,W1 is CR6; R6 is hydrogen, halogen (e. g. chlorine, fluorine), cyano, $C_1$-$C_4$alkyl (e. g. methyl), $C_1$-$C_4$alkyl-O-(e. g. methyl-O-), hydroxyl substituted $C_1$-$C_4$alkyl (e. g. hydroxymethyl, hydrox-

yethyl), cyano substituted $C_1$-$C_4$alkyl (e. g. cyanomethyl), 3-8-membered cycloalkyl (e. g. cyclopropyl)), $C_2$-$C_4$alkenyl, $C_2$-$C_4$alkynyl, $C_1$-$C_4$haloalkyl, $C_1$-$C_4$haloalkyl-O-(e. g.-$OCH_2CHF_2$,-$OCH_2CF_3$);
for example, W1 is CH, C (halogen), C(cyano),-C (cyclopropyl), C($C_1$-$C_4$alkyl), C($C_1$-$C_4$alkyl-O-), C($C_2$-$C_4$alkenyl), C($C_2$-$C_4$alkynyl), C($C_1$-$C_4$haloalkyl), C($C_1$-$C_4$haloalkyl-O-); another example is C(Cl).

In an embodiment of the present invention,Ra, Rb, Rc, Rd, Re, Rf, Rg and Rh are independently hydrogen, halogen (e. g. chlorine, fluorine), $C_1$-$C_6$alkyl (e. g. methyl), cyano substituted $C_1$-$C_6$alkyl (e. g. cyanomethyl), hydroxyl substituted $C_1$-$C_6$alkyl (e. g. hydroxymethyl, hydroxyethyl);
for example, hydrogen, Ci-Cealkyl (e. g. methyl), cyano substituted $C_1$-$C_6$alkyl (e. g. cyanomethyl).

In an embodiment of the present invention,any one of Ra, Rb, Re and Rf is hydrogen and the other is hydrogen, C1-C6 alkyl (e.g., methyl), or cyano-substituted C1-C6 alkyl (e.g., cyanomethyl).

In an embodiment of the present invention,any one of Rc, Rd, Rg and Rh is hydrogen, and the other is hydrogen, or C1-C6 alkyl.

In a embodiment of the present invention,any one of Ra, Rb, Rc, Rd, Re, Rf, Rg and Rh is hydrogen, C1-C6 alkyl (e.g., methyl), or cyano-substituted C1-C6 alkyl (e.g., cyanomethyl), and the rest of Ra, Rb, Rc, Rd, Re, Rf, Rg, Rh are hydrogen;

for example, any one of Ra, Rb, Re and Rf is hydrogen, C1-C6 alkyl (e.g., methyl), or cyano-substituted C1-C6 alkyl (e.g., cyanomethyl), and the rest of Ra, Rb, Rc, Rd, Re, Rf, Rg and Rh are hydrogen;
or, any one of Rc, Rd, Rg and Rh is hydrogen, or methyl, and the rest of Ra, Rb, Rc, Rd, Re, Rf, Rg and Rh are hydrogen.

In an embodiment of the present invention,Cy is a 3-10-membered partially unsaturated ring system or a 3-10-membered partially unsaturated ring system substituted by one or more Rp; the ring system may be a saturated or partially unsaturated ring system such as a monocyclic, or a fused ring;
for example, a 5-6-membered partially unsaturated ring system substituted by one or more Rp; the ring system is a partially unsaturated ring system such as a monocyclic, or a fused ring.

In an embodiment of the present invention,Rp is independently $C_1$-$C_4$alkyl (e. g. methyl); or, when two Rp are located on the same C-atom, they jointly form = O.

In an embodiment of the present invention, when R is halogen, or $C_1$-$C_6$haloalkyl group, the halogen or the halogenin $C_1$-$C_6$haloalkyl group is independently fluorine, chlorine, or bromine; for example, fluorine or chlorine.

In an embodiment of the present invention, when R is $C_1$-$C_6$alkyl, $C_1$-$C_6$alkyl-S-, $C_1$-$C_6$alkyl-SO-, $C_1$-$C_6$alkyl-$SO_2$-, $C_1$-$C_6$haloalkyl, $C_1$-$C_6$alkyl-O- or $C_1$-$C_6$alkyl-NR'R"-, the $C_1$-$C_6$alkyl (e. g. methyl, ethyl, propyl, butyl, pentyl or hexyl) in the $C_1$-$C_6$alkyl, $C_1$-$C_6$alkyl-S-, $C_1$-$C_6$alkyl-SO-, $C_1$-$C_6$alkyl-$SO_2$-, $C_1$-$C_6$haloalkyl, $C_1$-$C_6$alkyl-O- and $C_1$-$C_6$alkyl-NR'R"- are independently $C_1$-$C_4$alkyl, for example, methyl, ethyl, n-propyl, isopropyl, n-butyl, isobutyl, sec-butyl or tert-butyl); another example is methyl.

In an embodiment of the present invention,when R is Q1-($C_1$-$C_4$alkylene)-O-, Q2-($C_1$-$C_4$alkylene)-NR'-, Q4-($C_1$-$C_4$alkylene)-O-, QS-($C_1$-$C_4$alkylene)-NR'-, NR'R"-($C_1$-$C_4$alkylene)-O-, or NR'R"-CO-($C_1$-$C_4$alkylene)-NR'-, the $C_1$-$C_4$alkylene (such as methylene, ethylene, propylene or butylene) in the Q1-($C_1$-$C_4$alkylene)-O-, Q2-($C_1$-$C_4$alkylene)-NR'-, Q4-($C_1$-$C_4$alkylene)-O-, QS-($C_1$-$C_4$alkylene)-NR'-, NR'R"-($C_1$-$C_4$alkylene)-O- andNR'R"-CO-($C_1$-$C_4$alkylene)-NR'- are independently methylene (-$CH_2$-), 1, 2-ethylene (-$CH_2CH_2$-), 2,2-ethylene (-$CH(CH_3)$-), 2,3-propylidene (-$CH(CH_3)CH_2$-), isopropylidene (-$C(CH_3)_2$-), 1,3-propylidene (-$CH_2CH_2CH_2$-), butylidene (-$CH_2CH_2CH_2CH_2$-); another example is methylene.

In an embodiment of the present invention,when Q1, Q2, Q3, Q4, Q5 and Q6 are independently 3-7-membered heterocycloalkyl or 3-7-membered heterocycloalkyl substituted by one or more Rq, the 3-7-membered heterocycloalkyl in the 3-7-membered heterocycloalkyl and 3-7-membered heterocycloalkyl substituted by one or more Rq is a 4-6-membered heterocycloalkyl, and the heteroatom is selected from N, O and S, the number of heteroatoms is 1, 2, or 3; for example, in a 4-5-membered heterocycloalkyl, the heteroatom is N, and the number of heteroatoms is 1; for example, the 3-7-membered heterocycloalkyl is

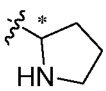 , 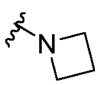 ;

another example is

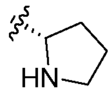 or 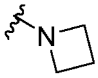.

In an embodiment of the present invention, when Rq is independently halogen, the halogen is independently fluorine, chlorine or bromine; for example, fluorine, or chlorine.

In an embodiment of the present invention, when Rq is independently $C_1$-$C_4$alkyl, the $C_1$-$C_4$alkylis independently methyl, ethyl, n-propyl, isopropyl, n-butyl, isobutyl, sec-butyl or tert-butyl; also for example, methyl.

In an embodiment of the present invention, when R' and R " are independently $C_1$-$C_6$alkyl, the $C_1$-$C_6$ alkyl (e. g., methyl, ethyl, propyl, butyl, pentyl or hexyl) is independently $C_1$-$C_4$alkyl group, such as methyl, ethyl, n-propyl, isopropyl, n-butyl, isobutyl, sec-butyl or tert-butyl; also for example methyl.

In an embodiment of the present invention, when R1, R2, R3, R4 and R5 are independently halogen, the halogen is independently fluorine, chlorine or bromine; for example, fluorine, or chlorine.

In an embodiment of the present invention, when R1, R2, R3, R4 and R5 are independently $C_1$-$C_6$alkyl, $C_1$-$C_6$alkyl-$SO_2$- or $C_1$-$C_6$alkyl-SO-, the $C_1$-$C_6$alkyl (e. g. methyl, ethyl, propyl, butyl, pentyl or hexyl) in the $C_1$-$C_6$alkyl, $C_1$-$C_6$alkyl-$SO_2$- and $C_1$-$C_6$alkyl-SO- are independently $C_1$-$C_4$alkyl; for example, methyl, ethyl, n-propyl, isopropyl, n-butyl, isobutyl, sec-butyl or tert-butyl; also for example, methyl.

In an embodiment of the present invention, when R6 is independently $C_1$-$C_6$alkyl, $C_1$-$C_6$alkyl-O-, hydroxyl substituted $C_1$-$C_6$alkyl, cyano substituted $C_1$-$C_6$alkyl, $C_1$-$C_6$haloalkyl,or $C_1$-$C_6$haloalkyl-O-, the $C_1$-$C_6$alkyl (such as methyl, ethyl, propyl, butyl, pentyl or hexyl group) in the $C_1$-$C_6$alkyl, Ci-Cealkyl-O-, hydroxyl substituted $C_1$-$C_6$alkyl, cyano substituted $C_1$-$C_6$alkyl, $C_1$-$C_6$haloalkyl, and Ci-Cehaloalkyl-O- are independently $C_1$-$C_4$alkyl; for example, methyl, ethyl, n-propyl, isopropyl, n-butyl, isobutyl, sec-butyl or tert-butyl; also for example, methyl.

In an embodiment of the present invention, when R6 is independently halogen, $C_1$-$C_6$haloalkyl, or Ci-Cehaloalkyl-O-, the halogen or the halogenin $C_1$-$C_6$haloalkyl and Ci-Cehaloalkyl-O- are independently fluorine, chlorine or bromine; for example, fluorine or chlorine.

In an embodiment of the present invention, when Ra, Rb, Rc, Rd, Re, Rf, Rg and Rh are independently halogen or$C_1$-$C_6$haloalkyl group, the halogen or the halogen in $C_1$-$C_6$haloalkyl are independently fluorine, chlorine or bromine; for example, fluorine or chlorine.

In an embodiment of the present invention, when Ra, Rb, Rc, Rd, Re, Rf, Rg and Rh are independently $C_1$-$C_6$alkyl, $C_1$-$C_6$alkyl-O-, $C_1$-$C_6$haloalkyl or cyano substituted $C_1$-$C_6$alkyl, the $C_1$-$C_6$alkyl (e. g. methyl, ethyl, propyl, butyl, pentyl or hexyl) in the $C_1$-$C_6$alkyl, $C_1$-$C_6$alkyl-O-, $C_1$-$C_6$haloalkyl and cyano substituted $C_1$-$C_6$alkyl are independently $C_1$-$C_4$alkyl; for example, methyl, ethyl, n-propyl, isopropyl, n-butyl, isobutyl, sec-butyl or tert-butyl; also for example, methyl.

In an embodiment of the present invention, when Cy is a saturated or partially unsaturated or unsaturated ring system of 3-8-membered or a saturated or partially unsaturated or unsaturated ring system of 3-10-membered substituted by one or more Rp, the 3-10-membered saturated or partially unsaturated or unsaturated ring system in the 3-10-membered saturated or partially unsaturated or unsaturated ring system or 3-10-membered saturated orpartially unsaturated or partially unsaturated ring system substituted by one or more Rp of Cy is a 6-10-membered saturated or partially unsaturated or unsaturated heterocyclyl, the heteroatom is selected from N, O and S, and the number of heteroatoms is 1, 2 or 3; for example, the Cy is

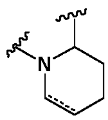 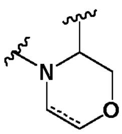, 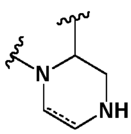, 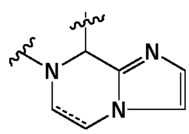;

also for example,

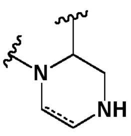.

In an embodiment of the present invention,when M is N, R is halogen, NR'R"-($C_1$-$C_4$alkylene)-O-, NR'R"-CO-($C_1$-$C_4$alkylene)-NR'-, Q1-($C_1$-$C_4$alkylene)-O-, Q2-($C_1$-$C_4$alkylene)-NR'-, or NR'R"-Q3-;

when M is CR5, R is hydrogen, halogen, NR'R"-($C_1$-$C_4$alkylene)-O-, NR'R"-CO-($C_1$-$C_4$alkylene)-NR'-, Q4-($C_1$-$C_4$alkylene)-O-, Q5-($C_1$-$C_4$alkylene)-NR'-, or NR'R"-Q6-;
Q1, Q2, Q3, Q4, Q5 and Q6 are independently 3-7-membered heterocycloalkyl or 3-7-membered heterocycloalkyl substituted by one or more Rq; when there are multiple substituents, they arethe same or different;
Rq is independently halogen, hydroxyl or $C_1$-$C_4$alkyl;
R5 is independently cyano;
R' and R" are independently hydrogen or $C_1$-$C_6$alkyl;
R1 is hydrogen or halogen;
R2 and R3 are independently hydrogen;
R4 is halogen;
m is 0,1 or 2;
W is independently C (halogen);
W1 is independently C (halogen);
W2 is CH;
any one of Ra, Rb, Re and Rf is hydrogen, C1-C6alkyl or cyano-substituted C1-C6alkyl, and the rest of Ra, Rb, Rc, Rd, Re, Rf, Rg and Rh are hydrogen; or, any one of Rc, Rd, Rg and Rh is hydrogen or methyl, and the rest of Ra, Rb, Rc, Rd, Re, Rf, Rg and Rh are hydrogen;
or either Rg orRh may form Cy together with M and the attached group; Cy is a saturated or partially unsaturated or unsaturated 3-10-membered ring system or a saturated or partially unsaturated or unsaturated 3-10-membered ring system substituted by one or more Rp;
Rp is independently $C_1$-$C_4$alkyl; or when two Rp are located on the same C-atom, they jointly form = O.

In an embodiment of the present invention,when M is N, R is halogen, NR'R"-($C_1$-$C_4$alkylene)-O-, NR'R"-CO-($C_1$-$C_4$alkylene)-NR'-, Q1-($C_1$-$C_4$alkylene)-O-, Q2-($C_1$-$C_4$alkylene)-NR'-, or NR'R"-Q3-;

when M is CR5, R is hydrogen, halogen, NR'R"-($C_1$-$C_4$alkylene)-O-, NR'R"-CO-($C_1$-$C_4$alkylene)-NR'-, Q4-($C_1$-$C_4$alkylene)-O-, Q5-($C_1$-$C_4$alkylene)-NR'-, or NR'R"-Q6-;
Q1, Q2, Q3, Q4, Q5 and Q6 are independently 3-7-membered heterocycloalkyl or 3-7-membered heterocycloalkyl substituted by one or more Rq; when there are multiple substituents, they are the same or different;
Rq is independently halogen, hydroxyl or $C_1$-$C_4$alkyl;
R5 is independently cyano;
R' and R" are independently hydrogen or $C_1$-$C_6$alkyl;
R1 is hydrogen or halogen;
R2 and R3 are independently hydrogen;
R4 is halogen;
m is 0,1 or 2;
W is independently C (halogen);
W1 is independently C (halogen);
W2 is CH;
when M is N, any one of Ra, Rb, Re and Rf is hydrogen, C1-C6alkyl or cyano-substituted C1-C6alkyl, and the rest of Ra, Rb, Rc, Rd, Re, Rf, Rg and Rh are hydrogen; or, any one of Rc, Rd, Rg and Rh is hydrogen or methyl, and the rest of Ra, Rb, Rc, Rd, Re, Rf, Rg and Rh are hydrogen;
When M is CR5, any one of Ra, Rb, Re and Rf is hydrogen or $C_1$-$C_6$alkyl, and the rest of Ra, Rb, Rc, Rd, Re, Rf, Rg and Rh are hydrogen; or any one of Rc, Rd, Rg and Rh is hydrogen or methyl, the rest of Ra, Rb, Rc, Rd, Re, Rf, Rg and Rh are hydrogen;
or either Rg orRh may form Cy together with M and the attached group; Cy is a saturated or partially unsaturated or unsaturated 3-10-membered ring system or a saturated or partially unsaturated or unsaturated 3-10-membered ring system substituted by one or more Rp;
Rp is independently $C_1$-$C_4$alkyl; or when two Rp are located on the same C-atom, they jointly form = O.

In an embodiment of the present invention, when M is N, R is NR'R"-($C_1$-$C_4$alkylene)-O-, Q1-($C_1$-$C_4$alkylene)-O- or NR'R"-Q3-;

When M is CR5, R is NR'R"-($C_1$-$C_4$alkylene)-O-, NR'R"-CO-($C_1$-$C_4$alkylene)-NR'-, Q4-($C_1$-$C_4$alkylene)-O-, QS-($C_1$-$C_4$alkylene)-NR'-, or NR'R"-Q6-;
Q1, Q3, Q4, Q5 and Q6 are independently 3-7-membered heterocycloalkyl or 3-7-membered heterocycloalkyl substituted by one or more Rq; when there are multiple substituents, they are the same or different;
Rq is independently halogen, hydroxyl or $C_1$-$C_4$alkyl;

R5 is independently cyano;
R’ and R" are independently hydrogen or $C_1$-$C_6$alkyl;
When M is N, R1 is hydrogen, when M is CR5, R1 is hydrogen or halogen;
R2 and R3 are independently hydrogen;
R4 is halogen;
m is 0,1 or 2;
W is independently C (halogen);
W1 is independently C (halogen);
W2 is CH;
when M is N, any one of Rc, Rd, Rg and Rh is hydrogen, or methyl, and the rest of Ra, Rb, Re, Rd, Re, Rf, Rg and Rh are hydrogen;
when M is CR5, any one of Ra, Rb, Re and Rf is hydrogen or $C_1$-$C_6$alkyl, and the rest of Ra, Rb, Re, Rd, Re, Rf, Rg and Rh are hydrogen; or any one of Re, Rd, Rg and Rh is hydrogen or methyl, the rest of Ra, Rb, Rc, Rd, Re, Rf, Rg and Rh are hydrogen.

In an embodiment of the present invention,when M is N, R is NR’R"-($C_1$-$C_4$alkylene)-O-, Q1-($C_1$-$C_4$alkylene)-O-, Q2-($C_1$-$C_4$alkylene)-NR’-, NR’R"-Q3-;

when M is CR5, R is hydrogen, Q4-($C_1$-$C_4$alkylene)-O-, Q5-($C_1$-$C_4$alkylene)-NR’-, NR’R"-CO-($C_1$-$C_4$alkylene)-NR’-, NR’R"-($C_1$-$C_4$alkylene)-O-, NR’R"-Q6-;
Q1, Q2, Q3, Q4, Q5 and Q6 are independently 3-7-membered heterocycloalkyl or 3-7-membered heterocycloalkyl substituted by one or more Rq; when there are multiple substituents, they are the same or different;
Rq is independently halogen, hydroxyl or $C_1$-$C_4$alkyl;
R5 is independently cyano;
R’ and R" are independently hydrogen or $C_1$-$C_6$alkyl;
R1 is hydrogen;
R2 and R3 are independently hydrogen;
R4 is halogen; for example, F;
m is 0,1 or 2; for example, 1;
W is independently C (halogen); For example, C(F);
W1 is independently C (halogen); For example, C(Cl);
W2 is CH;
When M is N, any one of Rc, Rd, Rg and Rh is hydrogen, or methyl, and the rest of Ra, Rb, Rc, Rd, Re, Rf, Rg and Rh are hydrogen;
When M is CR5, Ra, Rb, Rc, Rd, Re, Rf, Rg and Rh are hydrogen.

In an embodiment of the present invention,

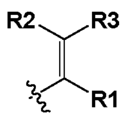

is

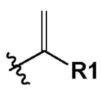,

for example,

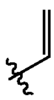 and 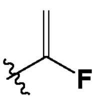.

In an embodiment of the present invention,

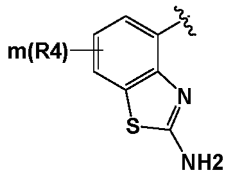

is

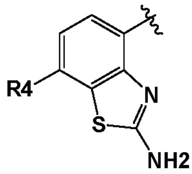 ,

for example,

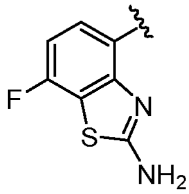 .

In an embodiment of the present invention,

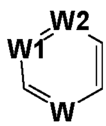

is

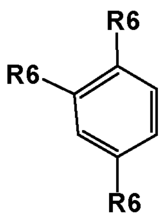 ,

for example,

, 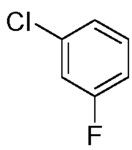 .

In an embodiment of the present invention,

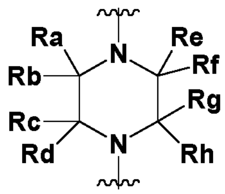

is

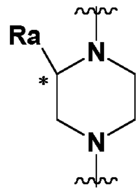

or

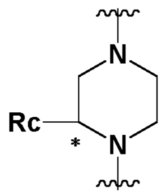

,

such as

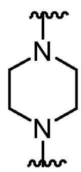, 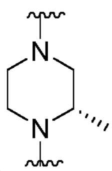, 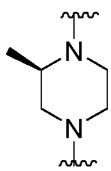, and 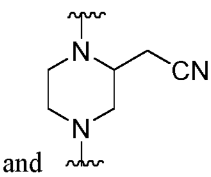.

The carbon atom with "*" means that when it is a chiral carbon atom, it is S configuration, R configuration or a mixture thereof.

In an embodiment of the present invention,R is hydrogen, chlorine,

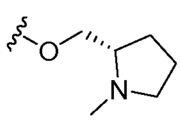, 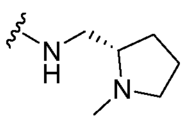,

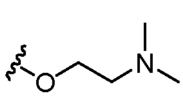, 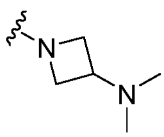, 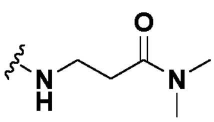, 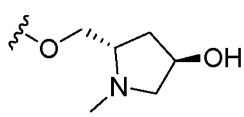, 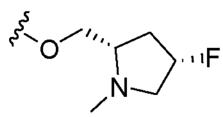,

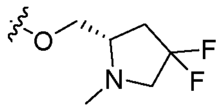

or

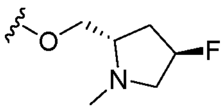
;

The carbon atom with "*" means that when it is a chiral carbon atom, it is S configuration, R configuration or a mixture thereof.

In an embodiment of the present invention,

Cy is selected from

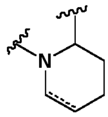, 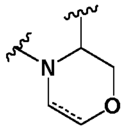, 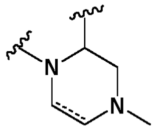, 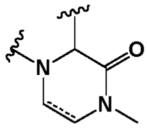 and 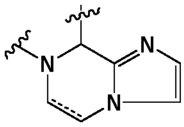;

for example,

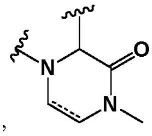,.

In an embodiment of the present invention, Acompound of formula (I), or a pharmaceutically acceptable salt thereof, or an enantiomer, a diastereoisomer, a tautomer, a torsional isomer, a solvate, a polymorph, or a prodrug thereof, is thecompound of formula IIA, IIB or IIC, or a pharmaceutically acceptable salt thereof, or an enantiomer, a diastereoisomer, a tautomer, a torsional isomer, a solvate, a polymorph, or a prodrug thereof:

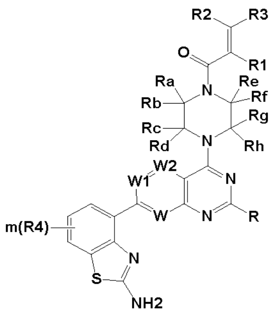 IIA, 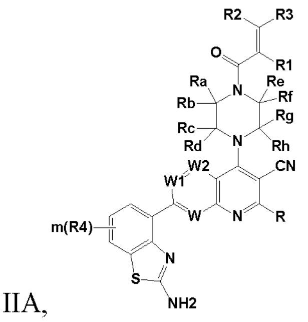 IIB, 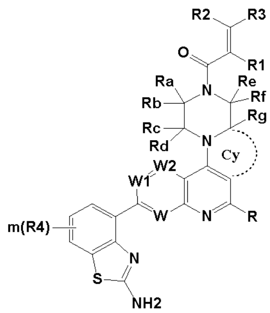 IIC;

wherein, Cy, R, R1, R2, R3, R4, m, Ra, Rb, Rc, Rd, Re, Rf, Rg, W, W1and W2 are as defined in the text.

In an embodiment of the present invention, the benzothiazolyl biaryl compound as shown in Formula I, wherein:

when M is N, R is independently selected from the group consisting of halogen, cyano, hydroxyl, nitro, amino, C1-C6alkyl, C1-C6alkyl-S-, $C_1$-$C_6$alkyl-SO-, $C_1$-$C_6$alkyl-$SO_2$-, $C_1$-$C_6$haloalkyl, $C_1$-$C_6$alkyl-O-, $C_1$-$C_6$alkyl-NR'R"-, 3-12-membered cycloalkyl, 3-12-membered heterocycloalkyl, 3-12-membered cycloalkyl-O-, 3-12-membered cycloalkyl-NR'R"-, 3-12-membered heterocycloalkyl-O-, 3-12-membered heterocycloalkyl-NR'R"-, 5-12-membered aryl and 5-12-membered heteroaryl;

when M is CR5, R is independently selected from the group consisting of hydrogen, halogen, cyano, hydroxyl, nitro, amino, $C_1$-$C_6$alkyl, $C_1$-$C_6$alkyl-S-, $C_1$-$C_6$alkyl-SO-, $C_1$-$C_6$alkyl-$SO_2$-, $C_1$-$C_6$haloalkyl, $C_1$-$C_6$alkyl-O-, $C_1$-$C_6$alkyl-NR'R"-, 3-12-membered cycloalkyl, 3-12-membered heterocycloalkyl, 3-12-membered cycloalkyl-O-, 3-12-membered cycloalkyl-NR'R"-, 3-12-membered heterocycloalkyl-O-, 3-12-membered heterocycloalkyl-NR'R"-, 5-12-membered aryl and 5-12-membered heteroaryl;

R5 is independently selected from the group consisting of hydrogen, halogen, cyano, hydroxyl, nitro, amino, $C_1$-$C_6$alkyl, $C_1$-$C_6$alkyl-S-, $C_1$-$C_6$alkyl-SO-, $C_1$-$C_6$alkyl-$SO_2$-, $C_1$-$C_6$haloalkyl, $C_1$-$C_6$alkyl-O-, $C_1$-$C_6$alkyl-NR'R"-, 3-12-membered cycloalkyl, 3-12-membered heterocycloalkyl, 3-12-membered cycloalkyl-O-, 3-12-membered cycloalkyl-NR'R"-, 3-12-membered heterocycloalkyl-O-, 3-12-membered heterocycloalkyl-NR'R"-, 5-12-membered aryl and5-12-membered heteroaryl; R', R" are independently selected from hydrogen, $C_1$-$C_6$alkyl, 3-12-membered cycloalkyl, 3-12-membered heterocycloalkyl, 5-12-membered aryl and heteroaryl;

R1 is independently selected from the group consisting of hydrogen, halogen, cyano, nitro, $C_1$-$C_6$alkyl, $C_1$-$C_6$alkyl-$SO_2$-, $C_1$-$C_6$alkyl-SO-, and$C_1$-$C_6$haloalkyl; R2 and R3 are independently selected from the group consisting of hydrogen, halogen, cyano, nitro, $C_1$-$C_6$alkyl, $C_1$-$C_6$alkyl-$SO_2$-, Ci-Cealkyl-SO- and $N(R_{2a})(R_{2b})$-$(CH_2)$x-; or $R_9$ and $R_{10}$together form a 5-10-membered nitrogen-containing heterocycloalkyl substituted by $C_1$-$C_6$alkyl; wherein, $R_{2a}$and $R_{2b}$ are independently selected from hydrogen and $C_1$-$C_6$alkyl, and x is selected from any integer of 0-5;

R4 is independently selected from hydrogen, halogen, cyano, nitro, $C_1$-$C_6$alkyl, $C_1$-$C_6$alkyl-$SO_2$-, $C_1$-$C_6$alkyl-SO-, $C_1$-$C_6$haloalkyl and $C_1$-$C_6$alkoxy, etc., m is an integer independently selected from 0-4;

W, W1 and W2 are independently selected from CR6 or N, R6 is independently selected from H, halogen, cyano, $C_1$-$C_6$alkyl, $C_1$-$C_6$alkoxy, haloalkyl,haloalkoxy, alkenyl, alkynyl, 3-8-membered cycloalkyl and 3-8-membered heterocycloalkyl, etc;

Ra, Rb, Rc, Rd, Re, Rf, Rg and Rh are independently selected from hydrogen, halogen, C1-C6 alkyl, alkoxy and haloalkyl, etc., respectively, or any two of Ra, Rb, Rc, Rd, Re, Rf, Rg and Rh form a saturated or partially unsaturated 3-8-membered ring system; or eitherRg orRh can form a saturated or partially unsaturated or unsaturated 3-8-membered ring system together with M.

One or more hydrogen atoms on any one of the abovementioned groups can be substituted by a substituent selected from the group consisting of(including but not limited to): deuterium, halogen, hydroxyl, amino or cycloamino, cyano, nitro, sulfone or sulfoxide, $C_1$-$C_8$alkyl, 3-8-membered cycloalkyl or heterocycloalkyl, $C_1$-$C_8$alkoxy, Ci-Csalkylamino, alkenyl, alkynyl, acyl or sulfonyl, urea or sulfonylurea, 5-8 membered aryl or heteroaryl; wherein the heteroaryl comprises 1-3 heteroatoms selected from the group consisting of N, O, P and S, the heterocycloalkyl group comprises 1-3 heteroatoms selected from the group consisting of N, O, P and S, and the ring system comprises saturated or partially unsaturated ring system such as a spiro ring, a bridged ring, a fused-ring, a fused ring, and the others.

In a further embodiment, the compound having the formula (I), or the pharmaceutically acceptable salt thereof, or an enantiomer, a diastereomer, a tautomer, a torsional isomer, a solvate, a polymorph or a prodrug thereof, preferably the compound is as shown in formula (IIA), (IIB), orthepharmaceutically acceptable salt thereof, or an enantiomer, a diastereoisomer, a tautomer, a torsional isomer, a solvate, a polymorph or a prodrug thereof:

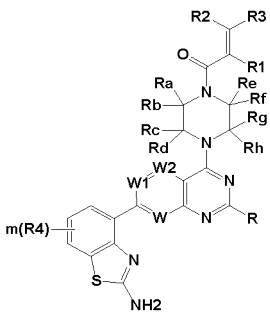
(IIA),
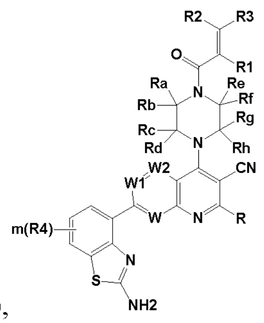
(IIB);

wherein, R, R1, R2, R3, R4, m, Ra, Rb, Rc, Rd, Re, Rf, Rg, W, W1, and W2 are as defined above.

In a further preferred embodiment, the compound having the formula (1), or a pharmaceutically acceptable salt thereof, or an enantiomer, a diastereoisomer, a tautomer, a torsional isomer, a solvate, a polymorph, or a prodrug thereof, wherein:

R1 is preferably selected from hydrogen, fluorine, methyl and cyano, etc.; R2 and R3 are independently preferably selected from hydrogen, methyl and fluorine, etc.; R4 is preferably selected from one or more fluorine; m is preferably selected from 0, 1 and 2;

Ra, Rb, Rc, Rd, Re, Rf, Rg and Rh are independently preferably selected from hydrogen, fluorine, methyl, hydroxymethyl, hydroxyethyl and cyano methyl, etc;

W is preferably selected from N, C-F, C-Cl, C-Me, C-OMe, C-OCH2CHF2, C-OCH2CF3, etc.; W2 is independently preferably selected from N, CH, C- F, C-Cl, C-Me and C-OMe, etc; W1 is independently selected from -CH, -C-halogen, -C-cyano, -C-cyclopropyl, -C-C1-C4alkyl, -C-C1-C4alkoxy, -C-C2-C4alkenyl, -C-C2-C4alkynyl, -C-C1-C4alkoxy, -C-C1-C4haloalkyl and -C-C1-C4haloalkoxy, etc; R is preferably selected from halogen, cyano, amino, C1-C6alkyl, C1-C6alkoxy, C1-C6alkylamino, C1-C6alkylaminoalkylene ether group, C1-C6alkylaminoalkylene amino group, 3-10 membered cycloalkylalkylene ether group and 3-10 membered heterocycloalkylalkylene ether group,

etc.

In an embodiment of the present invention, the benzothiazolyl biaryl compound as shown in Formula I hasthe following structure:

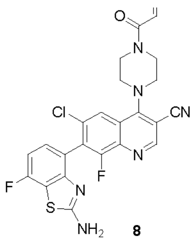
8
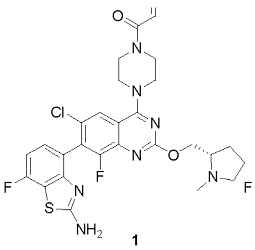
1
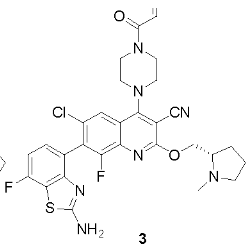
3
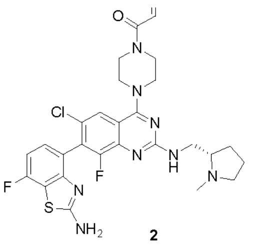
2
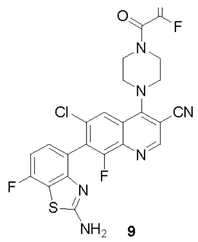
9

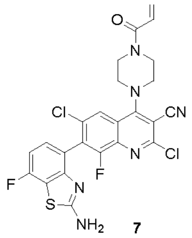
7
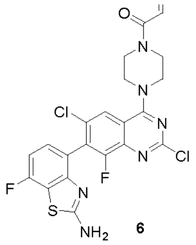
6
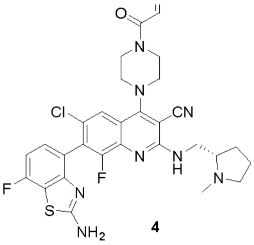
4
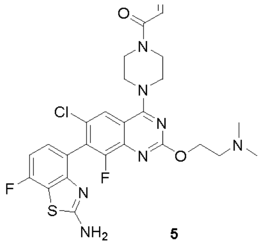
5
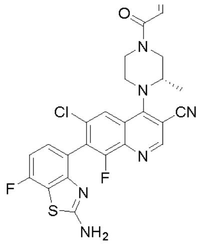

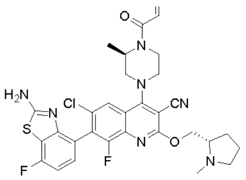
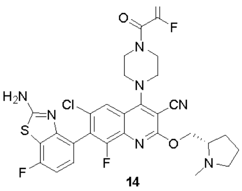
14
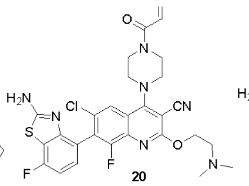
20
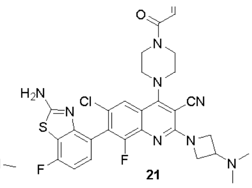
21
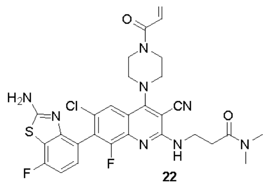
22

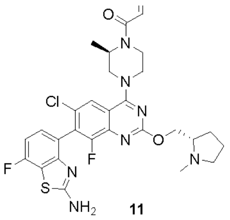
11
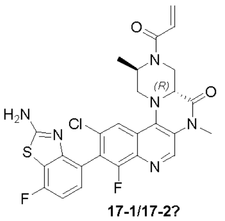
17-1/17-2?
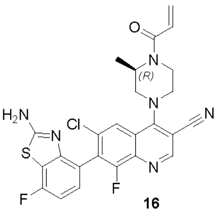
16
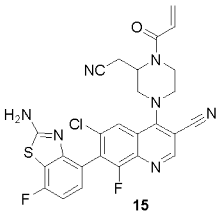
15
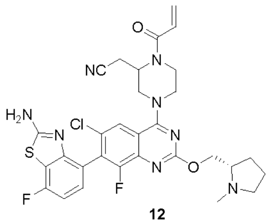
12

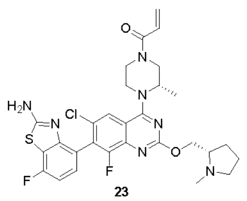
23
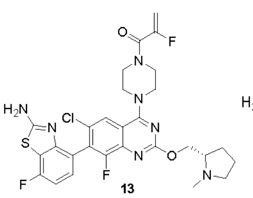
13
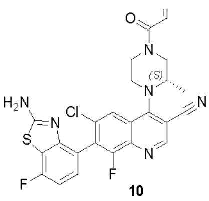
10
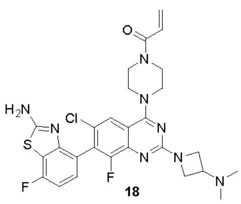
18
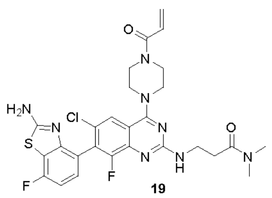
19

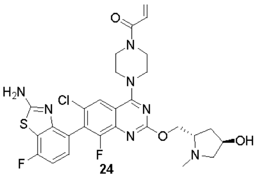
24
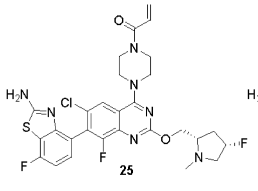
25
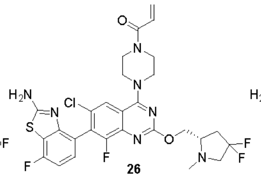
26
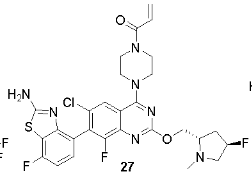
27
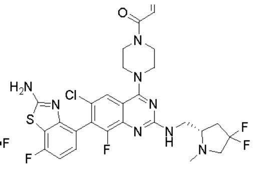

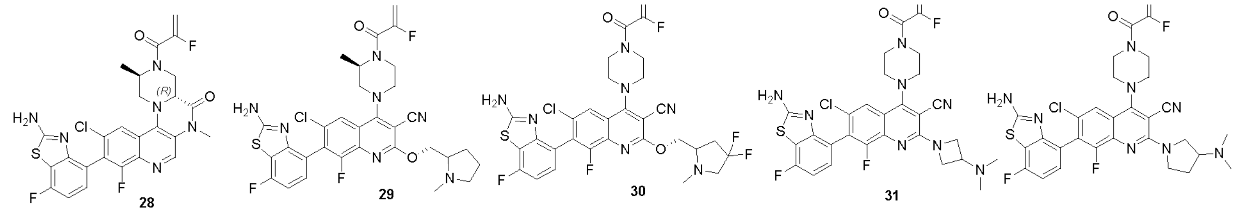

28 29 30 31

.

In a embodiment of the present invention, the benzothiazolyl biaryl compound as shown in Formula I is the following compound:

theretention time of the compound

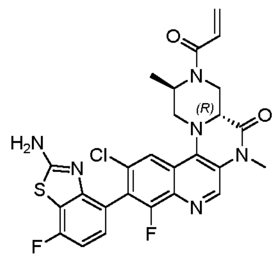

is 8.476min under the following conditions: preparative chromatographic resolution, chromatographic column:Sunfire C18 4.6*150mm, 5uM, elution gradient time: 13min, mobile phase: A:0.1% formic acid/water; B:0.1% formic acid/acetonitrile; flow rate:40 mL/min; wavelength:254nm;

theretention time of the compound

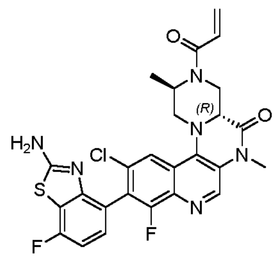

is 8.469 min under the following conditions: preparative chromatographic resolution, chromatographic column:Sunfire C18 4.6*150mm, SuM, elution gradient time: 13min, mobile phase: A:0.1% formic acid/water; B:0.1% formic acid/acetonitrile; flow rate:40 mL/min; wavelength:254nm.

Thus, throughout this specification, those skilled in the art may select the groups and their substituents in the benzothiazolyl biaryl compound as shown in Formula I, or a pharmaceutically acceptable salt thereof, or an enantiomer, a diastereoisomer, a tautomer, a torsional isomer, a solvate, a polymorph, or a prodrug thereof, to provide stable benzothiazolyl biaryl compounds as shown in formula I, or a pharmaceutically acceptable salt thereof, or an enantiomer, a diastereoisomer, a tautomer, a torsional isomer, a solvate, a polymorph, or a prodrug thereof, including but not limited to the compounds described in the examples of the present invention.

The benzothiazolyl biaryl compound shown in formula I, or a pharmaceutically acceptable salt thereof, or an enantiomer, a diastereoisomer, a tautomer, a torsional isomer, a solvate, a polymorph, or a prodrug thereof can be synthesized by a method that includes similar methods in the chemical field, and the steps and conditions can refer to the steps and conditions of similar reactions in the field, in particular, the synthesis is carried out according to the description herein. Starting materials are usually from commercial sources, such as Aldrich or can be easily prepared using methods known to those skilled in the art (obtained through SciFinder, Reaxys online database).

In the present invention, the benzothiazolyl biaryl compound shown in Formula I, or a pharmaceutically acceptable salt thereof, or an enantiomer, a diastereoisomer, a tautomer, a torsional isomer, a solvate, a polymorph, or a

prodrug thereof, can also be prepared by the obtained benzothiazolyl biaryl compound shown in formula I, or a pharmaceutically acceptable salt thereof, or an enantiomer, a diastereoisomer, a tautomer, a torsional isomer, a solvate, a polymorph, or a prodrug thereof, using the conventional method of the art to further obtained the other described benzothiazolyl biaryl compounds as shown in Formula I, or pharmaceutically acceptable salts thereof, or enantiomers, diastereomers, tautomers, torsional isomers, solvates, polymorphs, or prodrugs via peripheral modification.

In general, the compound of the present invention may be prepared by the methods described herein, unless further indicated, wherein the substituents are defined as shown in Formula I.The following reaction schemes and examples are used to further illustrate and exemplify the present invention.

The present invention also provides a method for the preparation of thebenzothiazolyl biarylcompound as shown in formula I, comprising steps a-c:

a) converting the compound of formula (A) to intermediate (B) by a transition metal-catalyzed coupling reaction with an arylboronic acid or an arylboronic ester or an aryl metal reagent (Ar-M); and

b) removing the protecting group PG from the compound of formula (B) by conventional functional groups to give the compound of formula (C);

c) condensation reaction of the compound of general formula (C) with acrylic acid or acryloyl chloride under appropriate conditions to produce general formula (I).

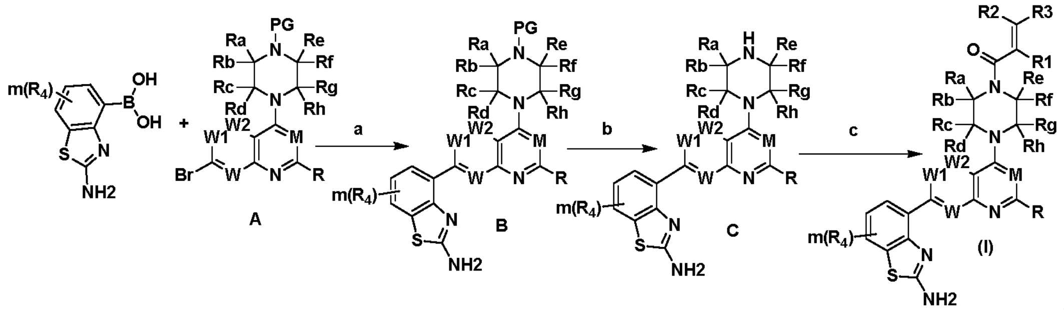

A B C (I)

The definitions of each group shown are as described above;

Preferably, the steps a), b) and c) are each carried out in respectivesolvent, and the solvent is selected from the group consisting of: water, methanol, ethanol, isopropanol, butanol, ethylene glycol, ethylene glycol methyl ether, N-methylpyrrolidone, dimethyl sulfoxide, tetrahydrofuran, toluene, dichloromethane, 1,2-dichloroethane, acetonitrile, N,N-dimethylformamide, N,N-dimethylacetamide, dioxane, and combinations thereof.

Preferably, the transition metal catalyst is selected from the group consisting of: tris (dibenzylidene acetone) dipalladium ($Pd_2(dba)_3$), tetrakis (triphenylphosphine) palladium ($Pd(PPh_3)_4$), palladium acetate, palladium chloride, dichlorobis (triphenylphosphine) palladium, palladium trifluoroacetate, palladium triphenylphosphine acetate, [1,1'-bis (diphenylphosphino) ferrocene] palladium dichloride, bis (tri-o-phenylmethylphosphine) palladium dichloride, 1,2-bis (diphenylphosphino) ethane palladium dichloride, or compositions thereof; the catalyst ligand is selected from the group consisting of: tri-tert-butylphosphine, tri-n-butylphosphine tetrafluoroborate, tri-n-butylphosphine, triphenylphosphine, tri-p-tolylphosphine, tricyclohexylphosphine, tri-o-tolylphosphine and combinations thereof.

Preferably, the combination of the condensing agent is selected from the group consisting of: DCC (dicyclohexylcarbodiimide), DIC (diisopropylcarbodiimide), CDI (carbonyldiimidazole), EDCI(1-ethyl -3 (3-dimethylpropylamine) Carbodiimide), HOAt (1-hydroxy-7-azabenzotriazole), HOBt (1-hydroxybenzotriazole), BOP (castros reagent), PyBOP (1H-benzotriazol-1-yl-oxytripyrrolidino-phosphonium hexafluorophosphate), HATU(2-(7-azabenzotriazole)-N,N,N',N'-tetramethyluroniumhexafluorophosphate), TBTU (2-(1H-Benzotriazole-1-yl)-1,1,3,3-tetramethyluronium tetrafluoroborate), etc., and combinations thereof.

Preferably, the inorganic base is selected from the group consisting of: sodium hydride, potassium hydroxide, sodium acetate, potassium acetate, potassium tert-butoxide, sodium tert-butoxide, potassium fluoride, cesium fluoride, potassium phosphate, potassium carbonate, potassium bicarbonate,sodium carbonate, sodium bicarbonate, and combinations thereof; the organic base is selected from the group consisting of: pyridine, triethylamine, N,N-diisopropylethylamine, 1, 8-Diazabicyclo [5.4.0] undec-7-ene (DBU), lithium hexamethyldisilyl, sodium hexamethyldisilyl, dimethylpyridine, and combinations thereof.

Preferably, the acid is selected from the group consisting of hydrochloric acid, sulfuric acid, phosphoric acid, methanesulfonic acid, toluenesulfonic acid, trifluoroacetic acid, formic acid, acetic acid, trifluoromethanesulfonic acid, and combinations thereof.

Preferably, the reducing agent is selected from the group consisting of: iron powder, zinc powder, stannous

chloride, sodium thiosulfate, sodium sulfite, hydrogen, etc.

The necessary raw materials or reagents for the preparation of compounds such as those in Formula I can be obtained commercially or prepared by synthetic methods known in the art.The compounds of the present invention can be prepared as free bases or as salts formed by addition of acids thereto by the methods described in the experimental section below.The term pharmaceutically acceptable salt refers to a pharmaceutically acceptable salt as defined herein and having all the pharmacological activity of the parent compound.A pharmaceutically acceptable salt can be prepared by adding the corresponding acid to a suitable organic solvent of an organic base and treating it according to conventional methods.

Examples of salt formation include: salts formed with inorganic acids, such as hydrochloric acid, hydrobromic acid, sulfuric acid, nitric acid or phosphoric acid; and salts formed with organic acids, such as acetic acid, benzenesulfonic acid, benzoic acid, camphor sulfonic acid, citric acid, ethanesulfonic acid, fumaric acid, gluconic acid, glutamic acid, glycolic acid, hydroxynaphthoic acid, 2-hydroxyethanesulfonic acid, lactic acid, maleic acid, malic acid, malonic acid, mandelic acid, methanesulfonic acid, mucofuranic acid, 2-naphthalenesulfonic acid, propionic acid, salicylic acid, succinic acid, tartaric acid, p-toluenesulfonic acid or trimethylacetic acid.

The benzothiazolyl biaryl compound shown in Formula I, or a pharmaceutically acceptable salt thereof, or an enantiomer, a diastereoisomer, a tautomer, a torsional isomer, a solvate, a polymorph, or a prodrug thereof may have one or more chiral carbon atoms, so it can be separated to obtain optically pure isomers, such as pure enantiomers, or racemates, or mixed isomers.Pure single isomers can be obtained by separation methods in the field, such as chiral crystallization into salts, or chiral preparative column separation.

Chemicals used in the synthetic route described in this patent, including solvents, reagents, catalysts, and protecting groups, deprotecting groups, protecting groups include tert-butoxycarbonyl (Boc).The abovementioned method may additionally include steps before or after the steps specifically described herein, where suitable protecting groups may be added or removed to obtain the target compound.In addition, various synthesis steps can be carried out alternately or sequentially to obtain the final target product.

The present invention also provides a compound as shown in formula A, B and C.

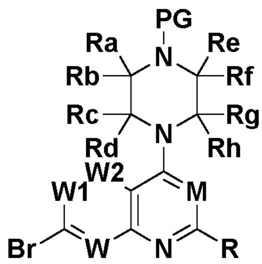

A

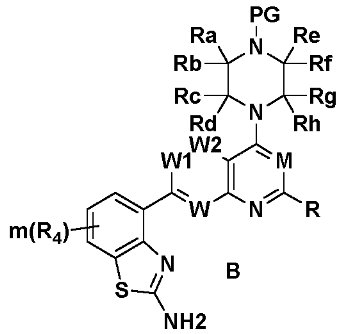

B

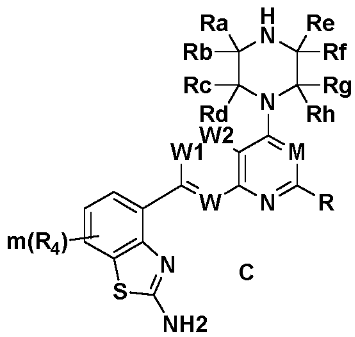

C

wherein, R, R4, m, Ra, Rb, Rc, Rd, Re, Rf, Rg, W, W1, W2 and M are as defined herein.

In an embodiment of the present invention, the compounds as shown in Formula A, B and C are respectively selected from the following compounds;

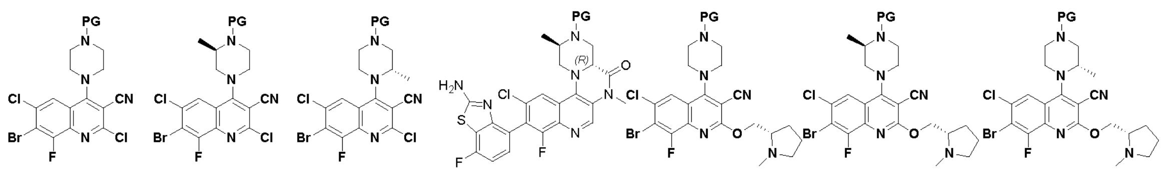

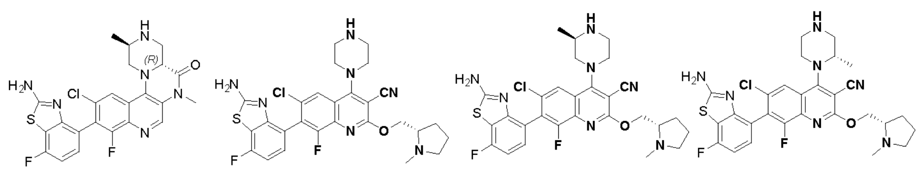

Another object of the present invention is to provide a medicamentand a composition thereof for treating or preventing tumors. The technical solutions to achieve the above objects are as follows:

The present invention provides a pharmaceutical composition comprising an effective amount of thebenzothiazolyl biaryl compound of formula I as described above, or a pharmaceutically acceptable salt thereof, or an enantiomer, a diastereomer, a tautomer, a torsional isomer, a solvate, a polymorph or a prodrug thereof, and (one or more) pharmaceutically acceptable carriers (pharmaceutical excipient).For example, such pharmaceutical compositions may comprise one or more additional benzothiazolyl biaryl compounds as shown in formula I, or a pharmaceutically acceptable salt thereof, or an enantiomer, a diastereomer, a tautomer, a torsional isomer, a solvate, a polymorph, or a prodrug thereof.In the pharmaceutical composition, the amount of the benzothiazolyl biaryl compound as shown in formula I, or the pharmaceutically acceptable salt thereof, or anenantiomer, a diastereomer, a tautomer, a torsional isomer, a solvate, a polymorph or a prodrug thereof may be a therapeutically effective amount.

The invention provides a pharmaceutical composition for treating tumors, which is consisting of the benzothiazolyl biaryl compound shown in the formula I, or a pharmaceutically acceptable salt thereof, or an enantiomer, a diastereoisomer, a tautomer, a torsional isomer, a solvate, a polymorph, or a prodrug thereof, and pharmaceutically acceptable carriers.

Another object of the present invention is to provide a use of the abovementioned compounds.The technical solutions to achieve the above objectsare as follows:

The present invention also provides an application of the benzothiazolyl biaryl compound as shown in formula I, or a pharmaceutically acceptable salt thereof, or an enantiomer, a diastereomer, a tautomer, a torsional isomer, a solvate, a polymorph or a prodrug thereof in the preparation of Ras (or ras or RAS) mutant protein inhibitor; in the application, the Ras mutantprotein can be $KRAS^{G12C}$(or $KRas^{G12C}$or $Kras^{G12C}$); the Ras mutantprotein inhibitor can be used in mammalian organisms; it can also be used in vitro, mainly for experimental purposes, for example, as a standard or control sample to provide a comparison, or to make a kit in accordance with the conventional methods in the field to provide rapid detection of the inhibitory effect of the Ras mutantprotein.

The present invention also provides a use of the benzothiazolyl biaryl compound as shown in formula I, or a pharmaceutically acceptable salt thereof, or an enantiomer, a diastereomer, a tautomer, a torsional isomer, a solvate, a polymorph or a prodrug thereof in the preparation of drugs; the drug can be a drug for the treatment of diseases related to the activity or expression of Ras mutant protein; or, the drug may be a therapeutic drug for tumors.The Ras mutant protein can be $KRAS^{G12C}$.The tumor can be independently selected from non-small cell lung cancer, small cell lung cancer, lung adenocarcinoma, lung squamous cell carcinoma, breast cancer, prostate cancer, liver cancer, skin cancer, gastric cancer, intestinal cancer, cholangiocarcinoma, brain cancer, leukemia, lymphoma, fibroma, sarcoma, basal cell carcinoma, glioma, kidney cancer, melanoma, bone cancer, thyroid cancer, nasopharyngeal cancer, and pancreatic cancer, etc.

Another aspect of the invention relates to a method for thepreventionand/or treatment ofdiseases associated with the activity or expression of Ras mutant protein, comprising administering to a patient a therapeutically effective amount of the benzothiazolyl biaryl compound as shown in formula I, or a pharmaceutically acceptable salt thereof, or an enantiomer, a diastereomer, a tautomer, a torsional isomer, a solvate, a polymorph, or a prodrug thereof.

Another aspect of the invention relates to a method of preventing and/or treating tumor comprising administering to a patient a therapeutically effective amount of the benzothiazolyl biaryl compound as shown in formula I, or a pharmaceutically acceptable salt thereof, or an enantiomer, a diastereomer, a tautomer, a torsional isomer, a solvate, a polymorph, or a prodrug thereof.

Another aspect of the present invention relates to a medicament for the prevention and/or treatment of diseases or tumors associated with the activity or expression of Ras mutant protein, comprising the benzothiazolyl biaryl compound as shown in formula I, or a pharmaceutically acceptable salt thereof, or an enantiomer, a diastereomer, a tautomer, a torsional isomer, a solvate, a polymorph or a prodrug thereof.

The benzothiazolyl biaryl compound of the formula I, or a pharmaceutically acceptable salt thereof, or an enantiomer, a diastereomer, a tautomer, a torsional isomer, a solvate, a polymorph, or a prodrug thereof, is used to prepare a drug for treating diseases related to the activity or expression of Ras mutant protein, especially a therapeutic drug for tumors.The tumor can be independently selected from non-small cell lung cancer, small cell lung cancer, lung adenocarcinoma, lung squamous cell carcinoma, breast cancer, prostate cancer, liver cancer, skin cancer, gastric cancer, intestinal cancer, cholangiocarcinoma, brain cancer, leukemia, lymphoma, fibroma, sarcoma, basal cell carcinoma, glioma, kidney cancer, melanoma, bone cancer, thyroid cancer, nasopharyngeal cancer, and pancreatic cancer, etc.

The invention relates to a compound with the structural characteristics of Formula I, which can inhibit a variety of tumor cells, especially can efficiently kill tumors that related tothe abnormal signal pathway of KRas G12C mutation protein, and is a kind of therapeutic drug with a new mechanism of action.

The pharmaceutical excipients described may be those excipients widely used in the field of pharmaceutical production.Excipients are mainly used to provide a safe, stable and functional pharmaceutical composition, and can also provide methods to enable the active ingredient to dissolve at a desired rate after the subject receivingadministration, or to promote the effective absorption of the active ingredient after the subject receivingthe composition.The pharmaceutical excipient may be an inert filler or provide a function such as stabilizing the overall pH of the composition or

preventing degradation of the active ingredient of the composition. The pharmaceutical excipient may include one or more of the following excipients: binders, suspending agents, emulsifiers, diluents, fillers, granulators, adhesives, disintegrants, lubricants, anti-adhesiveagents, glidants, wetting agents, gelling agents, absorption retardants, dissolution inhibitors, enhancers, adsorbents, buffers, chelating agents, preservatives, colorants, flavoring agents, and sweeteners.

Substances that can be used as pharmaceutically acceptable excipients include, but are not limited to, ion exchangers, aluminum, aluminum stearate, lecithin, serum proteins, such as human serum protein, buffer substances such as phosphate, glycine, sorbic acid, potassium sorbate, partial glyceride mixtures of saturated vegetablefatty acids, water, salts or electrolytes, such as protamine sulfate, disodium hydrogen phosphate, potassium hydrogen phosphate, sodium chloride, zinc salt, colloidal silicon, magnesium trisilicate, polyvinylpyrrolidone, polyacrylate, wax, polyethylene-polyoxypropylene-blocking polymer, lanolin, sugar, such as lactose, glucose and sucrose; starch such as corn starch and potato starch; cellulose and its derivatives such as sodium carboxymethyl cellulose, ethyl cellulose and cellulose acetate; gum powder; malt; gelatin; talcum powder; accessories such as cocoa butter and suppository wax; oils such as peanut oil, cotton seed oil, safflower oil, sesame oil, olive oil, corn oil and soybean oil; diol compounds, such as propylene glycol and polyethylene glycol; esters such as ethyl oleate and ethyl laurate; agar; buffers such as magnesium hydroxide and aluminum hydroxide; alginic acid; pyrogen-free water; isotonic salt; Ringer's solution; ethanol, phosphoric acid buffer solution, and other non-toxic suitable lubricants such as sodium laurate and magnesium stearate, colorants, release agents, coating materials, sweeteners, flavoring agents and fragrances, preservatives and antioxidants.

The pharmaceutical compositions of the present invention may be prepared according to the disclosure using any method known to those skilled in the art.For example, conventional mixing, dissolution, granulation, emulsification, grinding, encapsulation, embedding or freeze-drying processes.

The pharmaceutical dosage form of the compound of the present invention can be provided in the form of immediate release, controlled release, sustained release or target drug release system.For example, commonly used dosage form includes solutions and suspensions, (micro) emulsions, ointments, gels and patches, liposomes, tablets, sugar-coated pills, soft-or hard-shell capsules, suppositories, ovules, implants, amorphous or crystalline powders, aerosols and lyophilized preparations. Depending on the route of administration used, special devices may be required to applyor administer drugs, such as syringes and needles, inhalers, pumps, syringe pens, applicators or special bottles (Specialflask).Drug dosage forms often consist of drugs, excipients and container/sealing systems.One or more excipients (also called inactive ingredients) may be added to the compounds of the present invention to improve or promote the manufacture, stability, administration and safety of the drug, and may provide a method of obtaining the desired drug release profile. Therefore, the type of excipient added to the drug can depend on various factors, such as the physical and chemical characteristics of the drug, the route of administration, and the preparation steps.Medicinal excipients are present in this field and include those listed in various pharmacopoeia.(See U.S. Pharmacopoeia (U.S. Pharmacopeia, USP), Japanese Pharmacopoeia (Japanese Pharmacopoeia, JP), European Pharmacopoeia (European Pharmacopoeia, EP) and British Pharmacopoeia (British pharmacopoeia, BP); Publications of the U.S. Food and Drug Administration (www.fda.gov) Center for Drug Evaluation and Research (Centerfor Drug Evaluation and Research, CEDR), such as the Guide for Inactive Components (Inactive Ingredient Guide, 1996); Hand book of Pharmaceutical Additives, 2002, United Information Resources (Synapse Information Resources, Inc., Endicott NY;etc.) edited by Ash and Ash.

The pharmaceutical dosage form of the compounds of the present invention can be manufactured by any of the methods well known in the art, such as by conventional mixing, sieving, dissolving, melting, granulation, making sugar-coated pills, tableting, suspension, extrusion, spray drying, grinding, emulsifying, (nano/micron) encapsulating, embedding, or lyophilization processe.As described above, the compositions of the present invention may comprise one or more physiologically acceptable inactive ingredients which facilitate the processing of the active molecules into formulations for pharmaceutical use.

The pharmaceutical composition of the present invention can be administered topically or systemically, for example, for enteral administration, such as rectal or oral administration, or for parenteral administration to mammals (especially humans), and includes a therapeutically effective amount of a compound according to the present invention, its stereoisomer or pharmaceutically acceptable salts thereof as the active ingredient, together with pharmaceutically acceptable excipients, such as pharmaceutically acceptable carriers. The therapeutically effective amount of the active ingredient is as defined in the context and depends on the species of mammal, body weight, age, individual condition, individual pharmacokinetic parameters, disease to be treated and mode of administration. For enteral administration, such as oral administration, the compound of the present invention can be formulated in a wide range of dosage forms.

The pharmaceutical compositions and dosage forms may comprise one or more compounds of the present invention, stereoisomers thereof, or one or more pharmaceutically acceptable salts thereof as active components.The pharmaceutically acceptable carrier may be solid or liquid. Preparations in solid form include powders, tablets, pills, lozenges, capsules, bladders, suppositories, and dispersible granules. The solid carrier may also be one or more substances as diluents, flavorants, solubilizers, lubricants, suspending agents, binders, preservatives, tablet disintegrants, or encapsulating materials.In powders, the carrier is usually a finely divided solid, which is a mixture with finely divided

active components.In tablets, the active component is usually mixed with carriers with necessary adhesive ability in a suitable ratio and compacted according to the desired shape and size. Suitable carriers include, but are not limited to, magnesium carbonate, magnesium stearate, talc, sugar, lactose, pectin, dextrin, starch, gelatin, methyl cellulose, sodium carboxymethyl cellulose, low melting point wax, cocoa butter, etc.The formulation of the active compound may include encapsulation material as carrier, providing capsules, wherein the active component with or without the carrier is surrounded by the carrier bound thereto.

Other forms suitable for oral administration include liquid form preparations, including emulsions, syrups, elixirs, aqueous solutions, aqueous suspensions, or solid form preparations intended to be converted into liquid form preparations shortly before use.Emulsions may be prepared in solution, such as in aqueous propylene glycol, or may contain emulsifiers such as lecithin, sorbitan monooleate or arabicgum.The aqueous solution can be prepared by dissolving the active component in water and adding suitable colorants, fragrances, stabilizers and thickeners. The aqueous suspension can be prepared by dispersing fine particles of active ingredients in water with binders such as natural or synthetic gels, resins, methylcellulose, carboxymethylcellulose, and other commonly used suspending agents. Formulations in solid form include solutions, suspensions and emulsions, in addition to active components, they may also contain colorants, fragrances, stabilizers, buffers, artificial and natural sweeteners, dispersants, thickeners, solubilizers, etc.

Exemplary combinations for rectal administration include suppositories, which may include, for example, suitable non-irritating excipients, such as cocoa butter, synthetic glycerides, or polyethylene glycols, which are solid at room temperature, but melt and/or dissolve in the rectal cavity to release drug.

The compound of the invention may also be administered parenterally, for example, by inhalation, injection or infusion, such as by intravenous, intra-arterial, intra-osseous, intramuscular, intra-cerebral, extra-ventricular, intra-synovial, intra-sternal, intrathecal, intralesional, intracranial, intratumoral, intratumoral, intra-and subcutaneous injection or infusion.

Thus, for parenteral administration, the pharmaceutical composition of the present invention may be in the form of a sterile injectable or infusable formulation, for example, as a sterile aqueous or oily suspension.The suspension can be formulated using suitable dispersants or wetting agents (e. g. Tween 80) and suspending agents according to techniques known in the art.The sterile injectable or infusable formulation may also be a non-toxic parenterally acceptable diluent or a sterile injectable or infusable solution or suspension in a solvent.For example, the pharmaceutical composition may be a solution in 1, 3-butanediol.Other examples of acceptable media and solvents that may be used in the pharmaceutical compositions of the present invention include, but are not limited to, mannitol, water, Ringer's solutions, and isotonic sodium chloride solutions.In addition, sterile non-volatile oils are often used as solvents or suspension media.Any mild non-volatile oil, including synthetic monoglycerides or diglycerides, may be used for this purpose.Fatty acids such as oleic acid and its glyceryl derivatives can be used to prepare injections, as well as natural pharmaceutically acceptable oils, such as olive oil or castor oil, especially their polyoxyethylated form. These oil solutions or suspensions may also contain long-chain alcohol diluents or dispersants. Solutions for parenteral use may also include suitable stabilizers and, if desired, buffer substances. Suitable stabilizers include antioxidants, such as sodium bisulfate, sodium sulfite or ascorbic acid, citric acid and its salts and EDTA sodium salts, alone or in combination. Parenteral solutions can also contain preservatives such as benzalkonium chloride, p-hydroxybenzoic acid or propyl p-hydroxybenzoate and chlorobutanol.

For inhalation or nasal administration, suitable drug preparation is particles, aerosols, powders, mist or small drops, for example, withan average size of about 10 microns or less in diameter. For example, a composition for inhalation in the form of a solution may be prepared in brine, using benzyl alcohol or other suitable preservatives, absorption enhancers for improving bioavailability, fluorocarbons and/or other solubilizers or dispersants known in the art.

The pharmaceutical composition of the present invention can also be administered topically to the skin or mucous membranes.For topical applications, the pharmaceutical composition may be, for example, a lotion, a gel, a paste, a tincture, a transdermal patch, a gel for transmucosal delivery.

The pharmaceutical composition may be a suitable ointment formulation comprising active ingredients suspended or dissolved in a carrier. Carriers for topical administration of the compound of the invention include, but are not limited to, mineral oil, liquid petroleum, white petroleum, propylene glycol, polyoxyethylene, polyoxypropylene compounds, emulsified waxes and water. Alternatively, the pharmaceutical composition may be formulated as a suitable lotion or emulsion comprising active compoundssuspended or dissolved in a carrier. Suitable carriers include, but are not limited to, mineral oil, sorbitan monostearate, polysorbate 60, cetyl alcohol, 2-octyldecanol, benzyl alcohol and water. The pharmaceutical composition of the present invention can also be applied topically to the lower intestine by rectal suppository preparations or suitable enema preparations. Suitable pharmaceutical excipients (such as carriers) and methods for preparing pharmaceutical dosage forms are described in standard reference textbooks in the field of pharmaceutical preparations (Remington's Pharmaceutical Sciences, Mack Publishing Company).

It should be understood that within the scope of the present invention, the above-mentioned technical features of the present invention and the technical features specifically described in the following (such as embodiments) can be combined with each other to form a new or preferred technical solution. Limited by space, it will not be repeated.

## Terms

Unless otherwise defined, all scientific and technical terms herein have the same meaning as those generally understood by those skilled in the field to which the subject matter of the claim belongs.Unless otherwise specified, all patents, patent applications, and public materials cited in this article are incorporated into this article by reference.

It should be understood that the above brief description and the following detailed description are exemplary and for interpretation only, and do not limit the subject matter of the present invention.In this application, the use of the singular includes the plural unless otherwise specifically specified.It is important to note that the singular form used in this specification and claims includes the plural form of the thing referred to, unless the context clearly indicates otherwise.It should also be noted that "or", "or" are used to mean "and/or" unless otherwise specified.In addition, the terms "including" and other forms, such as "consisting of", "comprising" and "comprising", are not limiting.

Definitions of standard chemical terms can be found in references (including Carey and Sundberg "ADVANCED ORGANIC CHEMISTRY 4TH ED." Vols.A (2000) and B (2001), Plenum Press, New York).Unless otherwise specified, conventional methods within the technical scope of the art, such as mass spectrometry, NMR, IR and UV/VIS spectroscopy and pharmacological methods, are used.Unless specific definitions are proposed, the terms used in the description of analytical chemistry, organic synthetic chemistry, and pharmaceutical and pharmaceutical chemistry are known in the art. Standard techniques can be used in chemical synthesis, chemical analysis, drug preparation, formulation and delivery, and treatment of patients.For example, the reaction and purification may be carried out using manufacturer's instructions for the use of the kit, or in a manner known in the art or in accordance with the instructions of the present invention.The above techniques and methods can generally be implemented in accordance with conventional methods well known in the art, as described in a number of summary and relatively specific documents cited and discussed in this specification.In this specification, the group and its substituents can be selected by the skilled person in the art to provide a stable structural moiety and compound.

When the substituent is described by the conventional chemical formula written from left to right, the substituent also includes the chemically equivalent substituent obtained when writing the structural formula from right to left.For example, $-CH_2O-$is equivalent to$-OCH_2-$.

The chapter titles used in this article are only used for the purpose of organizing the article and should not be interpreted as a restriction on the topic in question. All documents or portions of documents cited in this application, including, but not limited to, patents, patent applications, articles, books, operation manuals and papers, are incorporated herein by reference as a whole.

Some chemical groups defined in this article are preceded by a simplified notation to represent the total number of carbon atoms present in this group.For example, C1-6 alkyl, C1-C6 alkyl, $C_{1-6}$alkyl, or $C_1$-$C_6$alkyl refers to an alkyl group having a total of 1 to 6 carbon atoms as defined below.The total number of carbon atoms in the simplified symbol does not include carbon that may be present in the substituents of the group.

Some chemical groups defined in this article are preceded by a simplified notation to represent the total number of carbon atoms present in this group.For example, a C1-C6 alkyl group means an alkyl group having a total of 1, 2, 3, 4, 5, or 6 carbon atoms as defined below.The total number of carbon atoms in the simplified symbol does not include carbon that may be present in the substituents of the group.

In this article, the numerical ranges defined in the substituent, such as 0 to 4, 1-4, 1 to 3, etc., indicate the integers in this range, for example, 1-6 is 1, 2, 3, 4, 5, 6.

In various parts of the present invention, the linking substituents are described. When the structure clearly requires a linking group, the Markush variable enumerated for the group should be understood as a linking group.For example, if the structure requires a linking group and the definition of the markush group for this variable enumerates "alkyl" or "aryl", it should be understood that the "alkyl" or "aryl" represents an alkylene group or an arylene group for the linkage, respectively.

In some specific structures, when the alkyl group is clearly represented as a linking group, the alkyl represents an alkylene for linkage, for example, the C- $C_6$alkyl in the "halo-$C_1$-$C_6$alkyl" should be understood as $C_1$-$C_6$alkylene (such as methylene, ethylene, propylene, butylene, pentylene, isopropylene, isobutylene, sec-butylene, tert-butylene, isoamylene, 2-methylbutylene, 1-methylbutylene, 1-ethyl propylene, 1 ,2-dimethyl propylene, neopentylene or 1,1-dimethyl propylene, etc.).

In addition to the foregoing, when used in the specification and claims of this application, the following terms have the meanings indicated below, unless otherwise specifically indicated.

The term "includes" is an open-ended expression, i.e., it includes what is specified in the present invention, but does not exclude other aspects.

The term "substituted" means that any one or more hydrogen atoms on a particular atom are substituted by substituents, including heavy hydrogen and hydrogen variants, as long as the valence state of the particular atom is normal and the substituted compound is stable.

In general, the term "substituted" means that one or more hydrogen atoms in a given structure are substituted

by a specific substituent. Further, when the group is substituted by one or more of the substituents, the substituents are independent of each other, that is, the one or more of the substituents may be different from each other or the same.Unless otherwise indicated, a substituent group can be substituted at each of the substitutable positions of the group being substituted. When more than one position in the given structural formula can be substituted by one or more substituents selected from specific groups, then the substituents can be at the same or different position.

In each part of this specification, the substituents of the compound disclosed in the present invention are disclosed according to the group type or range.In particular, the present invention includes each independent secondary combination of each member of these group types and ranges.For example, the term "$C_1$-$C_6$alkyl" or "$C_{1-6}$alkyl" specifically refers to independently disclosed methyl, ethyl, $C_3$alkyl, $C_4$alkyl, $C_5$alkyl and $C_6$alkyl; "$C_{1-4}$alkyl" specifically refers to independently disclosed methyl, ethyl, $C_3$alkyl (i. e., propyl, including n-propyl and isopropyl), $C_4$alkyl (i. e. butyl, including n-butyl, isobutyl, sec-butyl and t-butyl).

In this application, the term "halogen" refers to fluorine, chlorine, bromine or iodine; "hydroxy group" refers to-OH group; "hydroxy alkyl" refers to alkyl as defined below substituted by hydroxyl (-OH); "carbonyl" refers to-C(= O)-; "nitro" refers to-$NO_2$; "cyano" refers to-CN; "Amino" refers to-$NH_2$; "substituted amino" refers to amino substituted by one or two alkyl, alkyl carbonyl, aralkyl, and heteroaralkyl as defined below, for example, monoalkylamino, dialkylamino, alkylacylamino, aralkyl amino, heteroaralkyl amino; "carboxyl" refers to-COOH; "Acyl" refers to-C(= O)H; "Sulfone" refers to-S(= O)$_2$-; "Sulfoxide" refers to-S(= O)-; "Sulfonyl" refers to-S(= O)zH; "Urea" refers to-NH-C(= O)-$NH_2$; "Sulfonylurea" refers to the-S(= O)z-NH-C(= O)-$NH_2$; "alkoxy" refers to the alkyl-O- as defined below.

In this application, as a group or part of another group (e. g., used in a halogen-substituted alkyl group or the like), the term "alkyl" means a straight or branched hydrocarbon chain group consisting only of carbon and hydrogen atoms, free of unsaturated bonds, having, for example, 1 to 12 (preferably 1 to 8, more preferably 1 to 6) carbon atoms, and linked to the rest of the molecule by a single bond. Examples of alkyl include, but are not limited to, methyl, ethyl, n-propyl, isopropyl, n-butyl, isobutyl, sec-butyl, tert-butyl, n-pentyl, 2-methylbutyl, 2, 2-dimethylpropyl, n-hexyl, heptyl, 2-methylhexyl, 3-methylhexyl, octyl, nonyl, and decyl, etc.

As used herein, the term "alkylene " refers to a saturated, branched or straight chain or cyclic hydrocarbon group of the stated number of carbon atoms (typically 1-6 carbon atoms) and having two monovalent group centers derived by removing two hydrogen atoms from the same or two different carbon atoms of the parent alkane. Typical alkylene groups include but are not limited to methylene (-$CH_2$-), ethylene {including 1, 2-ethylene (-$CH_2CH_2$-), 2,2-ethylene (-CH($CH_3$)-)}, propylidene (including 2,3-propylidene (-CH($CH_3$)$CH_2$-), isopropylidene (-C($CH_3$)$_2$-), 1,3-propylidene (-$CH_2CH_2CH_2$-)}, butylidene (including 1, 4-butylidene (-$CH_2CH_2CH_2CH_2$-)}.

In this application, as a group or part of other groups, the term "alkenyl" means a linear or branched hydrocarbon chain group composed only of carbon atoms and hydrogen atoms, containing at least one double bond, having for example 2 to 14 (preferably 2 to 10, more preferably 2 to 6) carbon atoms, and connected to the rest of the molecule through a single bond, for example, but not limited to vinyl, propyl, allyl, but-1-enyl, but-2-enyl, pent-1-enyl, pent-1,4-dienyl, and the like.

In this application, as a group or part of other groups, the term "alkynyl" means a linear or branched hydrocarbon chain group consisting only of carbon atoms and hydrogen atoms, containing at least one triple bond and optionally one or more double bonds, having, for example, 2 to 14 (preferably 2 to 10, more preferably 2 to 6) carbon atoms and connected to the rest of the molecule through a single bond, for example, but not limited to ethynyl, prop-1-alkynyl, butyl-1-alkynyl, pent-1-en-4-alkynyl, etc.

In this application, as a group or part of other groups, the term "cycloalkyl" means a stable non-aromatic monocyclic or polycyclic hydrocarbon group composed only of carbon atoms and hydrogen atoms, which may include a fused ring system, a bridged ring system or a spiro ring system, having 3 to 15 carbon atoms, preferably 3 to 10 carbon atoms, more preferably 3 to 8 carbon atoms, and it is saturated or unsaturated and can be connected to the rest of the molecule through any suitable carbon atom through a single bond.Unless otherwise specified in this specification, the carbon atoms in the cycloalkyl group may optionally be oxidized. Examples of cycloalkyls include, but are not limited to, cyclopropyl, cyclobutyl, cyclopentyl, cyclopentenyl, cyclohexyl, cyclohexenyl, cyclohexadienyl, cycloheptyl, cyclooctyl, 1H-indenyl, 2,3-dihydroindenyl, 1,2,3,4-tetrahydro-naphthyl, 5,6,7,8-tetrahydro-naphthyl, 8, 9-dihydro-7H-benzocyclohepten-6-yl, 6,7,8, 9-tetrahydro-5H-benzocycloheptenyl, 5,6,7,8,9,10-hexahydro-benzocyclooctenyl, fluorenyl, bicyclo [2.2.1] heptyl, 7, 7-dimethyl-bicyclo [2.2.1] heptyl, bicyclo [2.2.1] heptene, bicyclo [2.2.2] octyl, bico [3.1.1] heptyl, bico [3.2.1] octyl, bico [2.2.2] octenyl, bico [3.2.1] octenyl, adamantyl, octahydro-4, 7-methylene-1H-indenyl, and octahydro-2, 5-methylene-o-cyclopentadienyl, etc.

In this application, the term "heterocyclic", as part of a group or other groups, means a stable 3-to 20-membered non-aromatic cyclic group consisting of 2 to 14 carbon atoms and 1 to 6 heteroatoms selected from the group consisting of nitrogen, phosphorus, oxygen and sulfur.Unless otherwise specified in this specification, the heterocyclic group may be a monocyclic, bicyclic, tricyclic or more ring system, which may include a fused ring system, a bridged ring system or a spiro ring system; the nitrogen, carbon or sulfur atoms in the heterocyclic group may optionally be oxidized; the nitrogen atoms may optionally be quaternized; and the heterocyclic may be partially or fully saturated. Heterocyclic

groups can be connected to the rest of the molecule via carbon atoms or heteroatoms through a single bond.Among the heterocyclic groups containing fused rings, one or more rings may be aryl or heteroaryl as defined below, provided that the point of attachment to the rest of the molecule is a non-aromatic ring atom.For the purposes of the present invention, the heterocyclic group is preferably a stable 4-to -11-member non-aromatic monocyclic, bicyclic, bridged or spiro ring group comprising 1 to 3 heteroatoms selected from nitrogen, oxygen and sulfur, more preferably a stable 4- to 8-membered non-aromatic monocyclic, bicyclic, bridged or spiro group comprising 1 to 3 heteroatoms selected from nitrogen, oxygen and sulfur.Examples of heterocyclic groups include, but are not limited to: pyrrolidyl, morpholino, piperazinyl, homopiperazinyl, piperidinyl, thiomorpholinyl, 2, 7-diaza-spiro [3.5] nonane-7-yl, 2-oxa-6-aza-spiro[3.3]heptan-6-yl, 2, 5-diaza-bicyclo[2.2.1]heptan-2-yl, azetidinyl, pyranyl, tetrahydropyranyl, thiopranyl, tetrahydrofuranyl, oxazinyl, dioxo-cyclopentyl, tetrahydroisoquinoline, decahydroisoquinoline, imidazolinyl, imidazolidinyl, quinolizinyl, thiazolidinyl, isothiazolidinyl, isoxazolidinyl, dihydroindolyl, octahydroindolyl, octahydroisoindolyl, pyrrolidyl, pyrazolidinyl, phthaloylimino, etc.

In this application, the term "heterocycloalkyl", as part of a group or other groups, means a stable 3-to 20-membered saturated cyclic group consisting of 2-14 carbon atoms and 1-6 heteroatoms selected from the group consisting of nitrogen, phosphorus, oxygen and sulfur.Unless otherwise specified in this specification, the heterocycloalkyl may either be monocyclic ("monocyclic heterocycloalkyl"), or a bicyclic, tricyclic, or more cyclic ring system, which may include a fused, bridged, or spiro ring system (e. g., a bicyclic system ("bicyclic heterocycloalkyl").The heterocycloalkyl bicyclic ring system may include one or more heteroatoms in one or both rings; and is saturated.For the purpose of the present invention, the heterocycloalkyl is preferably a stable 4-to 12-membered saturated monocyclic, bicyclic, bridged or spiro group comprising 1-3 heteroatoms selected from nitrogen, oxygen and sulfur, and more preferably a stable 4-to 7-membered saturated monocyclic, bicyclic, bridged or spiro group comprising 1-3 heteroatoms selected from nitrogen, oxygen and sulfur.In particular, the 4-to 7-membered heterocycloalkyl group may contain 3, 4, 5 or 6 carbon atoms and one or two above-mentioned heteroatoms or groups containing heteroatoms, provided that the total number of ring atoms is not greater than 7; more particularly, the heterocycloalkyl group may contain 3, 4 or 5 carbon atoms and one or two above-mentioned heteroatoms or groups containing heteroatoms, provided that the total number of ring atoms is not greater than 6 ("4- to 6-membered heterocycloalkyl").

In the present application, as part of a group or other groups, the term "aryl" means a conjugated hydrocarbon ring system group having 6 to 18 carbon atoms, preferably 6 to 10 carbon atoms.For the purpose of the present invention, the aryl group may be a monocyclic, bicyclic, tricyclic or more polycyclic ring system, and may also be fusedwith a cycloalkyl or heterocyclic group as defined above, provided that the aryl group is attached to the rest of the molecule by a single bond via an atom on the aromatic ring. Examples of aryl groups include, but are not limited to, phenyl, naphthyl, anthranyl, phenanthryl, fluorenyl, 2, 3-dihydro-1H-isoindolyl, 2-benzoxazolinone, 2H-1, 4-benzoxazin-3 (4H)-keto-7-yl, etc.

In this application, the term "arylalkyl" refers to an alkyl group as defined above substituted by an aryl group as defined above.

In this application, as part of a group orother groups, the term "heteroaryl" means a 5-to 16-membered conjugate ring system group having 1 to 15 carbon atoms (preferably 1 to 10 carbon atoms) and 1 to 6 heteroatoms selected from the group consisting of nitrogen, oxygen and sulfur.Unless otherwise specifically indicated in this specification, the heteroaryl group may be a monocyclic, bicyclic, tricyclic or more polycyclic ring system, and may also be fused with a cycloalkyl or heterocyclic group as defined above, provided that the heteroaryl group is attached to the rest of the molecule by a single bond via an atom on the aromatic ring.The nitrogen, carbon or sulfur atoms in the heteroaryl group may optionally be oxidized; the nitrogen atoms may optionally be quatemized.For the purpose of the present invention, the heteroaryl group is preferably a stable 5-to 12-membered aromatic group containing 1 to 5 heteroatoms selected from nitrogen, oxygen and sulfur, more preferably a stable 5-to 10-membered aromatic group containing 1 to 4 heteroatoms selected from nitrogen, oxygen and sulfur or a 5-to 6-membered aromatic group containing 1 to 3 heteroatoms selected from nitrogen, oxygen and sulfur.Examples of heteroaryl groups include, but are not limited to, thienyl, imidazolyl, pyrazolyl, thiazolyl, oxazolyl, oxadiazolyl, isoxazolyl, pyridinyl, pyrimidinyl, pyrazinyl, pyridazinyl, benzimidazolyl, benzopyrazolyl, indolyl, furanyl, pyrroyl, triazolyl, tetrazolyl, triazinyl, indolizinyl, isoindolyl, indazolyl, isoindazolyl, purinyl, quinolinyl, isoquinolinyl,diazinaphthyl, naphthyridinyl, quinoxalinyl, pteridyl, carbazolyl, carbolinyl, phenanthridinyl, phenanthrolinyl, acridinyl, phenazinyl, isothiazolyl, benzothiazolyl, benzothiophenyl, oxatriazolyl, cinnolinyl, quinazolinyl, thiophenyl, indolizinyl, o-phenanthroline, isoxazolyl, phenoxazinyl, phenothiazinyl, 4,5,6, 7-tetrahydrobenzo [b] thienyl, naphthyridyl, [1,2,4] triazolo [4,3-b] pyridazine, [1,2,4] triazolo [4,3-a] pyrazine, [1,2,4] triazolo [4,3-c] pyrimidine, [1,2,4] triazolo [4,3-a] pyridine, imidazo [1,2-a] pyridine, imidazo [1,2-b] pyridazine, imidazo [1,2-a] pyrazine, etc.

In this application, the term "heteroarylalkyl" refers to the alkyl group defined above substituted by the heteroaryl defined above.

It should be understood that the singular form used in the present invention, such as "a", includes the plural designation unless otherwise specified.In addition, the term "include" is open-ended and not closed, i.e. it includes what is specified in the present invention, but does not exclude other aspects.

Unless otherwise specified, the present invention uses conventional methods of mass spectrometry and elemental analysis, and the steps and conditions may be referred to the conventional operating steps and conditions in the field.

Unless otherwise specified, the present invention uses standard naming conventionand standard laboratory steps and techniques for analytical chemistry, organic synthetic chemistry, and optics.In some cases, standard techniques are used for chemical synthesis, chemical analysis, and luminescent device performance testing.

Furthermore, it should be noted that, unless otherwise expressly stated, the description "...independently" as used in the present invention should be understood in a broad sense to mean that the individuals described are independent of each other and can be independently of each other for the same or different specific moieties.In more detail, the description "...independently" can either mean that the specific options expressed between the same symbols in different groups do not affect each other, or that the specific options expressed between the same symbols in the same groups do not affect each other.

It will be understood by those skilled in the art that, in accordance with the practice used in the art, " 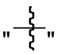 " used in the structural formula of the group described herein means that the corresponding group is linked to other fragments, groups in the compound through that site.

In this application, "optionally" or "optionally" means that a subsequently described event or condition may or may not occur, and the description includes both the occurrence and non-occurrence of the event or condition.For example, "optionally substituted aryl" means that the aryl group is substituted or unsubstituted, and the description includes both the substituted aryl and the unsubstituted aryl.

As used herein, the terms "part", "structural part", "chemical moiety", "group", "chemical group" refer to a specific fragment or functional group in the molecule. Chemical moiety are usually considered as chemical entities embedded or attached to molecules.

A "stereoisomer" refers to a compound composed of the same atoms, bonded by the same bond, but with a different three-dimensional structure. The invention will cover various stereoisomers and mixtures thereof.

When the compound of the present invention contains an olefin double bond, the compound of the present invention is intended to contain E-and Z-geometric isomers unless otherwise specified.

A "tautomer" refers to an isomer formed by the transfer of protons from one atom of the molecule to another atom of the same molecule.All tautomeric forms of the compound of the invention will also be included within the scope of the invention.

The compound of the present invention, or pharmaceutically acceptable salts thereof, may contain one or more chiral carbon atoms and may thus produce enantiomers, diastereomers, and other stereoisomeric forms.Each chiral carbon atom can be defined as (R)-or (S)-based on stereochemistry. The present invention is intended to include all possible isomers, as well as racemates and optically pure forms thereof.The preparation of the compound of the present invention can choose racemates, diastereomers or enantiomers as the raw material or intermediate. Optically active isomers can be prepared using chiral synthons or chiral reagents, or resolution using conventional techniques, such as crystallization and chiral chromatography.

Conventional techniques for preparing/separating individual isomers include chiral synthesis from suitable optically pure precursors, or the use of, for example, chiral high performance liquid chromatography to resolve racemates (or racemates of salts or derivatives), see, for example, Gerald Gübitz and Martin G. Schmid (Eds.), Chiral Separations, Methods and Protocols, Methods in Molecular Biology, Vol. 243, 2004;A.M. Stalcup, Chiral Separations, annu. Rev. Anal. Chem. 3:341-63, 2010;Fumiss et al. (eds.), VOGEL'S ENCYCLOPEDIA OF PRACTICAL ORGANIC CHEMISTRY 5.sup.TH ED., Longman Scientific and Technical Ltd., Essex, 1991, 809-816; Heller, Acc. Chem. Res. 1990, 23, 128.

In this application, the term "the pharmaceutically acceptable salt" includes pharmaceutically acceptable acid addition salts and pharmaceutically acceptable alkali addition salts.

"The pharmaceutically acceptable acid addition salt" refers to a salt formed with inorganic or organic acids that can maintain the bioavailability of free bases without other side effects. Inorganic acid salts include but are not limited to hydrochloride, hydrobromide, sulfate, nitrate, phosphate, etc.; organic acid salts include but are not limited to formate, acetate, 2, 2-dichloroacetate, trifluoroacetate, propionate, caproate, octanoate, decanoate, undecylenate, ethanolate, gluconate, lactate, sebacate, adipate, glutarate, malonate, oxalate, maleate, succinate, fumarate, tartarate, citrate, palmitate, stearate, oleate, cinnamate, laurate, malate, glutamate, pyroglutamate, aspartate, benzoate, methanesulfonate, benzenesulfonate, p-toluenesulfonate, alginate, ascorbate, salicylate, 4-aminosalicylate, naphthalenedisulfonate, etc.These salts can be prepared by methods known in this specialty.

"The pharmaceutically acceptable alkali addition salt" refers to a salt formed with an inorganic or organic base that can maintain the bioavailability of free acid without other side effects. The salts derived from inorganic bases include, but are not limited to, sodium salt, potassium salt, lithium salt, ammonium salt, calcium salt, magnesium salt, iron salt, zinc salt, copper salt, manganese salt, aluminum salt, etc.The preferred inorganic salts are ammonium salt, sodium salt, potassium salt, calcium salt and magnesium salt.Salts derived from organic bases include but are not limited to the

following salts: primary amines, secondary amines and tertiary amines, substituted amines, including natural substituted amines, cyclic amines and basic ion exchange resins, for example, ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucosamine, theobromine, purine, piperazine, piperidine, N-Ethylpiperidine, polyamine resin, etc. Preferred organic bases include isopropylamine, diethylamine, ethanolamine, trimethylamine, dicyclohexylamine, choline, and caffeine. These salts can be prepared by methods known in this specialty.

A "polymorph" refers to the different solid crystalline phases of certain compounds of the present invention due to the presence of two or more different molecular arrangements in the solid state.Certain compounds of the present invention may be present in more than one crystal form, and the present invention is intended to include various crystal forms and mixtures thereof.

In general, crystallization produces solvates of the compound of the invention. The term "solvate" as used in the present invention refers to an aggregate comprising one or more compound molecules of the present invention and one or more solvent molecules.The solvent can be water, in which case the solvate is hydrate. Alternatively, the solvent may be an organic solvent. Therefore, the compound of the present invention may be present as hydrates, including monohydrate, dihydrate, hemihydrate, sesquihydrate, trihydrate, tetrahydrate, etc., and the corresponding solvated forms. The compounds of the present invention can form a real solvate, but in some cases, it is also possible to retain only indefinite water or a mixture of water plus a partial indefinite solvent. The compound of the present invention can be reacted in a solvent or precipitated or crystallized from the solvent.The solvate of the compound of the present invention are also included within the scope of the present invention.

The invention also includes prodrugs of the above compound.In this application, the term "prodrug" refers to a compound that can be converted into the bioactive compound of the present invention under physiological conditions or by solvent decomposition. Thus, the term "prodrug" refers to a pharmaceutically acceptable metabolic precursor of a compound of the present invention. When given to an individual in need, the prodrug may not be active but converted in vivo to the active compound of the present invention.Prodrugs are usually transformed rapidly in the body to produce the parent compound of the present invention, for example by hydrolysis in the blood.Prodrug compounds usually provide the advantages of solubility, histocompatibility or slow release in mammalian organisms.Prodrugs include known amino and carboxyl protecting groups.For specific preparation methods of prodrugs, please refer to Saulnier, m. g., et al., bioorg. med. chem. lett. 1994, 4, 1985-1990;Greenwald, r. B., et al., j. med. chem. 2000, 43, 475.

In this application, "the pharmaceutical composition" refers to a formulation of the compound of the present invention and a medium commonly accepted in the art for delivery of biologically active compound to a mammal, such as a human. The medium comprises a pharmaceutically acceptable carrier.The purpose of the pharmaceutical composition is to promote the administration of organisms, facilitate the absorption of active ingredients and thus exert biological activity.

As used herein, the term "pharmaceutically acceptable" refers to a substance (such as a carrier or diluent) that does not affect the biological activity or properties of the compound of the present invention and is relatively non-toxic, i.e. the substance can be applied to an individual without causing an adverse biological reaction or interacting in an adverse manner with any component contained in the composition.

In this application, "pharmaceutically acceptable carriers" include, but are not limited to, any adjuvants, carriers, excipients, glidants, sweeteners, diluents, preservatives, dyes/colorants, flavoring agents, surfactants, wetting agents, dispersants, suspending agents, stabilizers, isotonic agents, solvents or emulsifiers that are approved byrelevant government management departmentsfor use in humans or livestock.

The "tumor" and "diseases related to abnormal cell proliferation" of the present invention include but are not limited to leukemia, gastrointestinal stromal tumor, histiocytic lymphoma, non-small cell lung cancer, small cell lung cancer, pancreatic cancer, lung squamous cell carcinoma, lung adenocarcinoma, breast cancer, prostate cancer, liver cancer, skin cancer, epithelial cell carcinoma, cervical cancer, ovarian cancer, intestinal cancer, nasopharyngeal cancer, brain cancer, bone cancer, esophageal cancer, melanoma, kidney cancer, oral cancer and other diseases.

As used herein, the terms "prophylactic,""preventing," and "prevention" include reducing the likelihood of a disease or condition occurring or worsening.

The term "therapy" and other similar synonyms as used herein include the following meanings:

(i) preventing the occurenceof a disease or condition in mammals, especially when such mammals are susceptible to the disease or condition but have not been diagnosed as having it;
(ii) suppressing the disease or condition, i.e., arresting its progression;
(iii) alleviating the disease or condition, i.e., causingthe state of the disease or condition to subside; or
(iv) alleviating the symptoms caused by the disease or condition.

As used herein, the terms "effective amount,""therapeutically effective amount," or "pharmaceutically effective

amount," refer to an amount of at least one agent or compound that, when taken, is sufficient to provide some relief of one or more symptoms of the disease or condition being treated. The result may be the abatement and/or remission of signs, symptoms or causes, or any other desired change in the biological system.For example, an "effective amount" for therapeutic use is the amount of a composition comprising a compound disclosed herein that is required to provide clinically significant relief of a condition. The effective amount suitable for any individual case can be determined using techniques such as dose-escalation tests.

As used herein, the terms "taking,""administering," "administration" and the like refer to a method capable of delivering a compound or composition to a desired site for biological action. These methods include, but are not limited to, oral route, transduodenal route, parenteral injection (including intravenous, subcutaneous, intraperitoneal, intramuscular, intra-arterial injection or infusion), topical administration and transrectal administration. Those skilled in the art are familiar with the application techniques that can be used in the compounds and methods described herein, such as those discussed in Goodman and Gilman, The Pharmacological Basis of Therapeutics, current ed.; Pergamon; and Remington's, Pharmaceutical Sciences (current edition), Mack Publishing Co., Easton, Pa.In preferred embodiments, the compounds and compositions discussed herein are administered orally.

As used herein, the terms "drug combination", "combination", "combination of drugs", "administration of other treatments,""administration of other therapeutic agents," and the like refer to drug treatment obtained by mixing or combining more than one active ingredient, which includes a fixed and non-fixed combination of the active ingredient.The term "fixed combination" refers to the simultaneous administration of at least one compound described herein and at least one synergistic agent to a patient in the form of a single entity or a single dosage form. The term "non-fixed combination" refers to the simultaneous administration, co-administration, or sequential administrationat variable intervals of at least one of the compound described herein and at least one synergistic formulation to a patient in the form of a separate entity. These are also applied to cocktail therapies, such as the administration of three or more active ingredients.

It will also be appreciated by those skilled in the art that functional groups of intermediate compound may need to be protected by appropriate protecting groups in the methods described below. Such functional groups include hydroxyl, amino, mercapto and carboxyl. Suitable hydroxyl protecting groups include trialkylsilyl or diarylalkylsilyl (e. g., tert-butyldimethylsilyl, tert-butyldiphenylsilyl, or trimethylsilyl), tetrahydropyran, benzyl, and the like. Suitable protecting groups of amino, amidino and guanidine groups include tert-butoxycarbonyl, benzyloxycarbonyl, etc. Suitable thiol protecting groups include -C(O)-R "(where "R" is alkyl, aryl or aralkyl), p-methoxybenzyl, trityl, etc. Suitable carboxyl protecting groups include alkyl, aryl or aralkyl esters.

The protecting groups may be introduced and removed according to standard techniques known to those skilled in the art and as described herein.The use of protecting group is detailed in Greene, T. W and P. G. M. Wuts, Protective Groups in Organic Synthesis, (1999), 4th Ed., Wiley. The protecting group can also be a polymer resin.

On the basis of not violating the common sense in the art, the above-mentioned preferred conditions can be combined arbitrarily, that is, each preferred example of the present invention can be obtained.

The reagents and raw materials used in the invention are commercially available.

The positive effect of the present invention is to provide the benzothiazolyl biaryl compound, which can be used as a $KRAS^{G12C}$inhibitor; It can be used to prepare anti-tumor drugs, prevent and/or treat tumors.

## Detailed Description of the Invention

After long-term and in-depth research, the inventor prepared a class of benzothiazolyl biaryl compound with novel structure shown in Formula I, and found that they have better inhibitory activity against $KRas^{G12C}$ protein, and the compound has a specific inhibitory effect on $KRas^{G12C}$protein at a very low concentration (as low as less than 100nM), moreover, it has excellent inhibitory activity on cell proliferation related to $KRas^{G12C}$ and downstream signal pERK, so it can be used to treat related diseases caused by mutation or abnormal expression of $KRas^{G12C}$, such as tumors.The present invention has been completed on this basis.

Hereinafter, the present invention will be further described by way of examples, but the present invention is not limited to the scope of the described examples.In the following examples, the test methods without specific conditions are usually in accordance with conventional conditions, or in accordance with the commodity instructions.In the following examples, the test methods without specific conditions are usually in accordance with conventional conditions or the conditions recommended by the manufacturer.Unless otherwise specified, percentages or parts are percentages by weight or parts by weight.

### Preparation method I of intermediate: synthesis of quinoline/quinazoline compounds

With reference to the synthetic routes and methods of patented WO2019110751A1, WO2019014402A1 and WO201367597A1, the quinoline/quinazoline intermediate compound 1A-1D isprepared.

| No. | Structure | LC-MS | No. | Structure | LC-MS |
|---|---|---|---|---|---|
| 1A | | 329.1/331.1 | 1B | | 353.1/355.1 |
| 1C | 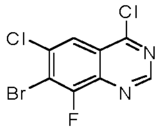 | 295.2/297.2 | 1D | 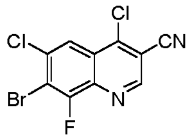 | 319.1/321.1 |

**General Preparation Method III of Example**

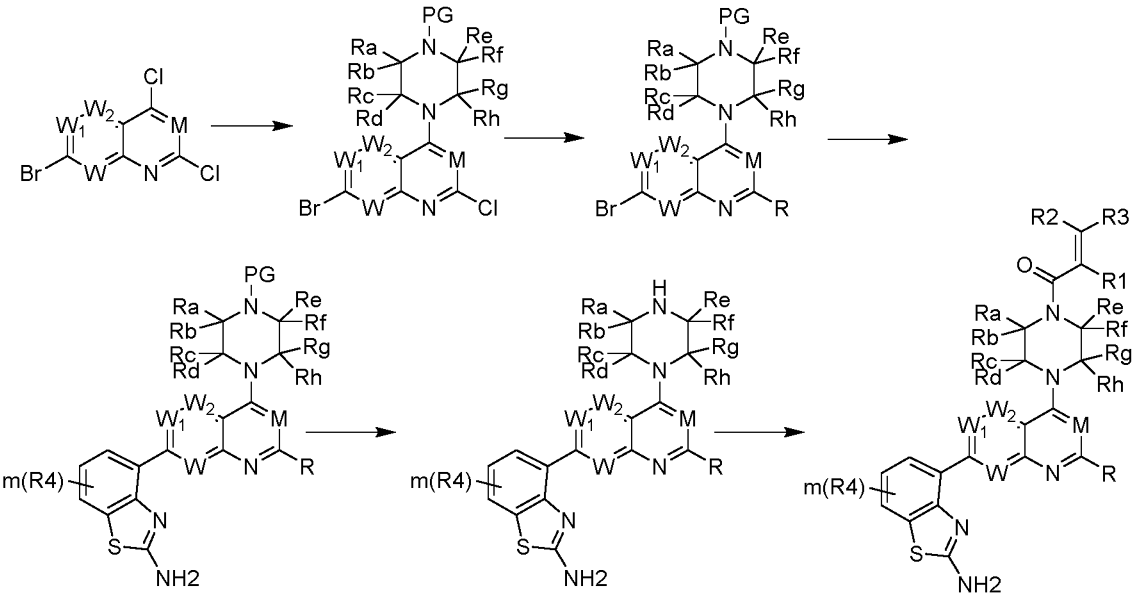

Step 1: A quinoline intermediate (1 eq.) was dissolved in tetrahydrofuran, followed by the addition of DIPEA(1.6 eq.) and piperazine intermediate (1.5 eq.), then heated to 60 degrees under nitrogen protection and stirred for 12 hours.The reaction was monitored completely by TLC, cooled to room temperature, concentrated under reduced pressure, the residue was added into water and dichloromethane to separate the phase, the aqueous phase was extracted three times with dichloromethane, the extract was dried with anhydrous sodium sulfate, concentrated under reduced pressure, and the residue was separated and purified by silica gel column chromatography to obtain the target product, and the structure is confirmed by NMR and mass spectrometry.

Step 2: The product of the step 1 above (1 eq.) was dissolved in an appropriate solvent, under the nitrogen protection, raw materials such as alcohol and amine were added, and heated and stirred for 12 hours.The reaction was monitored completely by TLC, cooled to room temperature, poured into saturated aqueous ammonium chloride solution andsolid precipitated. After filtraion, the filter cake was vacuum dried to obtain the target product, and the structure was confirmed by NMR and mass spectrometry.

Step 3: The product of the above step 2 (1 eq.) was dissolved in a mixture of anhydrous dioxane/water (4/1), followed by the addition ofbenzothiazolylboronic acid or boronic acid pinacol ester (2 eq.), anhydrous potassium carbonate powder (2.5 eq.) and Pd(dppf)$Cl_2$ (0.1 eq.), then heated to reflux for 2 h under nitrogen protection. The reaction was monitored completely by TLC, cooled to room temperature, concentrated under reduced pressure, the residue was diluted with dichloromethane, washed with saturated ammonium chloride solution and saturated salt water in turn, dried with anhydrous sodium sulfate, filtered, concentrated under reduced pressure, and the residue was separated and purified by silica gel column chromatography to obtain the target product, and the structure was confirmed by NMR and mass spectrometry.

Step 4: The product of step 3 above (1 eq.) was dissolved in methanol, and 4 M HCl methanol solution (20 eq.) was added, and the Boc protecting group was removed by stirring for 3 h at room temperature; or the Cbz protecting group was removed by palladium-carbon catalysis under hydrogen atmosphere. The reaction was monitored completely

by TLC, concentrated under reduced pressure, and the residue was used directly in the next reaction step.

Step 5: The residue of the previous step was dissolved in dichloromethane, followed by the addition of DIPEA (3 eq.) and acryloyl chloride (1 eq.) at 0 degrees. The reaction solution was stirred for 0.5 h, then washed with saturated ammonium chloride solution and saturated saline water, dried with anhydrous sodium sulfate, filtered, concentrated under reduced pressure, and the residue was purified by silica gel column chromatography to obtain the target compound, and the structure was confirmed by NMR and mass spectrometry.

**Example 1**:1-(4-(7-(2-amino -7-fluorobenzo [d] thiazol-4-yl) -6-chloro-8-fluoro-2-(((S)-1-methylpyrrolin-2-yl) methoxy) quinazolin-4-yl) piperazin-1-yl) prop-2-en-1-one

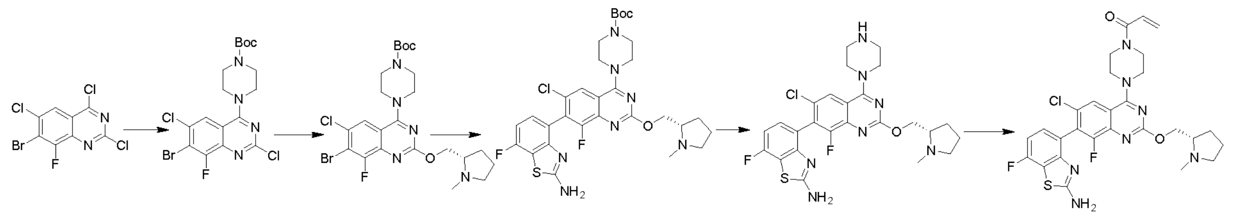

Step 1: 7-Bromo- 8-fluoro-2 ,4 ,6-trichloroquinazoline (984 mg, 3 mmol), DIPEA (580 mg, 4.5 mmol), N-Boc-piperazine (558 mg, 3 mmol) were dissolved in N,N-dimethylformamide (DMF) (15 mL) and the reaction was carried out under Ar protection by heating to 60 °C for 20 hours.The complete reaction of the raw materials was monitored by TLC, and after the reaction solution was cooled to room temperature, water (20 mL) was added and extracted with ethyl acetate (10 mL*2), the organic phases were combined, washed with saturated sodium chloride, the organic phases were concentrated and purified by column chromatography to give tert-butyl 4-(7-bromo-2,6-dichloro-8-fluoroquinazolin-4-yl)piperazine-1-carboxylate ester (white solid, 836 mg), ESI-MSm/z:479.1/481.1[M+H]+.$^1$H-NMR (400 MHz, $CDCl_3$) $\delta$:7.76 (d, *J*= 1.8 Hz, 1H), 3.90-3.87 (m, 4H), 3.67-3.64 (m, 4H), 1.49 (s, 9H).

Step 2: To a solution of compound tert-butyl 4-(7-bromo-2,6-dichloro-8-fluoroquinazolin-4-yl)piperazine-1-carboxylate ester(316 mg ,0.62 mmol) in dimethyl sulfoxide (DMSO) (10 mL) at room temperature was added raw material (S)-(1-methylpyrrolidin-2-yl)methanol (163 mg ,1.24 mmol) and potassium fluoride (KF) (290 mg ,4.96 mmol).The reaction mixture was stirred at 120 °C under argon for 16 h. After completetion of the reaction, the mixture was poured into ice waterand extracted with ethyl acetate, then the organic layer was washed with saturated saline, dried over anhydrous sodium sulfate ($Na_2SO_4$), concentrated under reduced pressure and purified by rapid column chromatography on silica gel to give tert-butyl (S)-4-(7-bromo-6-chloro-8-fluoro-2-((1-methylpyrrolidin-2-yl)methoxy)quinazolin-4-yl)piperazine-1-carbox ylate ester(180 mg, white solid).ESI-MS m/z:558.1/560/1[M+H]$^+$.$^1$H NMR (400 MHz, DMSO-$d_6$): $\delta$ 8.09 (s, 1H), 3.93 (s, 4H), 3.56 (s, 4H), 1.43 (s, 9H).

Step 3: To a solution of tert-butyl (S)-4-(7-bromo-6-chloro-8-fluoro-2-((1-methylpyrrolidin-2-yl)methoxy)quinazolin-4-yl)piperazine-1-carbox ylate ester(112 mg, 0.2 mmol) in 1 ,4-dioxane/water (12 mL/4 mL) at room temperature was added fluoro-substituted benzothiazolylboronic acid material(178 mg, 0.1 mmol), tetrakis(triphenylphosphine)palladium (24 mg ,0 .02 mmol) and sodium carbonate powder ($Na_2CO_3$) (108 mg ,1 mmol), and the reaction mixture was stirred at 100 °C under argon overnight.After the completion of the reaction, the mixture was extracted with ethyl acetate and the organic phase was washed with saturated saline, dried over anhydrous $Na_2SO_4$, filtered, concentrated under reduced pressure and purified by rapid column chromatography on silica gel to give tert-butyl 4-(7-(2-amino-7-fluorobenzo [d]thiazol-4-yl)-6-chloro-8-fluoro-2-(((S)-1-methylpyrrolidin-2-yl)methoxy)qui nazolin-4-yl)piperazin-1-carboxylate (86 mg, yellow solid).ESI-MS m/z:646.2/648.2[M+H]$^+$.

Step 4: To a stirred solution of tert-butyl 4-(7-(2-amino-7-fluorobenzo[d]thiazol-4-yl)-6-chloro-8-fluoro-2-(((S)-1-methylpyrrolidin-2-yl)methoxy)qui nazolin-4-yl)piperazine-1-carboxylate(83 mg ,0 .13 mmol) in dichloromethane (DCM) (6 mL) under ice bath cooling was added trifluoroacetic acid ($CF_3COOH$, TFA) (3 mL) and the reaction solution was warmed up to room temperature and stirred for 2h.After completion of the reaction, the mixture wasconcentrated under reduced pressure and the residue wasused directly in the next step without further purification.

Step 5: To a stirred solution of 4-(6-chloro-8-fluoro-2-(((S)-1-methylpyrrolidin-2-yl)methoxy)-4-(piperazin-1-yl)quinazolin-7-yl)-7-fluorob enzo[d]thiazol-2-amine (65 mg) in DCM (10 mL) was added triethylamine ($Et_3N$) (66 mg, 0.66 mmol) and cooled to 0 °C, then acryloyl chloride (18.1 mg, 0.144 mmol) was added.The reaction mixture was slowly warmed to room temperature and the reaction was monitored by LC-MS showing reactioncompleted, quenched with saturated sodium bicarbonate (NaHCOs) (10 mL), extracted with ethyl acetate, washed with water, dried over $Na_2SO_4$, concentrated and purified by reversed-phase preparative chromatography to give 1-(4-(7-(2-amino-7-fluorobenzo[d]thiazol-4-yl)-6-chloro-8-fluoro-2-(((S)-1-methylpyrrolidin-2-yl)methoxy) quinazolin-4-yl)piperazin-1-yl)prop-2-en-1-one (13

mg, yellow powder), ESI-MS m/z:600.1/602.1[M+H]+.$^1$H NMR (400 MHz, MeOD-$d_4$): $\delta$ 7.95 (s, 1H), 7.31-7.16 (m, 1H), 6.99 (t, 1H), 6.82 (dd, 1H), 6.28 (d, 1H), 5.81 (d, 1H), 4.56-4.38 (m, 2H), 4.01-3.95 (m, 8H), 3.10 (s, 1H), 2.81 (s, 1H), 2.53 (s, 3H), 2.37 (d, 1H), 2.25-1.64 (m, 4H).

**Example 2:** 1-(4-(7-(2-amino-7-fluorobenzo[d]thiazol-4-yl)-6-chloro-8-fluoro-2-((((S)-1 -methylpyrrolidin-2-yl)methyl)amino)quinazolin-4-yl)piperazin-1 -yl)prop-2-en-1 -one

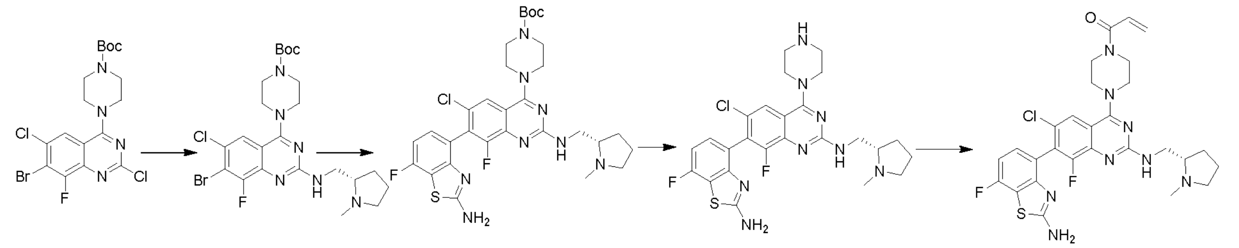

Example compound 2 (light yellow solid, 12 mg) was obtained by the same method as in Example 1 using (S)-1-methylpyrrolidinylmethylatnine as the raw material.ESI-MS m/z:599.1/601.1[M+H]$^+$.

**Example**

**3**:4-(4-Piperazinylacryloylpiperazin-1-yl)-7-(2-amino-7-fluorobenzo[d]thiazol-4-yl)-6-chloro-8-fluoro-2-(((S )-1 -methylpyrrolidin-2-yl)methoxy)quinoline-3-cyano

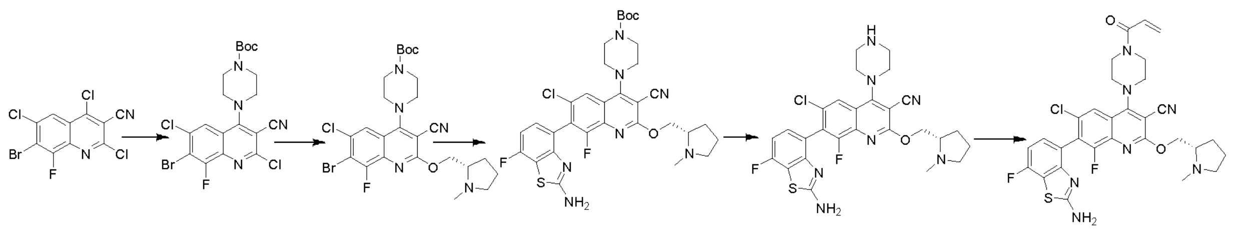

Example 3 (white solid, 13 mg) was prepared by the same method as Example 1 using intermediate 1B as raw material.ESI-MS m/z:624.1/626.1[M+H]+.$^1$H-NMR (400 MHz, MeOD-$d_4$): $\delta$ 7.96 (s, 1H), 7.20-7.24 (m, 1H), 6.97-7.02 (m, 1H), 6.29 (dd, 1H), 5.82 (dd, 1H), 4.74-4.88 (m, 1H), 4.48-4.54 (m, 1H), 3.96-3.98 (m, 4H), 3.72-3.78 (m, 4H), 3.29-3.34 (m, 2H), 2.78 (s, 3H), 2.69-2.78 (m, 1H), 2.19-2.26 (m, 1H), 1.85-2.00 (m, 3H).

**Example** 4: 4-(4-Piperazinylacryloylpiperazin-1-yl)-7-(2-amino-7-fluorobenzo[d]thiazol-4-yl)-6-chloro-8-fluoro-2-((((S)-1-methylpyrrolin-2-yl)methyl)amino)quinoline-3-cyano

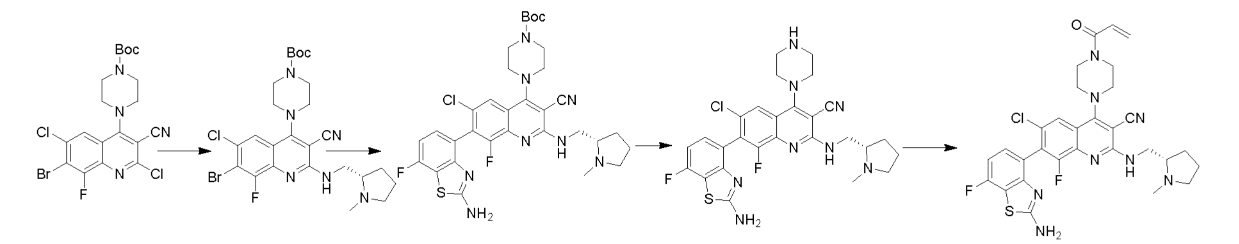

Example compound 4 (light yellow solid, 5 mg) was prepared using the same method as Example 3.ESI-MS m/z:623.1/625.1 [M+H]$^+$.

**Example**

**5**:1-(4-(7-(2-amino-7-fluorobenzo [d]thiazol-4-yl)-6-dichloro-2-(2-(dimethylatnino)ethoxy)-8-fluoroquinolin-4-yl)piperazin-1 -yl)prop-2-en-1 -one

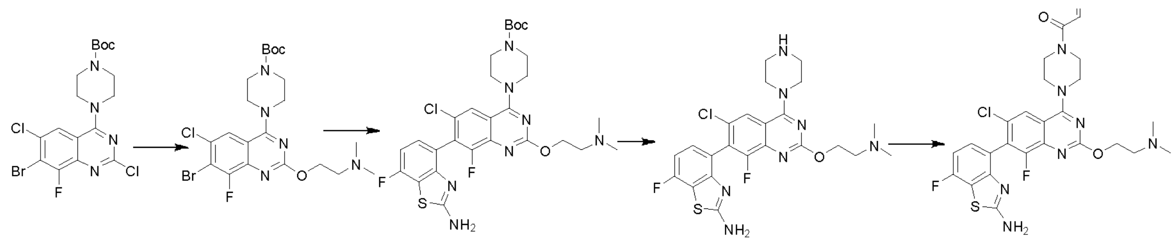

Example compound 4 (light yellow solid, 10 mg) was prepared using the same method as Example 3.ESI-MS m/z:574.2/576.1[M+H]$^+$.$^1$H-NMR (400 MHz,$CD_3OD$): $\delta$ 8.05 (d, 1H), 7.27-7.23 (m, 1H), 7.05-7.01 (m, 1H), 6.81 (dd, 1H), 6.29 (dd, 1H), 5.82 (dd, 1H), 4.16-4.10 (m, 4H), 3.97-3.90 (m, 4H), 3.66-3.64 (m, 2H), 3.33-3.31 (m, 2H).

**Example 6**:1-(4-(7-(2-amino-7-fluorobenzo [d] thiazol-4-yl)-2,6-dichloro-8-fluoroquinazolin-4-yl) piperazin-1-yl) prop-2-ene-1-one

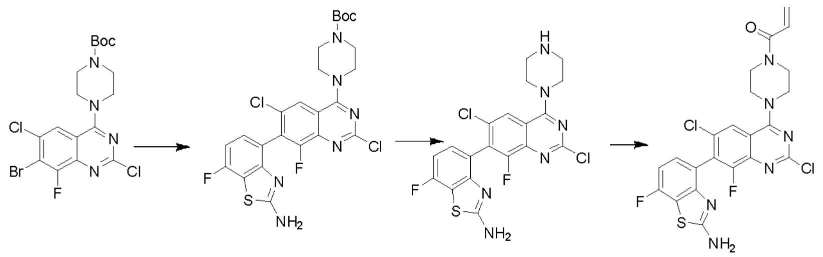

Example compound 4 (light yellow solid, 23 mg) was prepared using the same method as Example 3.ESI-MS m/z:521.2/523.1[M+H]$^+$.$^1$H-NMR (400MHz, DMSO-d6) $\delta$ 8.15(s, 1H), 7.94 (bs,2H) , 7.23(dd, J=8.4, 5.6Hz, 1H), 7.09(t, J=8.8Hz, 1H), 6.71-6.83(m, 1H), 6.27(dd, J=16.8, 2.0Hz, 1H), 5.73(dd, J=10.8, 2.0Hz, 1H), 3.62-3.73(m, 4H), 3.31-3.48(m, 4H).

**Example** 7: 4-(4-piperazinylacrylpiperazin-1-yl)-7-(2-amino-7-fluorobenzo[d]thiazol-4-yl)-2,6-dichloro-8-fluoroquinolin -3-cyano

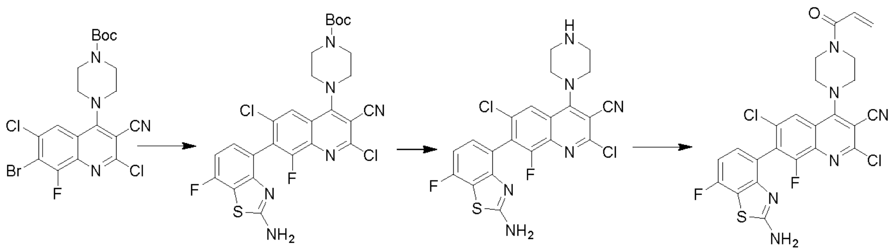

Example compound 4 (light yellow solid, 8 mg) was prepared using the same method as Example 3.ESI-MS m/z:545.2/547.1[M+H]$^+$.$^1$H-NMR (400MHz, DMSO-d6) $\delta$ 8.16(s, 1H), 7.93 (bs,2H) , 7.22(dd, J=8.4, 5.6Hz, 1H), 7.10(t, J=8.8Hz, 1H), 6.76-6.83(m, 1H), 6.25(dd, J=16.8, 2.0Hz, 1H), 5.78(dd, J=10.8, 2.0Hz, 1H), 3.52-3.64(m, 4H), 3.33(t,

J=5.4Hz, 4H).

**Example** 8: 4-(4-acryloylpiperazin-1-yl)-7-(2-amino-7-fluorobenzo[d]thiazol-4-yl)-6-chloro-8-fluoroquinolin-3-cyano

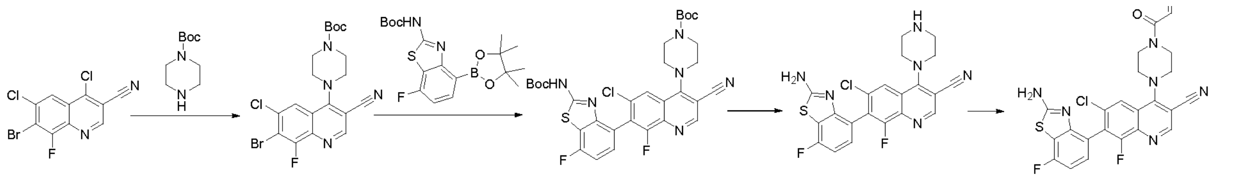

Step 1: A quinoline material (209 mg, 0.65 mmol) was dissolved in DMF (5 mL), followed by the addition of N-Boc piperazine (133.6 mg, 0.72 mmol) and DIPEA (252.7 mg, 1.96 mmol), stirred for 16 h, poured into 100 mL of water and extracted with ethyl acetate (50 mL*3), washed with saturated saline, dried, and concentrated by column chromatography to give tert-butyl 4-(7-bromo-6-chloro-3-cyano-8-fluoroquinolin-4-yl)piperazine-1-carboxylate (yellow solid, 249 mg).LC-MS[M H]: m/z 471.0/473.0.$^1$H-NMR (400 MHz, DMSO-$d_6$): $\delta$ 8.86 (s, 1H), 8.09 (s, 1H), 3.67 (d, 4H), 3.62 (d, 4H), 1.45 (s, 9H).

Step 2: The intermediate (50 mg, 0.11 mmol) obtained from the previous step and boronic acid ester (54.8 mg, 0.14 mmol) were dissolved in dioxane/water (1.5 mL/0.45 mL), under the nitrogen protection, 1,1'-Bis(di-tert-butylphosphino)ferrocene palladium dichloride (7 mg, 0.011 mmol) and potassium phosphate ( $K_3PO_4$) (34 mg, 0.16 mmol) were added, reacted at 90 °C for 2 h, poured into 100 mL of water and extracted with ethyl acetate (50 mL*3), washed with saturated salt water and dried to give a reddish-brown solid crude product (45 mg), which was directly used in the next step.LC-MS[M H]: m/z 657.2/659.2.

Step 3: The crude product (45 mg) from the previous step was dissolved in dichloromethane (5 mL), followed by the addition of TFA (1 mL) and stirred at room temperature for 3 h. After concentration, TFA was removed to obtain a yellow solid (35 mg), which was used directly in the next step.LC-MS[M H]: m/z 457.0/459.0.

Step 4: The yellow crude compound (35 mg, 0.077 mmol) from the previous step was dissolved in THF/$H_2O$ (4 mL/2 mL), followed by the addition of $K_3PO_4$ (654 mg, 0.154 mmol), cooled to 0 degree, added acryloyl chloride (6.95 mg, 0.077 mmol) dropwise, stirred for 1h, poured into 20 mL of water and extracted with ethyl acetate (20 mL*3), washed with saturated salt water, dried, concentrated, and purified by liquid phase preparative chromatography to give Example 8 (white solid, 21 mg).LC-MS[M H]: m/z 511.1/513.1.$^1$H NMR (400 MHz, MeOD-$d_4$): $\delta$ 8.79 (s, 1H), 8.11 (d, 1H), 7.28 (dd, 1H), 7.12-6.92 (m, 1H), 6.87 (dd, 1H), 6.30 (dd, 1H), 5.83 (dd, 1H), 4.09-3.92 (m, 4H), 3.86 (s, 4H).

**Example 9**:7-(2-amino-7-fluorobenzo [d] thiazol-4-yl) -6-chloro-8-fluoro-4-(4-(2-fluoroacryloyl) piperazin-1-yl) quinolin-3-cyano

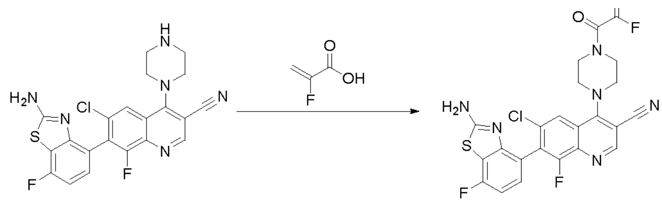

The compound 7-(2-amino-7-fluorobenzo[d]thiazol-4-yl)-6-chloro-8-fluoro-4-(piperazin-1-yl)quinolin-3-cyano (17.3 mg, 0.038 mmol) was dissolved in DMF (2 mL), followed by the addition of fluoroacrylic acid (3.4 mg, 0.038 mmol), 2-(7-azabenzotriazol-1-yl)-N,N,N' N'-tetramethyluronium hexafluorophosphate (HATU) (18.8 mg, 0.049 mmol), N,N-diisopropylethylamine (DIEA) (24.51 mg, 0.19 mmol), stirred for 1 h at room temperature, poured into 20 mL of water and extracted with ethyl acetate (20 mL*3), washed with saturated salt water, dried, concentrated and conducted preparative separation to give Example 9 compound (gray solid, 2.8 mg).LC-MS[M H]: m/z 529.0/531.0.$^1$H-NMR (400MHz, MeOD-$d_4$): $\delta$ 8.79 (s, 1H), 8.11 (d, 1H), 7.27 (dd, 1H), 7.03 (t, 1H), 5.38-5.26 (m, 2H), 3.97 (d, 4H), 3.86 (d, 4H).

**Example**

**10**:4-((S)-4-acryloyl-2-methylpiperazin-1-yl)-7-(2-amino-7-fluorobenzo [d]thiazol-4-yl)-6-chloro-8-fluoroqui nolin-3-cy-ano

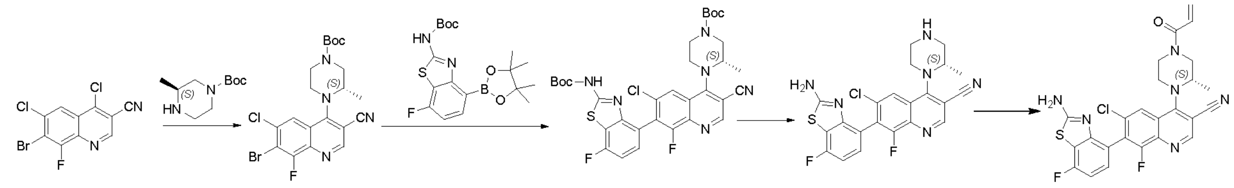

Step 1: 7-bromo-4,6-dichloro-8-fluoroquinolin-3-cyano (100 mg, 0.3 mmol) was dissolved in DMF (20 mL), tert-butyl (S)-3-methylpiperazine-1-carboxylate (66.0 mg, 0.33 mmol) and DIEA (116.1 mg, 0.9 mmol) were added and the reaction was carried out overnight at room temperature. The reaction was detected to be finished and spun dry DMF, then dissolved with ethyl acetate (30 mL) and separated by column chromatography (PE/EA=10/1, petroleum ether/ethyl acetate volume ratio) to give tert-butyl (S)-4-(7-bromo-6-chloro-3-cyano-8-fluoroquinolin-4-yl)-3-methylpiperazin-1-carboxylate ester (white solid, 100 mg).LC-MS[M H]: m/z 484.8.

Step 2: The compound (50.0 mg, 0.1 mmol) from the previous step, benzothiazole borate (54.0 mg, 0.13 mmol) were dissolved in dioxane/$H_2O$ (30 mL /10 mL), and potassium phosphate (32.1 mg, 0.15 mmol), 1,1'-bis(di-tert-butylphosphino)ferrocene palladium dichloride (Pd( dtbpf)$Cl_2$) (7.0 mg, 0.01 mmol) were added, reacted at 90 °C for 4 h under nitrogen protection, cooled down, mixed directly and separated by column chromatography (PE/EA=3/1) to give tert-butyl (3S)-4-(7-(2-((tert-butoxycarbonyl)amino)-7-fluorobenzo[d]thiazol-4-yl)-6-chloro-3-cyano-8-fluoroquinolin-4-yl) -3-methylpiperazin-1-carboxylate (white solid, 40.0 mg).LC-MS[M H]: m/z 670.9.

Step 3: The compound (40.0 mg, 0.06 mmol) from the previous step was dissolved in DCM (5 mL), TFA (1 mL) was added, and stirred at room temperature overnight. The reaction was detected completely by LCMS and spun dry to obtain a yellow crude solid (40.0 mg).LC-MS[M H]: 471.1 *m/z* .

Step 4: The yellow crude product (40 mg, 0.09 mmol) from the previous step was dissolved in THF/water (6 mL / 2 mL), followed by the addition of$K_3PO_4$ (38.0 mg, 0.18 mmol)andacryloyl chloride (10.0 mg, 0.099 mmol), then stirred at room temperature for 1 hour.10 mL of water was added, extracted with EA, dried over MgSO4, filtered, spun dry, and conducted preparative separation to give the compound of Example 10 (yellow solid, 10.0 mg).LC-MS[M H]: *m*/*z*. 525.2/527.2.$^1$H NMR (400 MHz, DMSO-$d_6$): $\delta$8.90 (s, 1H), 8.10 (s, 1H), 7.96 (s, 2H), 7.27-7.29 (m, 1H), 7.07-7.12 (m, 1H), 6.85-6.89 (m, 1H), 6.16-6.21 (m, 1H), 5.73-5.77 (m, 1H), 4.02-4.06 (m, 4H), 3.81-3.99 (m, 3H), 1.26 (d, 3H).

**Example 11**:1-((2R)-4-(7-(2-amino-7-fluorobenzo [d] thiazol-4-yl) -6-chloro-8-fluoro-2-(((S)-1-methylpyrrolidin-2-yl) methoxy) quinazolin-4-yl)-2-methylpiperazin-1-yl) prop -2-en-1-one

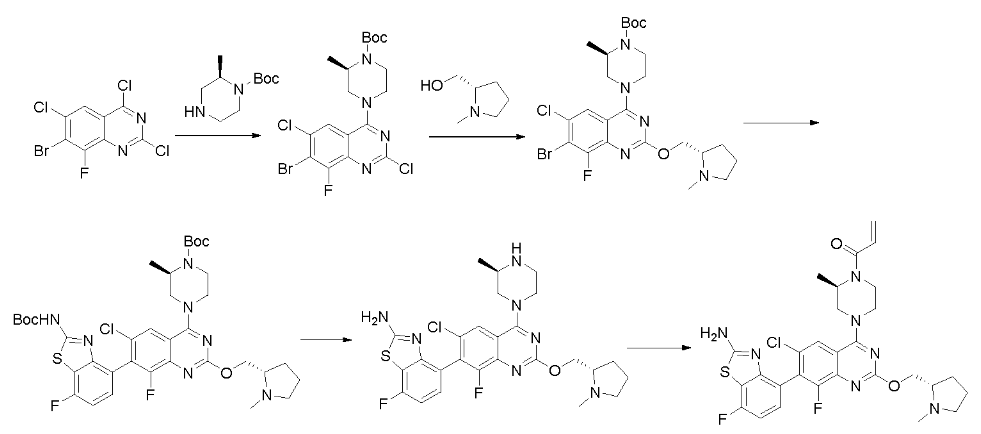

Example 11 (white solid, 6.5 mg) was prepared with reference to the synthesis method of Example 3. LC-MS[M+H]$^+$: m/z 614.1/616.1.$^1$H NMR (400 MHz, MeOD-$d_4$): $\delta$8.04 (s, 1H), 7.22-7.26 (m, 1H), 6.99-7.04 (m, 1H), 6.76-6.83

(m, 1H), 6.28 (d, 1H), 5.80 (d, 1H), 4.89-4.94 (m, 1H), 4.46-4.67 (m, 2.5H), 4.01-4.4 (m, 2H), 3.81-3.98 (m, 2.5H), 3.64-3.75 (m, 3H), 3.24-3.31 (m, 1H), 3.08 (s, 3H), 2.36-2.41 (m, 1H), 2.05-2.22 (m, 3H), 1.34 (d, 3H).

**Example 12:** 2-(1-acryloyl -4-(7-(2-amino-7-fluorobenzo [d] thiazol-4-yl) -6-chloro-8-fluoro-2-(((S)-1-methylpyrrolidin-2-yl) methoxy) quinazolin-4-yl) piperazin-2-yl) acetonitrile

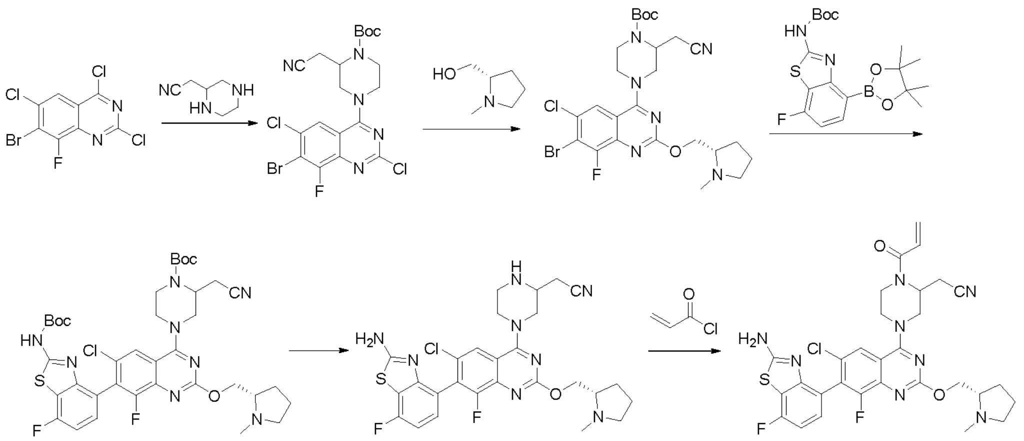

**Step 1:** Dichloroquinazoline raw material (200 mg, 0.61 mmol), 2-piperazine acetonitrile (76 mg, 0.61 mmol), DIEA (236 mg, 1.83 mmol) were dissolved in DMF (3 mL).The reaction was carried out overnight at room temperature under nitrogen protection.Then $Boc_2O$ (266 mg, 1.22 mmol) was added dropwise and reacted at room temperature for 3 h.The mixture was poured into 20 mL of water and extracted with ethyl acetate (20 mL*3), washed with saturated salt water, dried, concentrated and separated by column chromatography (PE:EA=3:1) to give a green oilysubstance(208 mg).

Step 2: The oily substance (430 mg, 0.83 mmol) from the previous step, (S)-(1-methylpyrrolin-2-yl)methanol (287 mg, 2.50 mmol), DIEA (645 mg, 5.00 mmol) were dissolved in N-methylpyrrolidone (NMP) (5 mL) and reacted at 50 °C for 3 days under nitrogen protection. The mixture was diluted with 20 mL of water and extracted with ethyl acetate (20 mL*3), washed with saturated saline, dried, concentrated and separated by column chromatography (DCM:Me-OH=10:1) to give a pale yellow oily substance (101 mg).LC-MS[M H]: m/z 597.1.

Step 3: The oily substance (95 mg, 0.16 mmol) from the previous step and benzothiazole borate (63 mg, 0.20 mmol) were dissolved in dioxane/water (3 mL/1 mL), under nitrogen protection, followed by the addition of 1,1'-bis(di-tert-butylphosphino)ferrocene palladium dichloride (13 mg, 0.02 mmol) and $K_3PO_4$ (50 mg, 0.24mmol), reacted at 90 °C for 3 h, poured into 100 mL of water and extracted with ethyl acetate (50 mL*3), washed with saturated saline, dried, concentrated and separated by column chromatograph (DCM:MeOH=10:1) to give a yellow oil (50 mg).LC-MS[M H]: m/z 785.2.

Step 4: The oil (50 mg) from the previous step was dissolved in dichloromethane (5 mL), and TFA (1.5 mL) was added, stirred at room temperature for 1 h.Afterconcentration, TFA was removed to give a yellow solid (37 mg), which was directly used in the next step.LC-MS[M H]: m/z 585.1.

Step 5: The yellow solid (37 mg, 0.06 mmol) from the previous step was dissolved in THF/$H_2O$ (4 mL/2 mL), followed by the addition of $K_3PO_4$ (50 mg, 0.24 mmol) , cooled to 0 degree, and acryloyl chloride (4 mg, 0.05 mmol) was added dropwise, stirred for 1 h, poured into 20 mL of water and extracted with ethyl acetate (20 mL*3), washed with saturated salt water, dried, concentrated, and prepared by reversed-phase column to give Example 12 (pale yellow solid, 3.3 mg).LC-MS[M H]:m/z 639.1/641.1.$^1$H NMR (400 MHz,$CD_3OD$): $\delta$ 8.05 (s, 1H), 7.24-7.21 (m, 1H), 7.03-6.99 (m, 1H), 6.80-6.79 (m, 1H), 6.33-6.29 (m, 1H), 5.85-5.82 (m, 1H), 5.18-5.06 (m, 1H), 4.80-4.48 (m, 5H), 4.35-3.69 (m, 6H), 3.16-2.98 (m, 5H), 2.43-2.37 (m, 1H), 2.22-2.03 (m, 3H).

**Example 13**:1-((3 S)-4-(7-(2-amino-7-fluorobenzo [d] thiazol-4-yl) -6-chloro-8-fluoro-2-(((S)-1-methylpyrrolidin-2-yl) methoxy) quinazolin-4-yl)-3-methylpiperazin-1-yl) prop -2-en-1-one

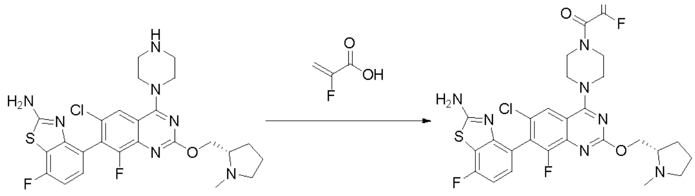


4-(6-chloro-8-fluoro-2-(((S)-1-methylpyrrolidin-2-yl)methoxy)-4-(piperazin-1-yl)quinazolin-7-yl)-7-flu orobenzo[d]thiazol-2-amine (24 mg, 0.045 mmol) was dissolved in DMF (1.5 mL), followed by the addition of fluoroacrylic acid (6.1 mg, 0.067 mmol), HATU (26 mg, 0.067mmol), and DIEA (17 mg, 0.14 mmol) in sequence, stirred at room temperature for 1 h, poured into 20 mL of water and extracted with ethyl acetate (20 mL*3), washed with saturated saline, dried, concentrated and conducted preparative separation (acid method) to give Example 13 (white solid, 11.6 mg).LC-MS[M H]:m/z 618.1/620.2.$^1$H-NMR (400 MHz, MeOD-$d_4$): $\delta$ 8.02 (s, 1H), 7.21-7.25 (m, 1H), 7.02 (dd, 1H), 5.36 (dd, 1H), 5.28 (dd, 1H), 4.91-4.92 (m, 1H), 4.62-4.67 (m, 1H), 4.06-4.11 (m, 4H), 3.89-3.94 (m, 5H), 3.71-3.75 (m, 1H), 3.08 (s, 3H), 2.36-2.41 (m, 1H), 2.05-2.22 (m, 3H).

**Example** 14: 7-(2-amino-7-fluoro-fluorophenyl[d]thiazol-4-yl)-6-chloro-8-fluoro-4-(4-(2-fluoroacryloyl) piperazin-1-yl)-2-(((S)-1-methylpyrroline-2-yl) methoxy) quinolin-3-cyano

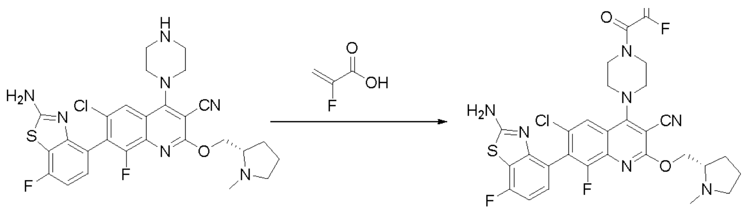

7-(2-amino-7-fluoro fluorophenyl[d]thiazol-4-yl)-6-chloro-8-fluoro-2-(((S)-1-methylpyrrolin-2-yl)methoxy)-4-(piperazin-1-yl)qu inolin-3-cyano (26 mg, 0.045 mmol) was dissolved in DMF (1.5 mL), followed by the addition of fluoroacrylic acid (6.1 mg, 0.067 mmol), HATU (26 mg, 0.067 mmol), and DIEA (17 mg, 0.14 mmol) in sequence, stirred at room temperature for 1 h, poured into 20 mL of water and extracted with ethyl acetate (20 mL*3), washed with saturated salt water, dried, concentrated and conducted preparative separation (acid method) to give Example 14 (white solid, 12.1 mg).LC-MS[M H]: m/z 642.1/644.1.$^1$H-NMR (400 MHz, MeOD-$d_4$): $\delta$ 8.09 (s, 1H), 7.98 (s, 2H), 7.23-7.28 (m, 1H), 7.05-7.11 (m, 1H), 5.36 (dd, 1H), 5.28 (dd, 1H), 4.91-4.92 (m, 1H), 4.62-4.67 (m, 1H), 4.06-4.11 (m, 4H), 3.89-3.94 (m, 5H), 3.71-3.75 (m, 1H), 3.08 (s, 3H), 2.36-2.41 (m, 1H), 2.05-2.22 (m, 3H).

**Example 15:** 4-(4-acryloyl -3-(cyanomethyl) piperazin-1-yl)-7-(2-amino-7-fluorobenzo [d] thiazol-4-yl)-6-chloro-8-fluoroquinolin-3-cyano

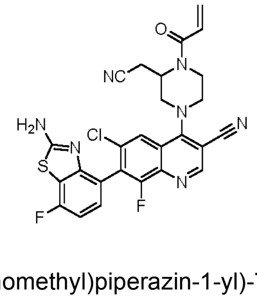

, the example compound 4-(4-acryloyl-3-(cyanomethyl)piperazin-1-yl)-7-(2-amino-7-fluorobenzo[d]thiazol-4-yl)-6-chloro-8-fluoroqu inolin-3-cyano (yellow solid, 14.0 mg) was prepared by the same synthetic method as in Example 10 using cyanomethyl piperazine compound as raw material.LC-MS[M H]: *m/z* 550.0/552.0.$^1$H-NMR (400 MHz, MeOD-$d_4$): $\delta$ 8.83 (s, 1H), 8.16 (s, 1H), 7.29-7.31(m, 1H), 7.25 (dd, 1H), 6.89-6.95 (m, 1H), 6.31-6.35 (m, 1H), 5.85-5.89 (m, 1H), 5.23-5.32 (m, 0.5H), 4.08-4.91 (m, 0.5H), 3.90-4.31 (m, 4H), 3.47-3.51(m, 2H), 3.22-3.29 (m, 1H), 3.13-3.18 (m, 1H).

**Example 16:** 4-((R)-4-acryloyl -3-methylpiperazin-1-yl)-7-(2-amino-7-fluorobenzo [d] thiazol-4-yl)-6-chloro-8-fluoroquinolin-3-cyano

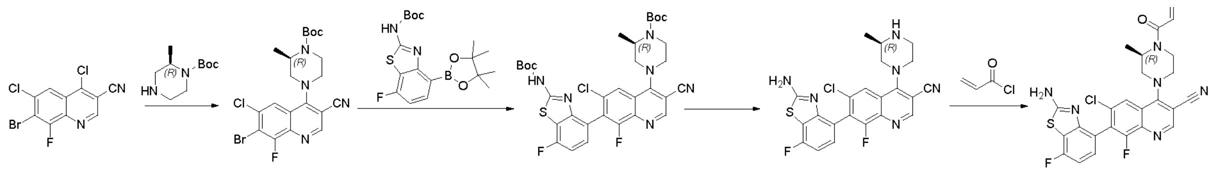

Step 1: Chloroquinoline (100.0 mg, 0.3 mmol) was dissolved in anhydrous DMF (20 mL), piperazine (66.0 mg, 0.33 mmol) and DIEA (116.1 mg, 0.9 mmol) were added, reacted overnight at room temperature. After the completion of the reaction was detected, spun dry DMF, and separated by column chromatography (PE/EA=10/1) to give tert-butyl (R ) -4-(7-bromo-6-chloro-3-cyano-8-fluoroquinolin-4-yl)-2-methylpiperazine-1-carboxylate (white solid, 96.0 mg).LC-MS[M H]: m/z 485.0.

Step 2: The white solid (50.0 mg, 0.1 mmol) from the previous step, and benzothiazole borate (54.0 mg, 0.13 mmol) were dissolved in dioxane/$H_2O$ (30 mL /10 mL),followed by the addition of potassium phosphate (32.1 mg, 0.15 mmol) and Pd(tbdpf)$Cl_2$ (7.0 mg, 0.01 mmol) in turn. The reaction was carried out at 90 °C for 4 h under nitrogen protection, cooled to room temperature, mixed directly and separated by column chromatography (PE/EA=3/1) to give tert-butyl (2R)-4-(7-(2-((tert-butoxycarbonyl)amino)-7-fluorobenzo[d]thiazol-4-yl)-6-chloro-3-cyano-8-fluoroquinolin -4-yl)-2-methylpiperazin-1-carboxylate (white solid, 50.0 mg ).LC-MS[M H]: m/z 671.1.

Step 3: The compound (50.0 mg, 0.07 mmol) from the previous step was dissolved in DCM (5 mL), followed by th addition of TFA(1mL), and stirred at room temperature overnight. The reaction was detected completely by LCMS and spun dry to give a yellow crude solid (40.0 mg), which was directly used in the next step.LC-MS[M + H]+: m/z 471.0.

Step 4: The crude compound (50 mg, 0.1 mmol) from the previous step was dissolved in THF/water (6 mL / 2 mL), followed by the addition of $K_3PO_4$ (43.0 mg, 0.2 mmol) and acryloyl chloride (10.0 mg, 0.11 mmol), then stirred for 1 h at room temperature.10 mL of water was added, extracted with EA, dried over MgSO4, filtered and spun dry, and conducted preparativeseparation to afford4-((R)-4-acryloyl-3-methylpiperazine-1-methyl)-7-(2-amino-7-fluorobenzo[d]thiazol-4-yl)-6-chloro-8-fluoroquinolin-3-cyano(yellow solid, 17.0 mg).LC-MS[M H]: *m/z.* 525.0/527.0.$^1$H-NMR (400 MHz, MeOD-$d_4$): $\delta$ 8.87 (s, 1H), 8.19 (d, 1H), 7.27-7.31(m, 1H), 7.05 (dd, 1H), 6.35-6.90 (m, 1H), 6.05-6.29 (m, 1H), 5.82-5.89 (m, 1H), 3.73-4.30 (m, 7H), 1.20 (s, 3H).

**Example 17:** (2R,4aR)-3-acryloyl -10-(2-amino-7-fluorobenzo [d] thiazol-4-yl) -11-chloro-9-fluor-2, 6-dimethyl-2, 3,4, 4a-tetrahydro-1H-pyrazine [1',2':4,5] pyrazin [2,3-c] quinolin -5(6H)-one

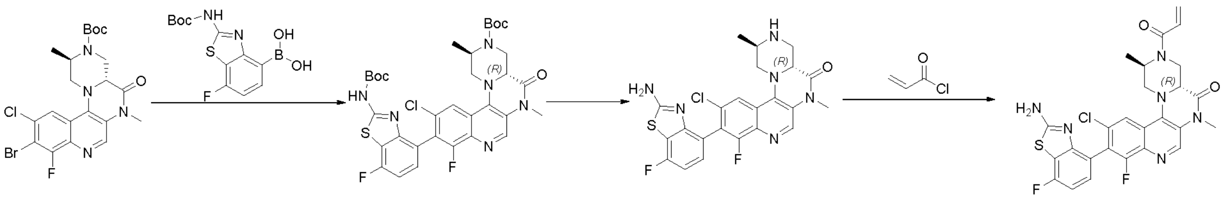

Step 1: tert-Butyl (2R,4aR)-10-bromo-11-chloro-9-fluoro-2,6-dimethyl-5-oxo-1,2,4,4a,5,6-hexahydro-3H-pyrazino[1',2':4,5]py razino[2,3-c]quinoline-3-carboxylate (35 mg, 0.068 mmol) and benzothiazolylboronic acid (32 mg, 0.10 mmol) were dissolved in dioxane/water (3 mL/1 mL), followed by the addition of $K_3PO_4$ (22 mg, 0.10 mmol), and Pd(dtbpf)$Cl_2$ (4.5 mg, 0.0068 mmol) under nitrogen protection, then heated to 90 °C and reacted overnight, spun dry and directly through the reversed-phase column to obtain the yellow crude product (36 mg).LC-MS[M + H]$^+$: *m/z* 701.6.

Step 2: The crude product (36 mg, 0.051 mmol) from the previous step was dissolved in DCM (1.5 mL), and TFA (0.5 mL) was added and stirred for 2 h at room temperature under nitrogen protection, and spun dry to obtain the yellow crude product (26 mg).LC-MS[M H]: 501.6 *m/z* .

Step 3: The crude product (26 mg, 0.051 mmol) from the previous step was dissolved in THF/water (3/3 mL), followed by the addition of $K_3PO_4$ (33 mg, 0.15 mmol) and acryloyl chloride (5.6 mg, 0.062 mmol), then stirred for 2 h at room temperature under nitrogen protection.10 mL of water was added andextracted with EA, dried over magnesium

sulfate ($MgSO_4$), filtered and spun dry.Preparative separation was conducted to give Example 17-1 (yellow solid, 8.8 mg) and Example 17-2 (yellow solid, 9.7 mg).

Example 17-1:LC-MS[M + H]$^+$: *m/z.* 555.1/557.1,RT:8.476 min (Column:Sunfire C18 4.6*150mm, 5uM,13min,0.1% formic acid/water).$^1$H-NMR (400 MHz, MeOD-$d_4$): $\delta$ 8.84 (s, 1H), 8.15 (s, 1H), 7.26-7.33 (m, 1H), 7.02-7.14 (m, 2H), 6.24-6.26 (m, 1H), 5.78-5.80 (m, 1H), 4.80-4.83 (m, 1H), 4.05-4.11 (m, 1H), 3.903.98 (m, 2H), 3.62 (s, 3H), 3.41-3.48 (m, 1H), 1.59 (d, 3H).

Example 17-2: LC-MS[M + H]$^+$: *m/z.* 555.1/557.1,RT:8.469 min (Column:Sunfire C18 4.6*150mm, 5uM,13min,0.1% formic acid/water).$^1$H-NMR (400 MHz, MeOD-$d_4$): $\delta$ 8.84 (s, 1H), 8.15 (s, 1H), 7.26-7.33 (m, 1H), 7.02-7.14 (m, 2H), 6.24-6.26 (m, 1H), 5.79-5.83 (m, 1H), 4.80-4.83 (m, 2H), 4.06-4.11(m, 2H), 3.92-3.96 (m, 1H), 3.62 (s, 3H), 3.41-3.48 (m, 1H), 1.59 (d, 3H).

**Example 18:** 1-(4-(7-(2-amino-7-fluorobenzo [d] thiazol-4-yl) -6-chloro-2-(3-(dimethylamino) azetin-1-yl)-8-fluoroquinazolin-4-yl) piperazin-1-yl) prop-2-en-1-one

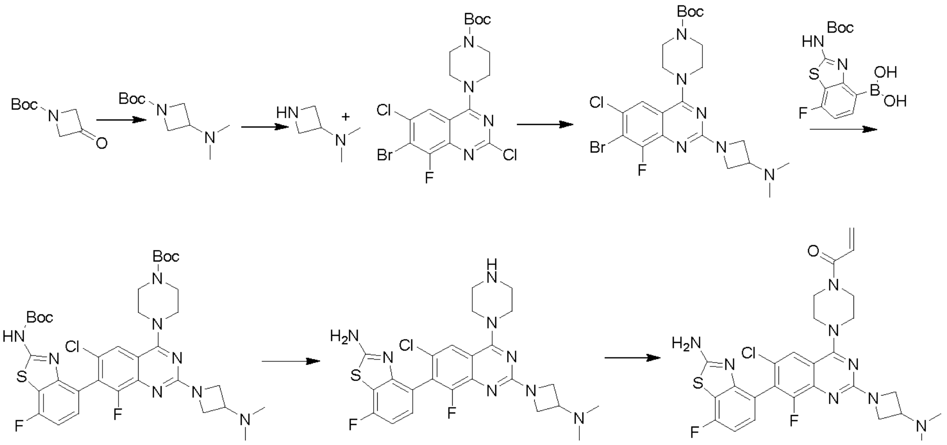

Step 1: Tert-butyl-3-oxoazetidine-1-carboxylate (1.0 g, 5.8 mmol), dimethylamine hydrochloride (947 mg, 11.7 mmol), palladium/carbon (Pd/C) (0.4 g) were dissolved in glacial acetic acid/methanol (AcOH/MeOH) (1 mL/8 mL).The reaction proceeded overnight at room temperature under hydrogen atmosphere, filtered, concentrated, extracted with ethyl acetate (50 mL), washed sequentially with saturated NaHCOs (30 mL) and saturated saline (30 mL), dried, and concentrated to give a yellow oil (1.1 g), which was used directly in the next step.LC-MS[M + H]+: m/z 201.5.

Step 2: The yellow oil (1.1 g, 5.5 mmol) from the previous step was dissolved in DCM (8 mL).TFA (2 mL) was added slowly dropwise and the reaction was carried out at room temperature for 1h.Concentration was conducted to obtain a yellow oil (610 mg), which was used directly in the next step.LC-MS[M + H]+: m/z 101.6.

Step 3: The oil (200 mg, 0.4 mmol) from the previous step, tert-butyl-4-(7-bromo-2,6-dichloro-8-fluoroquinazolin-4-yl)piperazin-1-carboxylate (82 mg, 0.8 mmol) and DIEA (325 mg, 2.5 mmol)were dissolved in DMF (3 mL) and then reacted for 1 day at room temperature under nitrogen protection. The reacting solution wasdiluted with water (30 mL) and extracted with ethyl acetate (30 mL*3), washed with saturated salt water, dried, concentrated, and seperated by column chromatography (EA) to obtain a dark green solid (102 mg).LC-MS[M + H]+: m/z 543.1.

Step 4: The solid (90 mg, 0.16 mmol) from the previous step and benzothiazole boronic acid compound (83 mg, 0.21 mmol) were dissolved in dioxane/water (6 mL/2 mL) under nitrogen protection, followed by the addition of 1,1'-bis(di-tert-butylphosphino)ferrocene palladium dichloride (13 mg, 0.02 mmol) and $K_3PO_4$ (53 mg, 0.25 mmol). The reacting solution wasreacted at 90°C for 3h, poured into 100 mL of water and extracted with ethyl acetate (50 mL*3), washed with saturated saline, dried, concentrated and separeated by column chromatography (DCM:MeOH=10:1) to give a yellow oil (100 mg).LC-MS[M + H]+: m/z 731.1.

Step 5: The oil (100 mg) from the previous step was dissolved in dichloromethane (5 mL), followed by the addition of TFA (1.5 mL), stirred for 1 h at room temperature, concentrated, and the TFA was removed to give a yellow solid (72 mg), which was directly used in next step.LC-MS[M + H]+: m/z 531.1.

Step 6: The solid (72 mg, 0.14 mmol) from the previous step, acrylic acid (13 mg, 0.17 mmol), HATU (79 mg, 0.20 mmol) and DIEA (52 mg, 0.41 mmol) were dissolved in DMF (2 mL) and reacted at room temperature for 1 h,separated by preparative reverse phase column to give the target compound (yellow solid, 11 mg) LC-MS[M + H]+: m/z 585.0.1H NMR (400 MHz, CD3OD): δ 8.02 (s, 1H), 7.26-7.22 (m, 1H), 7.05-7.00 (m, 1H), 6.80 (dd, 1H), 6.30 (dd, 1H), 5.83 (dd,

1H), 4.70-4.65 (m, 2H), 4.60-4.54 (m, 2H), 4.31-4.24 (m, 1H), 4.22-4.13 (m, 4H), 3.95-3.88 (m, 4H), 2.96 (s, 6H).

**Example 19**:3-((4-(4-acryloylpiperazin-1-yl)-7-(2-amino-7-fluorobenzo [d] thiazol-4-yl)-6-chloro-8-fluoroquinazolin-2-yl) amino)-N,N-dimethylpropanamide

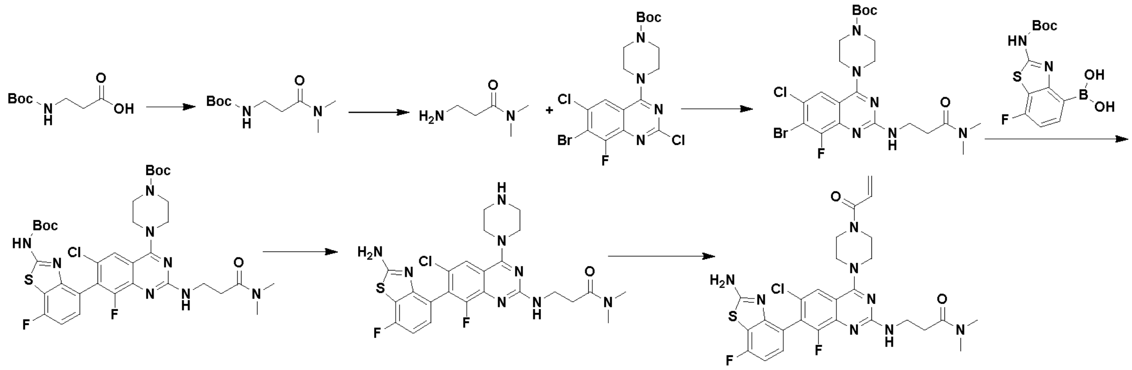

Step 1: The raw materials tert-butoxycarbonyl-aminopropionate (1.0 g, 5.3 mmol), dimethylamine hydrochloride (428 mg, 5.4 mmol), HATU (3.1 g, 7.9 mmol) and DIEA (2.1 g, 15.9 mmol) were dissolved in DMF (8 mL). The reaction proceeded at room temperature under nitrogen protection overnight, diluted with water (30 mL) and extracted with ethyl acetate (30 mL*3), the organic phase was washed with water (30 mL) and saturated saline (30 mL) in turn, dried, concentrated and separated by column chromatography (PE:EA=1:2) to give a brown oil (1.0 g).LC-MS[M H]: m/z 217.5.

Step 2: The oil (1.0 g, 4.6 mmol) from the previous step was dissolved in DCM (8 mL).TFA(2 mL) was added slowly and reacted at room temperature for 1h.Concentration was conductedto obtain a yellow oil (530 mg), which was used directly in the next step.LC-MS[M H]: m/z 117.6.

Step 3: The yellow oil (200 mg, 0.4 mmol) from the previous step, tert-butyl-4-(7-bromo-2,6-dichloro-8-fluoroquinazolin-4-yl)piperazin-1-carboxylate (98 mg, 0.8 mmol) and DIEA (325 mg, 2.5 mmol) were dissolved in DMF (3 mL) and reacted for 1 day at room temperature under the protection of nitrogen. The reaction was diluted with water (30 mL) and extracted with ethyl acetate (30 mL*3), washed with saturated salt water, dried, concentrated and separated by column chromatography (PE:EA=1:2) to obtain a pale yellow solid (180 mg).LC-MS[M H]: m/z 558.9.

Step 4: The solid (90 mg, 0.16 mmol) from the previous step and benzothiazole boronic acid (83 mg, 0.21 mmol) were dissolved in dioxane/water (6 mL/2 mL), under the nitrogen protection, followed by the addition of 1,1'-bis(di-tert-butylphosphino)ferrocene palladium dichloride (13 mg, 0.02 mmol) and $K_3PO_4$ (53 mg, 0.25 mmol), reacted at 90°Cfor 3h, poured into 100 mL of water and extracted with ethyl acetate (50 mL*3), washed with saturated saline, dried, concentrated and separated by column chromatography (DCM:MeOH=10:1) to give a yellow oil (120 mg).LC-MS[M H]: m/z 747.1.

Step 5: The oil (120 mg) from the previous step was dissolved in dichloromethane (5 mL), followed by the addition of TFA (1.5 mL), stirred for 1 h at room temperature, concentrated, and the TFA was removed to give a yellow solid (88 mg), which was directly used in next step.LC-MS[M H]: m/z 547.0.

Step 6: The yellow solid (88 mg, 0.16 mmol) from the previous step, acrylic acid (15 mg, 0.21 mmol), HATU (93 mg, 0.24 mmol) and DIEA (62 mg, 0.48 mmol) were dissolved in DMF (2 mL) and reacted for 1h at room temperature, and then separated bypreparative reverse phase column to give the target compound (yellow solid, 7.6 mg).LC-MS[M H]: m/z 601.1.$^1$H NMR (400 MHz, $CD_3OD$): $\delta$ 8.06 (s, 1H), 7.27-7.23 (m, 1H), 7.04-7.00 (m, 1H), 6.80 (m, 1H), 6.31 (m, 1H), 5.83 (m, 1H), 4.38-4.28 (m, 4H), 4.02-3.84 (m, 6H), 3.05 (s, 3H), 2.96 (s, 3H), 2.80-2.77 (m, 2H).

**Example 20:** 4-(4-acryloyl piperazin-1-yl)-7-(2-amino-7-fluorobenzo [d] thiazol-4-yl) -6-chloro-2-(2-(dimethylamino) ethoxy)-8-fluoroquinolin-3-cyano

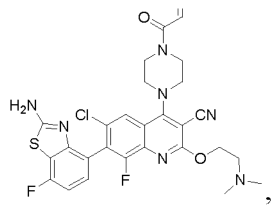

the intermediate 1B was used as the raw material and synthesized by the method of Example 3 to obtain Example 20.LC-MS[M H]: m/z 598.2/600.2.

**Example 21**:4-(4-acryloyl piperazin-1-yl)-7-(2-amino-7-fluorobenzo [d] thiazol-4-yl) -6-chloro-2-(3-(dimethylamino) azetidine-1-yl)-8-fluoroquinolin-3-cyano

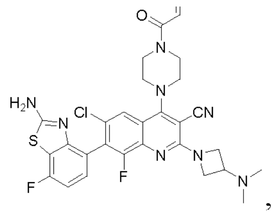

the intermediate 1B was used as the raw material and synthesized by the method of Example 18 to obtain Example 20.LC-MS[M H]: m/z 609.2/611.2.

**Example 22**:3-((4-(4-acryloylpiperazin-1-yl)-7-(2-amino-7-fluorobenzo [d] thiazol-4-yl)-6-chloro-3-cyano-8-fluoroquinolin-2-yl) amino)-N,N-dimethylpropanamide

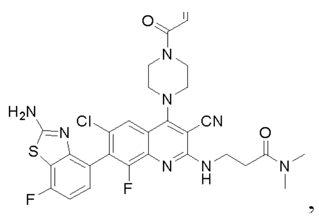

the intermediate 1B was used as the raw material and synthesized by the method of Example 19 to obtain Example 20.LC-MS[M H]: m/z 625.1/627.1.

**Example 23:** 1-((3S)-4-(7-(2-amino-7-fluorobenzo [d] thiazol-4-yl) -6-chloro-8-fluoro-2-(((S)-1-methylpyrrolin-2-yl) methoxy) quinazolin-4-yl)-3-methylpiperazin-1-yl) prop-2-en-1-one

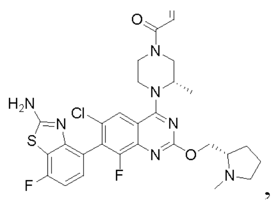

Example 23 was obtained by synthesis referring to the method of Example 11.LC-MS[M H]: m/z 614.1/616.1.$^1$H-NMR (400 MHz,$CD_3OD$) $\delta$7.92 (d, 1H), 7.25-7.21 (m, 1H), 7.03-6.98 (m, 1H), 6.89-6.77 (m, 1H), 6.32-6.28 (m, 1H), 5.83 (d,

1H), 4.98-4.89 (m, 2H), 4.66-4.61 (m, 1H), 4.54-4.32 (m, 2H), 4.22-4.01 (m, 1H), 3.91-3.61 (m, 4H), 3.24-3.12 (m, 2H), 3.08 (s, 3H), 2.44-2.35 (m, 1H), 2.25-2.03 (m, 3H), 1.45-1.39 (m, 3H).

**Example 24**:1-(4-(7-(2-amino-7-fluorobenzo[d]thiazol-4-yl)-6-chloro-8-fluoro-2-(((2S,4R)-4-hydroxy-1-methylpyrrol in-2-yl) methoxy) quinazolin-4-yl) piperazin-1-yl) prop-2-en-1-one

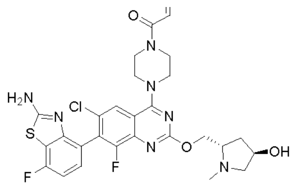,

Example 24 (white solid, 12.1 mg) was obtained by synthesis referring to the method of Example 11.LC-MS[M H]: m/z 616.1.$^1$H NMR (400 MHz, MeOD-$d_4$): $\delta$ 7.95 (s, 1H), 7.21 (dd, J=5.6, 8.0Hz, 1H), 6.99 (t, J=8.4Hz,1H), 6.78-6.86(m, 1H), 6.28 (dd, J=2.0, 16.8Hz, 1H), 5.81 (dd, J=2.0, 10.8Hz, 1H), 4.49-4.61(m, 2H), 4.35-4.39 (m, 1H), 3.92-4.00 (m, 8H), 3.39-3.43 (m, 1H), 3.17-3.22 (m, 1H), 2.59 (s, 3H), 2.37-2.42 (m, 1H), 2.00-2.04 (m, 1H).

**Example 25:** 1-(4-(7-(2-amino-7-fluorobenzo[d]thiazol-4-yl)-6-chloro-8-fluoro-2-(((2S,4S)-4-fluoro-1-methylpyrrolin-2-y 1) methoxy) quinazolin-4-yl) piperazin-1-yl) prop-2-en-1-one

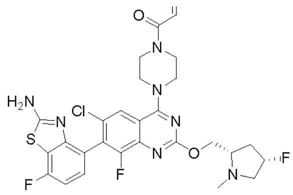,

Example 25 (white solid, 13.5 mg) was obtained by synthesis referring to the method of Example 11.LC-MS[M H]: m/z 618.0.$^1$H NMR (400MHz, MeOD-$d_4$): $\delta$ 7.94 (s, 1H), 7.21 (dd, 1H), 6.99 (t, 1H), 6.81 (dd, 1H), 6.28 (dd, 1H), 5.81 (dd, 1H), 5.06-5.23 (m, 1H), 4.55-4.60 (m, 1H), 4.43-4.49 (m, 1H), 3.92-4.00 (m, 8H), 3.24-3.31 (m, 1H), 2.82-2.86 (m, 1H), 2.43-2.62 (m, 5H), 1.89-2.04 (m, 1H).

**Example 26:** 1-(4-(7-(2-amino-7-fluorobenzo[d]thiazol-4-yl)-6-chloro-2-(((S)-4,4-difluoro-1-methylpyrrolidin-2-yl)metho xy)-8-fluoroquinazolin-4-yl)piperazin-1 -yl)prop-2-en-1 -one

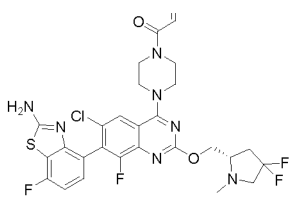,

Example 26 (white solid, 6.3 mg) was obtained by synthesis referring to the method of Example 11.LC-MS[M H]: m/z 636.0.

**Example 27:** 1-(4-(7-(2-amino-7-fluorobenzo[d]thiazol-4-yl)-6-chloro-8-fluoro-2-(((2S,4R)-4-fluoro-1-methylpyrrolidin-2 -yl)methoxy)quinazolin-4-yl)piperazin-1 -yl)prop-2-en-1 -one

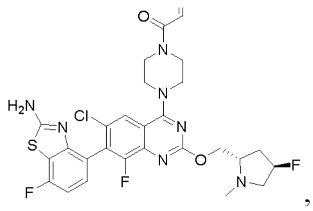

Example 27 (white solid, 12.3 mg) was obtained by synthesis referring to the method of Example 11.LC-MS[M H]: m/z 618.1.

**Example 28**:(2R,4aR)-10-(2-amino-7-fluorobenzo[d]thiazol-4-yl)-11-chloro-9-fluoro-3-(2-fluoroacryloyl)-2, 6-dimethyl-2, 3,4, 4a-tetrahydro-1H-pyrazin [1 ’,2’:4,5] pyrazin [2,3-c] quinolin -5(6H)-one

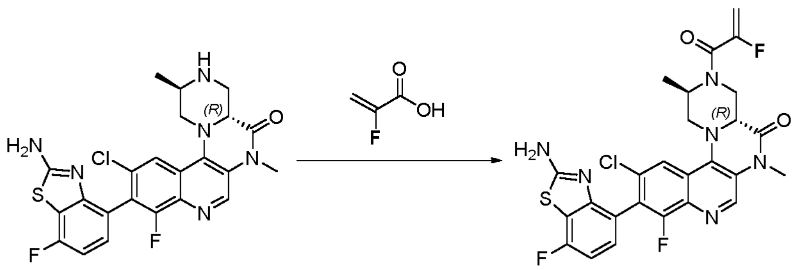

(2R,4aR)-10-(2-amino-7-fluorobenzo[d]thiazol-4-yl)-11-chloro-9-fluoro-2,6-dimethyl-2,3,4,4a-tetrahyd ro-1H-pyrazin[1’,2’:4,5]pyrazin[2,3-c]quinolin-5(6H)-one and fluoroacrylic acid were synthesized by the same route as in Example 17 and separated by silica gel column chromatography to give Example 28 (white solid, 8.2 mg).LC-MS[M H]: *m*/*z*. 573.2/575.2.$^1$H-NMR (400 MHz, MeOD-$d_4$): $\delta$ 8.84 (s, 1H), 8.15 (s, 1H), 7.26-7.33 (m, 1H), 7.02-7.14 (m, 2H), 5.81-5.83 (m, 1H), 4.80-4.82 (m, 2H), 4.06-4.10(m, 2H), 3.92-3.95 (m, 1H), 3.62 (s, 3H), 3.41-3.48 (m, 1H), 1.59 (d, 3H).

**Example 29**:7-(2-amino-7-fluorobenzo[d]thiazol-4-yl)-6-chloro-8-fluoro-4-((R)-4-(2-fluoroacryloyl)-3-methylpiperazi n-1-yl)-2-((1-methylpyrroline-2-yl) methoxy) quinolin-3-cyano

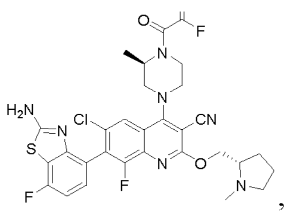

Example 29 (white solid, 10.0mg) was obtained by synthesis referring to the method of Example 13.LC-MS[M H]: m/z 656.3.

**Example 30**:7-(2-amino-7-fluorobenzo[d]thiazol-4-yl)-6-chloro-2-((4,4-difluoro-1-methylpyrrolin-2-yl) methoxy) -8-fluoro-4-(4-(2-fluoroacryloyl) piperazin-1-yl) quinolin-3-cyano

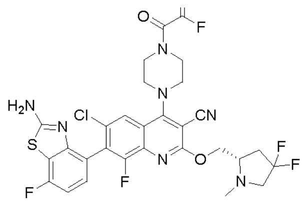
,

Example 30 (white solid, 6.3mg) was obtained by synthesis referring to the method of Example 13.LC-MS[M H]: m/z 678.1.

**Example 31**:7-(2-amino-7-fluorobenzo [d] thiazol-4-yl) -6-chloro-2-(3-(dimethylamino) azetidine-1-yl) -8-fluoro-4-(4-(2-fluoroacryloyl) piperazin-1-yl)quinolin-3-cyano

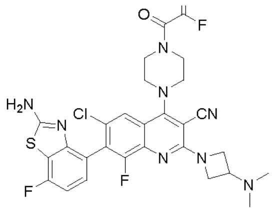
,

Example 31 (white solid, 9.7mg) was obtained by synthesis referring to the method of Example 18.LC-MS[M H]: m/z 627.1.

**Comparative compound 1**:1-(4-(7-(2-amino-7-fluorobenzo[d]thiazol-4-yl)-6-chloro-8-fluoroquinazolin-4-yl) piperazin-1-yl) prop-2-en-1-one

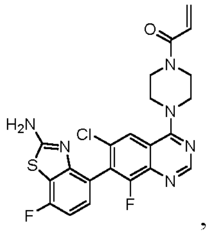
,

Comparative compound 1 was synthesized by referring to the method of Example 1 in patent document WO2020081282.LC-MS[M H]: m/z 487.0/489.0.$^1$H-NMR (400 MHz, $CD_3OD$) $\delta$ 8.74 (s, 1H), 8.24 (s, 1H), 7.27-7.30(m, 1H), 7.01-7.06 (m, 1H), 6.80 (dd, J=16.8Hz, 2.8Hz, 1H), 6.31 (dd, J=1.6Hz, 16.8Hz, 1H), 5.83 (dd, J=1.6Hz, 10.8Hz, 1H), 4.37-4.39(m, 4H), 3.95-3.99(m, 4H).

**Comparative compound 2:** (2R,4aR)-3-acryloyl-11-chloro-9-fluoro-10-(2-fluoro-6-hydroxyphenyl)-2,6-dimethyl-2,3,4,4a-tetrahydro-1 H-pyrazin[1',2':4,5]pyrazin[2,3-c]quinolin-5(6H)-one

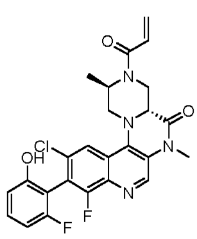
,

Comparative compound 2-1 and 2-2 was synthesized by referring to the method of Example 21 and 22 in patent document WO2019110751A1.Comparative compound 2-1:LC-MS[M + H]+: m/z. 499.1/501.1,RT:5.466 min (column: Sunfire C18 4.6*150mm, 5uM, elution gradient time: 13min, mobile phase: A:0.1% formic acid/water; B:0.1% formic acid/acetonitrile), wavelength: 254nM.$^1$H-NMR (400 MHz, MeOD-d4): $\delta$ 8.84 (s, 1H), 8.13 (s, 1H), 7.30-7.36 (m, 1H), 7.07-7.14 (m, 1H), 6.79 (d, 1H), 6.71-6.77 (m, 1H), 6.23 (d, 1H), 5.80 (d, 1H), 4.80-4.83 (m, 1H), 3.90-3.99 (m, 2H), 3.56 (s, 3H), 3.34-3.37 (m, 1H), 3.15-3.19 (m, 1H), 1.59 (d, 3H).

Comparative compound 2-2:LC-MS[M + H]+: m/z. 499.1/501.1,RT:5.851 min (column: Sunfire C18 4.6*150mm, 5uM, elution gradient time: 13min, mobile phase: A:0.1% formic acid/water; B:0.1% formic acid/acetonitrile), wavelength: 254nM.$^1$H-NMR (400 MHz, MeOD-d4): d 8.84 (s, 1H), 8.12 (s, 1H), 7.30-7.36 (m, 1H), 7.07-7.14 (m, 1H), 6.78 (d, 1H), 6.71-6.76 (m, 1H), 6.23 (d, 1H), 5.80 (d, 1H), 4.80-4.85 (m, 1H), 3.90-3.97 (m, 2H), 3.56 (s, 3H), 3.34-3.37 (m, 1H), 3.10-3.14 (m, 1H), 1.59 (d, 3H).

**Test Example 1 Kras$^{G12C}$ Functional Analysis**

CisBio's KRAS$^{G12C}$/SOS1 kit was used to test the efficacyof the compound on inhibiting the protein-protein interaction between SOS1 and KRAS$^{G12C}$by Binding assay.Results were expressed as $IC_{50}$values.

Test methods: (1) the test compound was tested at a concentration of 1000 nM. Diluted to 3-fold diluted compound of a 200-fold final concentration of 100% DMSO solution in a 384-well plate at 10 concentrations.50 nL of 200-fold final concentration of compound was transferred to the destination plate 384 well plate using a dispenser Echo 550.50 nL of 100% DMSO was added to the negative control wells and positive control wells; (2) 4-fold final concentration of Tag1 SOS1 solution was prepared using Diluent buffer; (3) 2.5 μL of 4-fold final concentration of Tag1 SOS1 solution was added to the 384-well plate; (4) 4-fold final concentration of Tag2 KRAS$^{G12C}$ solution was prepared using Diluent buffer; (5) 2.5 μL of 4-fold final concentration of Tag2 KRAS$^{G12C}$ solution was added to compound wells and positive control wells, respectively; 2.5 μL of diluent buffer was added to negative control wells; (6) the 384-well plate was centrifuged at 1000 rpm for 30 seconds, shaken and mixed, and incubated at room temperature for 15 min; (7) 1-fold final concentration of Anti Tag1 TB3+ solution and 1-fold final concentration of Anti Tag2 XL665 solution were prepared with Detection buffer, and 5 μL of the mixture was added to each well after mixing the two solutions; (8) the 384-well plate was centrifuged at 1000 rpm for 30 seconds, shaken and mixed, and incubated at room temperature for 120 minutes; (9) the Envision enzyme marker was used to read Em665/620; (10) data analysis, calculation formula $\text{Inhibition\%}=\frac{\text{Max signal} - \text{Compound signal}}{\text{Max signal} - \text{Min signal}} \times 100$; where Min signal is the mean value of negative control wells, Max signal is the mean value of positive control wells.The log value of concentration was used as the X-axis and the percentage inhibition as the Y-axis to fit the quantitative efficacy curve using the log(inhibitor) vs. response Variable slope of the quantitative efficacy curve of the analytical software GraphPad Prism 5 to derive the $IC_{50}$ value of each compound on the enzyme activity. The fitting formula was: Y=Bottom+(Top Bottom)/(1+10^((LogIC50X)* HillSlope)).

RESULTS: Most of the compound of the present invention showed significant inhibition againstKRAS$^{G12C}$/SOS1 interaction with an inhibitory activity $IC_{50}$ less than 500 nM, and in some embodiments the ICso was less than 100 nM or even less than 50 nM.The results are shown in table 1, A<50 nM, 50 nM ≤B<200 nM, 200 nM ≤C<1000 nM, D>1000 nM.

| No. | $IC_{50}$/nM | No. | $IC_{50}$/nM | No. | $IC_{50}$/nM | No. | $IC_{50}$/nM |
|---|---|---|---|---|---|---|---|
| 1 | A | 2 | A | 3 | A | 4 | A |
| 5 | A | 6 | B | 7 | B | 8 | A |
| 9 | B | 10 | B | 11 | B | 12 | B |
| 13 | B | 14 | B | 15 | C | 16 | B |
| 17-1/17-2 | B/B | 18 | A | 19 | B | 20 | A |
| 21 | A | 22 | B | 23 | A | 24 | A |
| 25 | A | 26 | A | 27 | A | 28 | B |
| 29 | B | 30 | B | 31 | B | | |
| Comparative Compound 1 | B | | | Comparative Compound 2-1 | B | Comparative Compound 2-2 | D |

**Test Example 2: Effect of the compound of the present invention on the proliferation and downstream signaling ERK phosphorylation ability of NCI-H358 and MiaPaca-2 cells assay**

Test method 1 (2D):NCI-H358 (lung cancer) and MiaPaca-2 cells (pancreatic cancer) cells (100 μL/well, 20,000 cells/mL) were inoculated in 96-well culture plates supplemented with 10% fetal bovine serum and 1% penicillin/streptomycin sulfate, respectively.Using 0.5% dimethyl sulfoxide as a blank control, cells were treated with a solution of the compound to be tested at a starting concentration of 10 μM, tripled diluted testing solution of the compound in eight gradients were used to treat cells, and then incubated in a 5% $CO_2$ incubator for a certain period of time (5-7 days).At the end of incubation, 10 μL of MTT stock solution (5 mg/mL) was added to each well. The culture plates were incubated at 37°C for 4 h and then the medium was removed. Dimethyl sulfoxide (100 μL) was added to each well and then shaken sufficiently. The absorbance of the product formazanwas measured at 570 nm on the Thermo Scientific Varioskan Flash multimode reader.$IC_{50}$ values were obtained by fitting dose-response data into a three-parameter nonlinear regression model using GraphPad Prism 6.0 software.

Results: The example compound provided by the present invention showed proliferation inhibitory activity against NCI-H358 and MiaPaca-2 cells with $IC_{50}$ values less than 5000 nM; some of the Example compounds such as Examples 1, 3, 5, 8, 10, 11, 13, 16, 18, 20, 21, 25, 29 and 31 showed significant proliferation inhibitory activity against NCI-H358 and MiaPaca-2 cells with $IC_{50}$ values less than 100 nM, and the proliferation inhibitory activity of some examples was even less than 10 nM, showing excellent in vitro antitumor effects.As shown in Table (II), the data of proliferation inhibitory activity of the compound of examples of the present invention on MiaPaca-2 cells are listed.

| Example No. | $IC_{50}$/nM | Example No. | $IC_{50}$/nM | Example No. | $IC_{50}$/nM |
|---|---|---|---|---|---|
| 1 | 5.5 | 2 | 13.2 | 3 | <0.3 |
| 4 | 1.1 | 5 | 2.3 | 6 | 35.0 |
| 7 | 23.1 | 8 | 13.8 | 9 | 362.0 |
| 10 | 57.2 | 11 | 31.0 | 12 | 19.3 |
| 13 | 12.1 | 14 | 0.90 | 15 | 390 |
| 16 | 22.3 | 17-1/17-2 | 24.8/23.1 | 18 | 0.67 |
| 19 | 28.0 | 20 | 1.3 | 21 | 0.89 |
| 22 | 0.93 | Comparative Compound 1 | 2.6 | Comparative Compound 2-1/2-2 | 96.4/>10000 |
| 23 | 1.2 | 24 | <1.0 | 25 | <1.0 |
| 26 | <1.0 | 27 | <1.0 | 28 | 63.2 |
| 29 | <1.0 | 30 | <1.0 | 31 | <1.0 |

Test method 2 (3D): The tumor cells in the logarithmic growth phase were diluted with culture solution to a certain concentration and inoculated on the 96-well plate on the ultra-low attachment surface, and the culture medium was 80 μL/well.Cells were incubated overnight at 37°C in a humidity chamber. On the second day, a series of diluted test compounds (10 concentrations, 3 times dilution) were added to the plate, 20 μL/well, incubated in an incubator for 96h.The plates are removed and placed at room temperature and incubated with an equal volume of Cell Titer Glo®3D reagent for 1 h. The signal is detected by the En Vision™ plate reader. The signal is converted to percentage inhibition using the following formula: % Inhibition = 100 - [(test compound signal - median minimum signal) / (median maximum signal - median minimum signal) × 100].The maximum signal is the signal value of the inhibitor-free well, and the minimum signal is the signal value of the wellcontaining a reference inhibitor sufficient to completely inhibit cell proliferation. A four-parameter nonlinear regression curve was fitted to the percent inhibition for each concentration of the compound and the $IC_{50}$ was calculated.

Results: The example compound provided by the present invention showed proliferation inhibitory activity against NCI-H358 and MiaPaca-2 cells with $IC_{50}$ values all less than 1000 nM; some Examples such as Examples 1, 2, 3, 5, 8, 10, 11, 13, 16, 18, 20, 29, 31, etc. had an $IC_{50}$ less than 200 nM for cell proliferation inhibitory activity against NCI-H358 and MiaPaca-2, and some Examples such as Examples 1, 3, 13, 18 had an $IC_{50}$ even less than 10 nM.

Test method 3 (ERK phosphorylation): Miapaca-2 or H358 cells were inoculated at certain concentrations in 96-well plates and incubated overnight at 37 °C, 5% CO2 in a cell culture incubator, and serial dilutions of the test compound (5 concentrations, 3-fold dilution) were added to the plates the next day for 24 h (Miapaca-2) or 3 h (H358), then lysis solution containing protease and phosphatase inhibitor were added to lyse the cells to extract proteins and

western blot method was used to detect the level of p-ERK.

RESULTS: The example compounds provided by the present invention such as Examples 1, 3, 5, 8, 10, 11, 13, 16, 18, 20, 21, 25, 29 and 31 etc. inhibited the phosphorylated ERK levels of NCI-H358 and MiaPaca-2 significantly, with $IC_{50}$ less than 500 nM.

**Test Example 3:**ADMET test ofExample compounds

(1) Metabolic stability assay: A warm incubation for metabolic stability was performed with a system of 150 μL of liver microsomes (final concentration 0.5 mg/mL) containing NADPH (final concentration 1 mM), 1 μM of the test compound and positive control midazolam or negative control atenolol, and the reaction was terminated with acetonitrile containing tinidazole at 0 min, 5 min, 10 min and 30 min, respectively. The reaction was vortexed for 10 min and centrifuged at 15000 rmp for 10 min, and 50 μL of supernatant was taken into the 96-well plate for sampling.The metabolic stability of the compound was calculated by measuring the relative reduction of the drugs.

Results: the example compounds of the invention were stable to various genera (rat, mouse, monkey, human) of liver microsomes with half-lives greater than 30 min, such as Example compounds 1, 3, 8, 11, 13, 14, 17-1, 20, 21, 23, etc.

| Example No. | Liver microsomal stability ($T_{1/2}$ /min) | | | |
|---|---|---|---|---|
| | Rat | Mouse | monkey | human |
| 1 | 155 | 249 | 163 | 403 |
| 3 | 147 | 99 | 47 | 403 |
| 8 | 40 | 34 | 60 | 262 |
| 11 | 48 | 110 | 104 | 403 |
| 13 | 45 | 95 | 184 | 221 |
| 14 | 121 | 403 | 403 | 48 |
| 17-1 | 403 | 44 | 64 | 131 |
| 20 | 36 | 35 | 52 | 113 |
| 21 | 37 | 166 | 57 | 202 |
| 23 | 28 | 241 | 47 | 68 |
| Comparative Compound 1 | 31 | 32 | 31 | 44 |
| Comparative Compound 2-1 | 41 | 47 | 42 | 54 |

(2) Direct inhibition test (DI test): direct inhibition warm incubation was performed with a system of 100 μL of human liver microsomes (final concentration 0.2 mg/mL) containing NADPH (final concentration 1 mM), 10 μM compound, positive inhibitor cocktail (ketoconazole 10 μM, quinidine 10 μM, sulfobenzyrazole 100 μM, α-naphthoflavone 10 μM, tranylcypromine 1000 μM), a negative control (BPS with 0.1% DMSO) and a mixed probe substrate (midazolam 10 μM, testosterone 100 μM, dextromethorphan 10 μM, diclofenac 20 μM, phenacetin100 μM, mephenytoin100 μM), and the reaction was terminated after a warm incubation for 20 min.The relative enzyme activity was calculated by measuring the relative amount of metabolites produced.

Results: The example compounds of the present invention, such as 3, 8, 11, 17-1, 21,etc., did not significantly inhibit major metabolic enzyme isoforms (e.g., CYP1A2, 2C8, 2C19, 3A4) with $IC_{50}$ greater than 10 μM.

(3) hERG inhibition test: 20 mM of compound stock solution was diluted with DMSO, 10 μL of 20 mM compound stock solution was added to 20 μL of DMSO solution, and 3-fold serial dilution was made to 6 DMSO concentrations; 4 μL of each of the 6 DMSO concentrations were added to 396 μL of extracellular solution and diluted 100-fold to 6 intermediate concentrations, then 80 μL of each of the 6 intermediate concentrations were added to 320 μL of extracellular solutions respectively and diluted 5-fold to the final concentration to be tested; the highest test concentration was 40 μM, followed by 6 concentrations of 40, 13.3, 4.4, 1.48, 0.494, and 0.165 μM, respectively; the DMSO content in the final test concentration did not exceed 0.2%, and this concentration of DMSO had no effect on the hERG potassium

channel; compound preparation was done by Bravo apparatus throughout the dilution process; current and time course plots of compounds on hERG potassium channels were read, and curves were fitted to make the inhibition curves of compoundsagainst hERG.

Results: The example compounds of the present invention, such as 3, 8, 11, 17-1, 21, etc., had no significant inhibitory effect on hERG potassium channels with $IC_{50}$ greater than 20 uM.

Test Example 4:In vivo pharmacokinetic parameter testing of example compounds in mice

Six male SPF ICR mice (Shanghai Sipur-Bikai experimental animals) were divided into two groups, the test compounds were configured into a suitable solution or suspension; one group was administered intravenously and one group was administered orally.Blood was collected via jugular venous puncture at approximately 0.2 mL/time point for each sample, anticoagulated with sodium heparin,blood was collected at the following time points: before and 5, 15 and 30 min, 1, 2, 4, 6, 8 and 24 h after administration; blood samples were collected and placed on ice, plasma was separated by centrifugation (centrifugation conditions: 8000 rpm, 6 min, 2-8°C) and collected plasma was stored at -80°C before analysis.Plasma samples were analyzed by LC-MS/MS.The pharmacokinetic parameters of $AUC_{0-t}$, $AUC_{0-\infty}$, $MRT_{0-\infty}$, $C_{max}$, $T_{max}$, $T_{1/2}$ and $V_d$ of the subjects and their mean and standard deviation were calculated separately based on the blood concentration data of the drug using the pharmacokinetic calculation software WinNonlin 5.2 non-atrial chamber model.In addition, the bioavailability (F) will be calculated by the following formula. For samples with concentrations below the lower limit of quantification (BLQ), when performing pharmacokinetic parameter calculations, samples sampled before reaching $C_{max}$ should be calculated as zero values, and samples sampled at points after reaching $C_{max}$ should be calculated as not quantifiable (BLQ).

| | *iv*(1mg/kg) | | |
|---|---|---|---|
| No. | Below the curve and $AUC_{0-t}$ (nM.h) | Clearance rate Cl (mL/min/kg) | Half-life $T_{1/2}$ (h) |
| 3 | 4095±373 | 6.35±0.67 | 6.5 |
| 8 | 2848±363 | 11.53±1.52 | 0.52 |
| 13 | 985±42 | 26.59±1.29 | 1.9 |
| 17-1 | 3315±187 | 9.04±0.50 | 1.1 |
| 21 | 1295±70 | 21.62±1.05 | 1.3 |
| Comparative Compound 1 | 1620±188 | 21.16±2.36 | 0.6 |

Results: the pharmacokinetic properties of some Examples 3, 8, 13, 17-1, 21of the present invention in mice were significantly better than those of the comparative compound 1: for example, the AUC of Example 3, 8, 17-1 were significantly increased compared to the comparative compound 1; the clearance rate Cl of Examples 3, 8, 17-1 were significantly lower than that of the comparative compound 1; and the in vivo metabolic half-life of Example 3 was significantly longer than that of the comparative compound 1.

**Test Example 5: Test of Example Compound for Growth Inhibition of the Transplanted Tumor of MiaPaca-2, NCI-H358 Tumor Cells in Nude Mice**

The tumor tissues in the peak growth period were cut into 1.5 $mm^3$ and inoculated into the right axillary subcutis of nude mice under aseptic conditions. The subcutaneous transplanted tumors of nude mice were measured by vernier calipers for the diameter of the transplanted tumors, and the animals were randomly grouped when the average volume of the tumors reached about 130 $mm^3$.The example compound (injection water containing 1% Tween 80 was prepared to the desired concentration and then left to be used) was administered orally at the given dose daily for three weeks, and the solvent control group was given an equal amount of solvent. The transplanted tumor diameter was measured twice a week throughout the experiment, and the mice were weighed at the same time. The tumor volume (TV) was calculated by the formula: $TV = 1/2 \times a \times b^2$, where a and b arethe length and width, respectively. The relative tumor volume (RTV) was calculated from the measured results by the formula: $RTV = V_t/V_0$.Where Vo is the tumor volume measured at the time of cagingand administration(i.e., d0), and Vt is the tumor volume at each measurement.The evaluation indexes of antitumor activity are 1) relative tumor proliferation rate T/C (%), calculated as follows: T/C (%) = (TRTV/CRTV) × 100%, TRTV: RTV of the treatment group; CRTV: RTV of the negative control group; 2) tumor volume growth inhibition rate GI%, calculated as follows: $GI\% = [1-(TV_t-TV_0)/(CV_t- CT_0)]\times 100\%$, TVt is the tumor volume measured in each treatment group; $TV_0$ is the tumor volume obtained when the treatment group was caged and administered;

CVt is the tumor volume measured in each control group; CVois tumor volume obtained when the control group was caged; 3) tumor weight inhibition rate, calculated as follows: tumor weight inhibition rate%=($W_c$-WT)/$W_c$×100%, $W_c$: tumor weight of the control group, WT. tumor weight of the treatment group.

Results: The example compounds of the present invention such as Examples 3, 8, 17-1 showed significant tumor growth inhibition at 10 mg/kg and 30 mg/kg doses administered orally once daily for 21 days, with tumor inhibition rates greater than 60%; Example 8 showed tumor inhibition rates greater than 90% at 10 mg/kg doses, and tumor regression was achieved after two weeks of administration.

All documents referred to in the present invention are incorporated by reference herein as if each document is individually incorporated by reference. Further, it should be understood that upon reading the above teaching of the present invention, various modifications or revisonsmay be made to the present invention by those skilled in the art, and those equivalents also fall within the scope defined by the appended claims of the present application.

## Claims

1. A benzothiazolyl biaryl compound having formula I, or a pharmaceutically acceptable salt thereof, or an enantiomer, a diastereoisomer, a tautomer, a torsional isomer, a solvate, a polymorph, or a prodrug thereof,

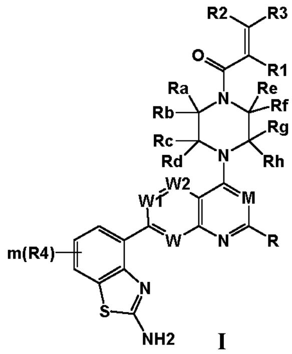

I

Wherein, M is N or CR5;

When M is N, R is independently halogen, cyano, hydroxyl, nitro, amino, $C_1$-$C_6$alkyl, $C_1$-$C_6$alkyl-S-, $C_1$-$C_6$alkyl-SO-, $C_1$-$C_6$alkyl-$SO_2$-, $C_1$-$C_6$haloalkyl, $C_1$-$C_6$alkyl-O-, $C_1$-$C_6$alkyl-NR'R"-, 3-12-membered cycloalkyl, 3-12-membered heterocycloalkyl, 3-12-membered cycloalkyl-O-, 3-12-membered cycloalkyl-NR'R"-, 3-12-membered heterocycloalkyl-O-, 3-12-membered heterocycloalkyl-NR'R"-, Q1-($C_1$-$C_4$alkylene)-O-, Q2-($C_1$-$C_4$alkylene)-NR'-, NR'R"-($C_1$-$C_4$alkylene)-O-, NR'R"-CO-($C_1$-$C_4$alkylene)-NR'-, NR'R"-Q3-, 5-12-membered aryl or 5-12-membered heteroaryl;

When M is CR5, R is independently hydrogen, halogen, cyano, hydroxyl, nitro, amino, $C_1$-$C_6$alkyl, $C_1$-$C_6$alkyl-S-, $C_1$-$C_6$alkyl-SO-, $C_1$-$C_6$alkyl-$SO_2$-, $C_1$-$C_6$haloalkyl, $C_1$-$C_6$alkyl-O-, $C_1$-$C_6$alkyl-NR'R"-, 3-12-membered cycloalkyl, 3-12-membered heterocycloalkyl, 3-12-membered cycloalkyl-O-, 3-12-membered cycloalkyl-NR'R"-, 3-12-membered heterocycloalkyl-O-, 3-12-membered heterocycloalkyl-NR'R"-, Q4-($C_1$-$C_4$alkylene)-O-, Q5-($C_1$-$C_4$alkylene)-NR'-, NR'R"-($C_1$-$C_4$alkylene)-O-, NR'R"-CO-($C_1$-$C_4$alkylene)-NR'-, NR'R"-Q6-, 5-12 membered aryl or 5-12 membered heteroaryl;

Q1, Q2, Q3, Q4, Q5 and Q6 are independently 3-7-membered heterocycloalkyl or 3-7-membered heterocycloalkyl substituted by one or more Rq; when there are multiple substituents, they are the same or different;

Rq is independently halogen, cyano, hydroxyl, amino, or $C_1$-$C_4$alkyl;

R5 is independently hydrogen, halogen, cyano, hydroxyl, nitro, amino, $C_1$-$C_6$alkyl, C1-$C_6$alkyl-S-, $C_1$-$C_6$alkyl-SO-, $C_1$-$C_6$alkyl-$SO_2$-, $C_1$-$C_6$haloalkyl, $C_1$-$C_6$alkyl-O-, $C_1$-$C_6$alkyl-NR'R"-, 3-12-membered cycloalkyl, 3-12-membered heterocycloalkyl, 3-12-membered cycloalkyl-O-, 3-12-membered cycloalkyl-NR'R"-, 3-12-membered heterocycloalkyl-O-, 3-12-membered heterocycloalkyl-NR'R"-, 5-12-membered aryl or 5-12-membered heteroaryl;

R' and R" are independently hydrogen, $C_1$-$C_6$alkyl, 3-12-membered cycloalkyl, 3-12-membered heterocycloalkyl, 5-12-membered aryl or heteroaryl;

R1 is independently hydrogen, halogen, cyano, nitro, $C_1$-$C_6$alkyl, $C_1$-$C_6$alkyl-$SO_2$-, $C_1$-$C_6$alkyl-SO-, or $C_1$-$C_6$haloalkyl;

R2, R3 are independently hydrogen, halogen, cyano, nitro, $C_1$-$C_6$alkyl, $C_1$-$C_6$alkyl-$SO_2$-, $C_1$-$C_6$alkyl-SO-, $N(R_{2a})(R_{2b})$-$(CH_2)$x-;

$R_{2a}$ and $R_{2b}$ are each independently hydrogen or $C_1$-$C_6$alkyl, x is selected from any integer inbetween 0-5; the above alkyl can be further substituted by deuterium, halogen, substituted or unsubstituted amino/cyclic amine group;

or, $R_{2a}$ and $R_{2b}$ together form a 5-10 membered nitrogen-containing heterocycloalkyl substituted by $C_1$-$C_6$alkyl;

R4 is independently hydrogen, halogen, cyano, nitro, $C_1$-$C_6$alkyl, $C_1$-$C_6$alkyl-$SO_2$-, $C_1$-$C_6$alkyl-SO-, $C_1$-$C_6$haloalkyl, or $C_1$-$C_6$alkyl-O-;

m is an integer independently selected from0-4;

W,W1,W2 are independently CR6 or N;

R6 is independently H, halogen, cyano, $C_1$-$C_6$alkyl, $C_1$-$C_6$alkyl-O-, $C_1$-$C_6$haloalkyl, $C_1$-$C_6$haloalkyl-O-, hydroxyl substituted $C_1$-$C_6$alkyl, cyano substituted $C_1$-$C_6$alkyl, $C_2$-$C_6$alkenyl, $C_2$-$C_6$alkynyl, 3-8 membered cycloalkyl or 3-8-membered heterocycloalkyl;

Ra, Rb, Rc, Rd, Re, Rf, Rg, Rh are independently hydrogen, halogen, $C_1$-$C_6$alkyl, $C_1$-$C_6$alkyl-O-, $C_1$-$C_6$haloalkyl, cyano substituted $C_1$-$C_6$alkyl;

or Ra, Rb, Rc, Rd, Re, Rf, Rg and Rh form a saturated or partially unsaturated 3-8-membered ring system between the two;

or either Rg orRh can form Cy together with M and the attached moiety; Cy is a saturated or partially unsaturated or unsaturated 3-10-membered ring system or a saturated or partially unsaturated or unsaturated 3-10-membered ring system substituted by one or more Rp; when there are multiple substituents, the substituents are the same or different;

Rp is independently halogen, cyano, hydroxyl, amino, $C_1$-$C_4$alkyl; or, when two Rp are located on the same C-atom, they jointly form = O;

one or more hydrogen atoms on any of the abovementioned groups can be substituted by Rr substituents selected from the group consisting of: deuterium, halogen, hydroxyl, amino or cycloamino, cyano, nitro, sulfone or sulfoxide, $C_1$-$C_8$alkyl, 3-8-membered cycloalkyl or heterocycloalkyl, $C_1$-$C_8$alkoxy, $C_1$-$C_8$alkylamino, $C_2$-$C_6$alkenyl, $C_2$-$C_6$alkynyl, acyl or sulfonyl, urea or sulfonylurea, 5~8-membered aryl or heteroaryl; or when two Rr are located on the same C-atom, they jointly form = O;

the heteroaryl comprises 1-3 heteroatoms selected from the group consisting of: N, O, P and S;

the heterocycloalkyl comprises 1-3 heteroatoms selected from the group consisting of: N, O, P and S;

the ring system comprises saturated or partially unsaturated ring system such as a spiro ring, a bridged ring, a fused-ring and a fused ring.

**2.** The benzothiazolyl biaryl compound of formula I of claim 1, or a pharmaceutically acceptable salt thereof, or an enantiomer, a diastereoisomer, a tautomer, a torsional isomer, a solvate, a polymorph, or a prodrug thereof, wherein,

when M is N, R is halogen, cyano, amino, $C_1$-$C_6$alkyl, $C_1$-$C_6$alkyl-O-, $C_1$-$C_6$alkylamino, NR'R"-($C_1$-$C_4$alkylene)-O-, NR'R"-CO-($C_1$-$C_4$alkylene)-NR'-, Q1-($C_1$-$C_4$alkylene)-O-, Q2-($C_1$-$C_4$alkylene)-NR'-, NR'R"-Q3-; for example, halogen, NR'R"-($C_1$-$C_4$alkylene)-O-, NR'R"-CO-($C_1$-$C_4$alkylene)-NR'-, Q1-($C_1$-$C_4$alkylene)-O-, Q2-($C_1$-$C_4$alkylene)-NR'-, or NR'R"-Q3-;

and/or, when M is CR5, R is hydrogen, halogen, cyano, amino, $C_1$-$C_6$alkyl, $C_1$-$C_6$alkyl-O-, $C_1$-$C_6$alkylamino, NR'R"-($C_1$-$C_4$alkylene)-O-, NR'R"-CO-($C_1$-$C_4$alkylene)-NR'-, Q4-($C_1$-$C_4$alkylene)-O-, Q5-($C_1$-$C_4$alkylene)-NR'-, NR'R"-Q6-; for example, hydrogen, halogen, NR'R"-($C_1$-$C_4$alkylene)-O-, NR'R"-CO-($C_1$-$C_4$alkylene)-NR'-, Q4-($C_1$-$C_4$alkylene)-O-, Q5-($C_1$-$C_4$alkylene)-NR'-, or NR'R"-Q6-;

and/or, Q1, Q2, Q3, Q4, Q5, and Q6 are independently 3-7-membered heterocycloalkyl substituted by one or more Rq;

and/or, Rq is independently halogen, hydroxyl, or $C_1$-$C_4$alkyl;

and/or, R5 is independently cyano;

and/or, R', R" are independently hydrogen, or $C_1$-$C_6$alkyl;

and/or, R1 is hydrogen, halogen, cyano, or $C_1$-$C_6$alkyl; for example, hydrogen, or halogen;

and/or, R2, R3 are independently hydrogen, $C_1$-$C_6$alkyl, or halogen; For example, hydrogen;

and/or, R4 is halogen; e.g. fluorine;

and/or, m is 0,1 or 2; for example 1;

and/or, W is independently CR6; for example, R6 is halogen;

and/or, W1 is independently CR6; for example, R6 is hydrogen;

and/or, W2 is independently CR6; for example, R6 is halogen;

and/or, R6 is hydrogen, halogen, cyano, $C_1$-$C_6$alkyl, $C_1$-$C_6$alkyl-O-, hydroxyl substituted $C_1$-$C_6$alkyl, cyano substituted $C_1$-$C_6$alkyl, 3-8-membered cycloalkyl, $C_2$-$C_6$alkenyl, $C_2$-$C_6$alkynyl, $C_1$-$C_6$haloalkyl, or $C_1$-$C_6$haloalkyl-O-; for example, hydrogen, halogen, $C_1$-$C_4$alkyl, or $C_1$-$C_4$haloalkyl-O-;

and/or, Ra, Rb, Rc, Rd, Re, Rf, Rg, Rh are independently hydrogen, halogen, $C_1$-$C_6$alkyl, cyano substituted

$C_1$-$C_6$alkyl, hydroxyl substituted $C_1$-$C_6$alkyl; for example, hydrogen, $C_1$-$C_6$alkyl, or cyano substituted $C_1$-$C_6$alkyl; and/or, Cy is a 3-10-membered partially unsaturated ring system or a 3-10-membered partially unsaturated ring system substituted by one or more Rp; for example, a 5-6-membered partially unsaturated ring system substituted by one or more Rp;
and/or, Rp is independently $C_1$-$C_4$alkyl; or, when two Rp are located on the same C, they jointly form = O.

**3.** The benzothiazolyl biaryl compound of formula I of claim 1, or a pharmaceutically acceptable salt thereof, or an enantiomer, a diastereoisomer, a tautomer, a torsional isomer, a solvate, a polymorph, or a prodrug thereof, wherein,

when R is halogen, or $C_1$-$C_6$haloalkyl, the halogen andthe halogen in$C_1$-$C_6$haloalkyl is independently fluorine, chlorine, or bromine; for example, fluorine or chlorine.
and/or, when R is $C_1$-$C_6$alkyl, $C_1$-$C_6$alkyl-S-, $C_1$-$C_6$alkyl-SO-, $C_1$-$C_6$alkyl-$SO_2$-, $C_1$-$C_6$haloalkyl, $C_1$-$C_6$alkyl-O-, $C_1$-$C_6$alkyl-NR'R"-, the $C_1$-$C_6$alkyl in $C_1$-$C_6$alkyl, $C_1$-$C_6$alkyl-S-, $C_1$-$C_6$alkyl-SO-, $C_1$-$C_6$alkyl-$SO_2$-, $C_1$-$C_6$haloalkyl, $C_1$-$C_6$alkyl-O- and$C_1$-$C_6$alkyl-NR'R"- are independently methyl, ethyl, n-propyl, isopropyl, n-butyl, isobutyl, sec-butyl or tert-butyl; for example, methyl;
and/or, when R is Q1-($C_1$-$C_4$alkylene)-O-, Q2-($C_1$-$C_4$alkylene)-NR'-, Q4-($C_1$-$C_4$alkylene)-O-, Q5-($C_1$-$C_4$alkylene)-NR'-, NR'R"-($C_1$-$C_4$alkylene)-O-, NR'R"-CO-($C_1$-$C_4$alkylene)-NR'-, the $C_1$-$C_4$alkylene in Q1-($C_1$-$C_4$alkylene)-O-, Q2-($C_1$-$C_4$alkylene)-NR'-, Q4-($C_1$-$C_4$alkylene)-O-, Q5-($C_1$-$C_4$alkylene)-NR'-, NR'R"-($C_1$-$C_4$alkylene)-O-, NR'R"-CO-($C_1$-$C_4$alkylene)-NR'- are independently-$CH_2$-,-$CH_2CH_2$-,-$CH(CH_3)$-, -$CH(CH_3)CH_2$-, -$C(CH_3)_2$-, -$CH_2CH_2CH_2$-, -$CH_2CH_2CH_2CH_2$-; for example, methylene;
and/or,when Q1, Q2, Q3, Q4, Q5 and Q6 are independently 3-7-membered heterocycloalkyl or 3-7-membered heterocycloalkyl substituted by one or more Rq, the 3-7-membered heterocycloalkyl in the 3-7-membered heterocycloalkyl and3-7-membered heterocycloalkyl substituted by one or more Rq is a 4-6-membered heterocycloalkyl, the heteroatom is selected fromN, O and S, and the number of heteroatoms is 1, 2 or 3; for example, in a4-5-membered heterocycloalkyl, the heteroatom is selected from N, and the number of heteroatoms is 1; for example,

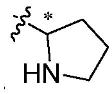 , 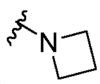 ;

also for example,

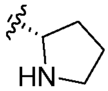 or 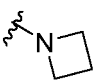 ;

and/or, when Rq is independently halogen, the halogen is independently fluorine, chlorine or bromine; for example fluorine, or chlorine;
and/or,when Rq is independently $C_1$-$C_4$alkyl, the $C_1$-$C_4$alkyl is independently methyl, ethyl, n-propyl, isopropyl, n-butyl, isobutyl, sec-butyl or t-butyl; also for example, methyl;
and/or, when R', R" areindependently $C_1$-$C_6$alkyl, the $C_1$-C alkyl is independently methyl, ethyl, n-propyl, isopropyl, n-butyl, isobutyl, sec-butyl or t-butyl; for example, methyl;
and/or, when R1, R2, R3, R4 and R5 are independently halogen, the halogen is independently fluorine, chlorine or bromine; for example,fluorine, chlorine;
and/or, when R1, R2, R3, R4 and R5 are independently $C_1$-$C_6$alkyl, $C_1$-$C_6$alkyl-$SO_2$-, $C_1$-$C_6$alkyl-SO-, the $C_1$-$C_6$alkyl in the $C_1$-$C_6$alkyl, $C_1$-$C_6$alkyl-$SO_2$-and $C_1$-$C_6$alkyl-SO- are independently $C_1$-$C_4$alkyl, such as methyl, ethyl, n-propyl, isopropyl, n-butyl, isobutyl, isobutyl, sec-butyl or t-butyl; also for example, methyl;
and/or, when R6 is independently $C_1$-$C_6$alkyl, $C_1$-$C_6$alkyl-O-, hydroxyl substituted $C_1$-$C_6$alkyl, cyano substituted $C_1$-$C_6$alkyl, $C_1$-$C_6$haloalkyl, $C_1$-$C_6$haloalkyl-O-, the $C_1$-$C_6$alkyl in the $C_1$-$C_6$alkyl, $C_1$-Calkyl$_6$-O-, hydroxyl substituted $C_1$-$C_6$alkyl, cyano substituted $C_1$-$C_6$alkyl, $C_1$-$C_6$haloalkyl, $C_1$-$C_6$haloalkyl-O- are independently $C_1$-$C_4$alkyl group, such as methyl, ethyl, n-propyl, isopropyl, n-butyl, isobutyl, sec-butyl or tert-butyl; also for example, methyl;
and/or,when R6 is independently halogen, $C_1$-$C_6$haloalkyl, or $C_1$-$C_6$haloalkyl-O-, the halogen and the halogen in$C_1$-$C_6$haloalkyl and Ci-Cehaloalkyl-O- are independently fluorine, chlorine or bromine; for example, fluorine or chlorine;

and/or, when Ra, Rb, Rc, Rd, Re, Rf, Rg and Rh are independently halogen or $C_1$-$C_6$haloalkyl group, the halogen and the halogen in$C_1$-$C_6$haloalkyl are independently fluorine, chlorine or bromine; for example, fluorine or chlorine;
when Ra, Rb, Rc, Rd, Re, Rf, Rg and Rh are independently $C_1$-$C_6$alkyl, $C_1$-$C_6$alkyl-O-, $C_1$-$C_6$haloalkyl or cyano substituted $C_1$-$C_6$alkyl, the $C_1$-$C_6$alkyl in the $C_1$-$C_6$alkyl, $C_1$-$C_6$alkyl-O-, $C_1$-$C_6$haloalkyl and cyano substituted $C_1$-$C_6$alkyl are independently methyl, ethyl, n-propyl, isopropyl, n-butyl, isobutyl, sec-butyl or tert-butyl; for example, methyl;
and/or, when Cy is a3-8-membered saturated or partially unsaturated or unsaturated ring system or a 3-10-membered saturated or partially unsaturated or unsaturated ring system substituted by one or more Rp, the 3-10-membered saturated or partially unsaturated or unsaturated ring system in the 3-10-membered saturated or partially unsaturated or unsaturated ring system or 3-10-membered saturated orpartially unsaturated or unsaturated ring system substituted by one or more Rp of Cy is a 5-10-membered saturated or partially unsaturated or unsaturated heterocyclyl, the heteroatom is selected from N, O and S, and the number of heteroatoms is 1, 2 or 3; for example,

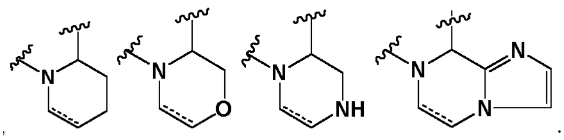

**4.** The benzothiazolyl biaryl compound of formula I of claim 1, or a pharmaceutically acceptable salt thereof, or an enantiomer, a diastereoisomer, a tautomer, a torsional isomer, a solvate, a polymorph, or a prodrug thereof, wherein,

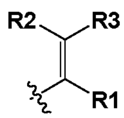

is

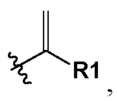,

such as

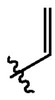 and 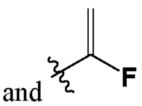.

and/or,

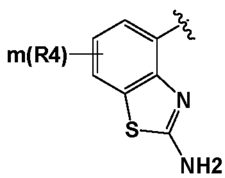

is

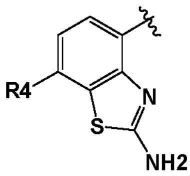
,

such as

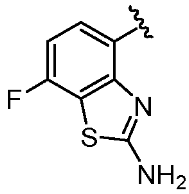
;

and/or,

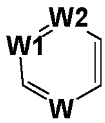
is

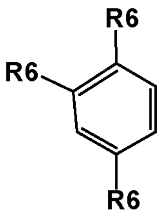
,

such as

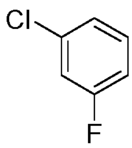
;

and/or,

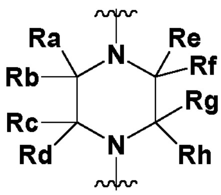

is

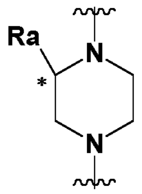, 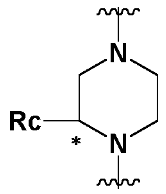,

such as

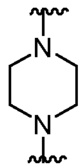, 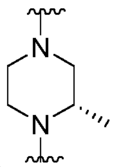, 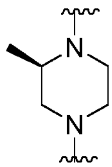, 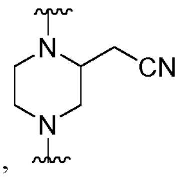;

and/or, R is hydrogen, chlorine,

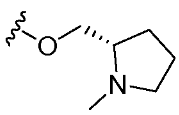 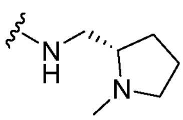 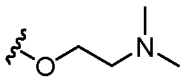 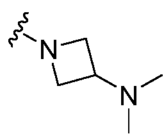

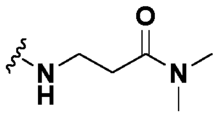 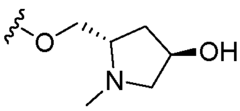 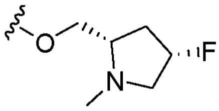 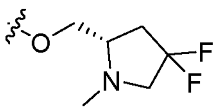 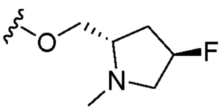;

and/or, Cy is selected from the

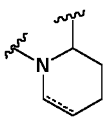 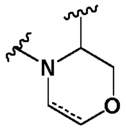 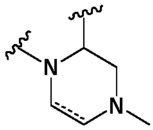 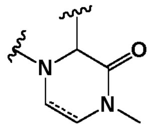 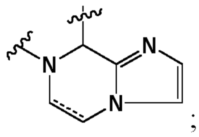;

for example,

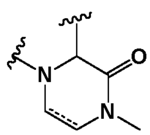.

**5.** The benzothiazolyl biaryl compound of formula I of claim 1, or a pharmaceutically acceptable salt thereof, or an enantiomer, a diastereoisomer, a tautomer, a torsional isomer, a solvate, a polymorph, or a prodrug thereof, wherein, in the formula:

when M is N, R is independently selected from the group consisting of halogen, cyano, hydroxyl, nitro, amino, C1-C6alkyl, C1-$C_6$alkyl-S-, $C_1$-$C_6$alkyl-SO-, $C_1$-$C_6$alkyl-$SO_2$-, $C_1$-$C_6$haloalkyl, $C_1$-$C_6$alkyl-O-, $C_1$-$C_6$alkyl-NR'R"-, 3-12-membered cycloalkyl, 3-12-membered heterocycloalkyl, 3-12-membered cycloalkyl-O-, 3-12-membered cycloalkyl-NR'R"-, 3-12-membered heterocycloalkyl-O-, 3-12-membered heterocycloalkyl-NR'R"-, 5-12-membered aryl and 5-12-membered heteroaryl;
when M is CR5, R is independently selected from the group consisting of hydrogen, halogen, cyano, hydroxyl, nitro, amino, $C_1$-$C_6$alkyl, $C_1$-$C_6$alkyl-S-, $C_1$-$C_6$alkyl-SO-, $C_1$-$C_6$alkyl-$SO_2$-, $C_1$-$C_6$haloalkyl, $C_1$-$C_6$alkyl-O-,

$C_1$-$C_6$alkyl-NR'R"-, 3-12-membered cycloalkyl, 3-12-membered heterocycloalkyl, 3-12-membered cycloalkyl-O-, 3-12-membered cycloalkyl-NR'R"-, 3-12-membered heterocycloalkyl-O-, 3-12-membered heterocycloalkyl-NR'R"-, 5-12-membered aryl and 5-12-membered heteroaryl;

R5 is independently selected from the group consisting of hydrogen, halogen, cyano, hydroxyl, nitro, amino, $C_1$-$C_6$alkyl, $C_1$-$C_6$alkyl-S-, $C_1$-$C_6$alkyl-SO-, $C_1$-$C_6$alkyl-$SO_2$-, $C_1$-$C_6$haloalkyl, $C_1$-$C_6$alkyl-O-, $C_1$-$C_6$alkyl-NR'R"-, 3-12-membered cycloalkyl, 3-12-membered heterocycloalkyl, 3-12-membered cycloalkyl-O-, 3-12-membered cycloalkyl-NR'R"-, 3-12-membered heterocycloalkyl-O-, 3-12-membered heterocycloalkyl-NR'R"-, 5-12-membered aryl and5-12-membered heteroaryl; R', R" are independently selected from hydrogen, $C_1$-$C_6$alkyl, 3-12-membered cycloalkyl, 3-12-membered heterocycloalkyl, and 5-12-membered aryl and heteroaryl;

R1 is independently selected from the group consisting of hydrogen, halogen, cyano, nitro, $C_1$-$C_6$alkyl, $C_1$-$C_6$alkyl-$SO_2$-, $C_1$-$C_6$alkyl-SO-, and$C_1$-$C_6$haloalkyl; R2 and R3 are independently selected from the group consisting of hydrogen, halogen, cyano, nitro, $C_1$-$C_6$alkyl, $C_1$-$C_6$alkyl-$SO_2$-, $C_1$-$C_6$alkyl-SO- and $N(R_{2a})(R_{2b})$-$(CH_2)$x-; or $R_9$ and $R_{10}$together form a 5-10-membered nitrogen-containing heterocycloalkyl substituted by $C_1$-$C_6$alkyl; wherein, $R_{2a}$and $R_{2b}$ are independently selected from hydrogen and $C_1$-$C_6$alkyl, and x is selected from any integer of 0-5;

R4 is independently selected from hydrogen, halogen, cyano, nitro, $C_1$-$C_6$alkyl, $C_1$-$C_6$alkyl-$SO_2$-, $C_1$-$C_6$alkyl-SO-, $C_1$-$C_6$haloalkyl and $C_1$-$C_6$alkoxy, etc., m is an integer independently selected from the integer of 0-4;

W, W1 and W2 are independently selected from CR6 or N, R6 is independently selected from H, halogen, cyano, $C_1$-$C_6$alkyl, $C_1$-$C_6$alkoxy, haloalkyl,haloalkoxy, alkenyl, alkynyl, 3-8-membered cycloalkyl and heterocycloalkyl, etc;

Ra, Rb, Rc, Rd, Re, Rf, Rg and Rh are independently selected from hydrogen, halogen, C1-C6 alkyl, alkoxy and haloalkyl, etc., respectively, orany two of Ra, Rb, Rc, Rd, Re, Rf, Rg and Rh form a saturated or partially unsaturated 3-8-membered ring system; or eitherRg or Rh can form a saturated or partially unsaturated or unsaturated 3-8-membered ring system together with M;

one or more hydrogen atoms on any of the abovementioned groups can be substituted by a substituent selected from the group consisting of: including but not limited to deuterium, halogen, hydroxyl, amino or cycloamino, cyano, nitro, sulfone or sulfoxide, $C_1$-$C_8$alkyl, 3-8-membered cycloalkyl or heterocycloalkyl, $C_1$-$C_8$alkoxy, Ci-Csalkylamino, alkenyl, alkynyl, acyl or sulfonyl, urea or sulfonylurea, 5-8 membered aryl or heteroaryl; wherein the heteroaryl comprises 1-3 heteroatoms selected from the group consisting of N, O, P and S, the heterocycloalkyl group comprises 1-3 heteroatoms selected from the group consisting of N, O, P and S, the ring system comprises saturated or partially unsaturated ring system such as a spiro ring, a bridged ring, a fused-ring and a fused ring.

6. The benzothiazolyl biaryl compound of formula I according to claim 1 or 5, or a pharmaceutically acceptable salt thereof, or an enantiomer, a diastereomer, a tautomer, a torsional isomer, a solvate, a polymorph or a prodrug thereof, wherein the compound of formula I, or a pharmaceutically acceptable salt thereof, or an enantiomer, a diastereoisomer, a tautomer, a torsional isomer, a solvate, a polymorph, or a prodrug thereof, is the compound of formula IIA, IIB or IIC, or a pharmaceutically acceptable salt thereof, or an enantiomer, a diastereoisomer, a tautomer, a torsional isomer, a solvate, a polymorph, or a prodrug thereof:

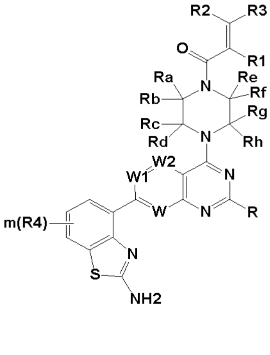
IIA,
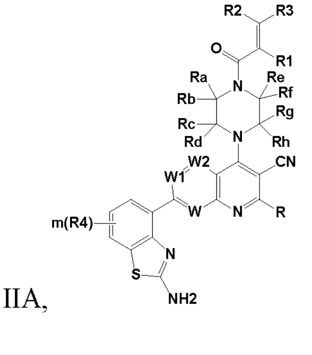
IIB,
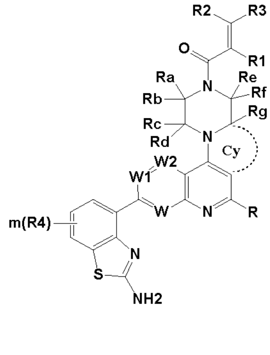
IIC;

wherein, Cy, R, R1, R2, R3, R4, m, Ra, Rb, Rc, Rd, Re, Rf, Rg, W, W1and W2 are as defined in any one of claims 1-4.

7. The benzothiazolyl biaryl compound of formula I of claim 6, or a pharmaceutically acceptable salt thereof, or an enantiomer, a diastereoisomer, a tautomer, a torsional isomer, a solvate, a polymorph, or a prodrug thereof, wherein,

R1 is preferably selected from hydrogen, fluorine, methyl and cyano, etc.; R2 and R3 are independently preferably

selected from hydrogen, methyl and fluorine, etc.; R4 is preferably selected from one or more fluorine; m is preferably selected from 0, 1 and 2;

Ra, Rb, Rc, Rd, Re, Rf, Rg and Rh are independently preferably selected from hydrogen, fluorine, methyl, hydroxymethyl, hydroxyethyl and cyano methyl, etc;

W is preferably selected from N, C-F, C-Cl, C-Me, C-OMe, C-OCH2CHF2, C-OCH2CF3, etc.; W2 is independently preferably selected from N, CH, C- F, C-Cl, C-Me and C-OMe, etc; W1 is independently selected from -CH, -C-halogen, -C-cyano, -C-cyclopropyl, -C-C1-C4alkyl, -C-C1-C4alkoxy, -C-C2-C4alkenyl, -C-C2-C4alkynyl, -C-C1-C4alkoxy, -C-C1-C4haloalkyland -C-C1-C4haloalkoxy, etc; R is preferably selected from halogen, cyano, amino, C1-C6alkyl, $C_1$-$C_6$alkoxy, C1-C6alkylamino, C1-C6alkylaminoalkylene ether group, C1-C6alkylaminoalkylene amino group, 3-10membered cycloalkylalkylene ether group and 3-10 membered heterocycloalkylalkylene ether group, etc.

**8.** The benzothiazolyl biaryl compound of formula I of claim 1, or a pharmaceutically acceptable salt thereof, or an enantiomer, a diastereoisomer, a tautomer, a torsional isomer, a solvate, a polymorph, or a prodrug thereof, wherein the benzothiazolyl biaryl compound of formula I is as the following scheme 1, scheme 2, scheme 3 or scheme 4;

Scheme 1,

when M is N, R is halogen, NR'R"-($C_1$-$C_4$alkylene)-O-, NR'R"-CO-($C_1$-$C_4$alkylene)-NR'-, Q1-($C_1$-$C_4$alkylene)-O-, Q2-($C_1$-$C_4$alkylene)-NR'-, or NR'R"-Q3-;
when M is CR5, R is hydrogen, halogen, NR'R"-($C_1$-$C_4$alkylene)-O-, NR'R"-CO-($C_1$-$C_4$alkylene)-NR'-, Q4-($C_1$-$C_4$alkylene)-O-, Q5-($C_1$-$C_4$alkylene)-NR'-, or NR'R"-Q6-;
Q1, Q2, Q3, Q4, Q5 and Q6 are independently 3-7-membered heterocycloalkyl or 3-7-membered heterocycloalkyl substituted by one or more Rq; when there are multiple substituents, they are the same or different;
Rq is independently halogen, hydroxyl or $C_1$-$C_4$alkyl;
R5 is independently cyano;
R' and R" are independently hydrogen or $C_1$-$C_6$alkyl;
R1 is hydrogen or halogen;
R2 and R3 are independently hydrogen;
R4 is halogen;
m is 0,1 or 2;
W is independently C (halogen);
W1 is independently C (halogen);
W2 is CH;
any one of Ra, Rb, Re and Rf is hydrogen, C1-C6alkyl or cyano-substituted $C_1$-$C_6$alkyl, and the rest of Ra, Rb, Rc, Rd, Re, Rf, Rg and Rh are hydrogen; or, any one of Rc, Rd, Rg and Rh is hydrogen or methyl, and the rest of Ra, Rb, Rc, Rd, Re, Rf, Rg and Rh are hydrogen;
or either Rg orRh may form Cy together with M and the attached group; Cy is a saturated or partially unsaturated or unsaturated 3-10-membered ring system or a saturated or partially unsaturated or unsaturated 3-10-membered ring system substituted by one or more Rp;
Rp is independently $C_1$-$C_4$alkyl; or, when two Rp are located on the same C-atom, they jointly form = O;

Scheme 2,

when M is N, R is halogen, NR'R"-($C_1$-$C_4$alkylene)-O-, NR'R"-CO-($C_1$-$C_4$alkylene)-NR'-, Q1-($C_1$-$C_4$alkylene)-O-, Q2-($C_1$-$C_4$alkylene)-NR'-, or NR'R"-Q3-;
when M is CR5, R is hydrogen, halogen, NR'R"-($C_1$-$C_4$alkylene)-O-, NR'R"-CO-($C_1$-$C_4$alkylene)-NR'-, Q4-($C_1$-$C_4$alkylene)-O-, Q5-($C_1$-$C_4$alkylene)-NR'-, or NR'R"-Q6-;
Q1, Q2, Q3, Q4, Q5 and Q6 are independently 3-7-membered heterocycloalkyl or 3-7-membered heterocycloalkyl substituted by one or more Rq; when there are multiple substituents, they are the same or different;
Rq is independently halogen, hydroxyl or $C_1$-$C_4$alkyl;
R5 is independently cyano;
R' and R" are independently hydrogen or $C_1$-$C_6$alkyl;
R1 is hydrogen or halogen;
R2 and R3 are independently hydrogen;
R4 is halogen;
m is 0,1 or 2;
W is independently C (halogen);

W1 is independently C (halogen);
W2 is CH;
when M is N, any one of Ra, Rb, Re and Rf is hydrogen, C1-C6alkyl or cyano-substituted $C_1$-$C_6$alkyl, and the rest of Ra, Rb, Rc, Rd, Re, Rf, Rg and Rh are hydrogen; or, any one of Re, Rd, Rg and Rh is hydrogen or methyl, and the rest of Ra, Rb, Rc, Rd, Re, Rf, Rg and Rh are hydrogen;
when M is CR5, any one of Ra, Rb, Re and Rf is hydrogen or $C_1$-$C_6$alkyl, and the rest of Ra, Rb, Re, Rd, Re, Rf, Rg and Rh are hydrogen; or any one of Re, Rd, Rg and Rh is hydrogen or methyl, the rest of Ra, Rb, Rc, Rd, Re, Rf, Rg and Rh are hydrogen;
or either Rg or Rh may form Cy together with M and the attached group; Cy is a saturated or partially unsaturated or unsaturated 3-10-membered ring system or a saturated or partially unsaturated or unsaturated 3-10-membered ring system substituted by one or more Rp;
Rp is independently $C_1$-$C_4$alkyl; or, when two Rp are located on the same C-atom, they jointly form = O;

Scheme 3,

when M is N, R is NR'R"-($C_1$-$C_4$alkylene)-O-, Q1-($C_1$-$C_4$alkylene)-O- or NR'R"-Q3-;
when M is CR5, R is NR'R"-($C_1$-$C_4$alkylene)-O-, NR'R"-CO-($C_1$-$C_4$alkylene)-NR'-, Q4-($C_1$-$C_4$alkylene)-O-, Q5-($C_1$-$C_4$alkylene)-NR'-, or NR'R"-Q6-;
Q1, Q3, Q4, Q5 and Q6 are independently 3-7-membered heterocycloalkyl or 3-7-membered heterocycloalkyl substituted by one or more Rq; when there are multiple substituents, they are the same or different;
Rq is independently halogen, hydroxyl or $C_1$-$C_4$alkyl;
R5 is independently cyano;
R' and R" are independently hydrogen or $C_1$-$C_6$alkyl;
When M is N, R1 is hydrogen, when M is CR5, R1 is hydrogen or halogen;
R2 and R3 are independently hydrogen;
R4 is halogen;
m is 0,1 or 2;
W is independently C (halogen);
W1 is independently C (halogen);
W2 is CH;
when M is N, any one of Rc, Rd, Rg and Rh is hydrogen, or methyl, and the rest of Ra, Rb, Rc, Rd, Re, Rf, Rg and Rh are hydrogen;
when M is CR5, any one of Ra, Rb, Re and Rf is hydrogen or $C_1$-$C_6$alkyl, and the rest of Ra, Rb, Rc, Rd, Re, Rf, Rg and Rh are hydrogen; or any one of Rc, Rd, Rg and Rh is hydrogen or methyl, the rest of Ra, Rb, Rc, Rd, Re, Rf, Rg and Rh are hydrogen;

Scheme 4,

when M is N, R is NR'R"-($C_1$-$C_4$alkylene)-O-, Q1-($C_1$-$C_4$alkylene)-O-, Q2-($C_1$-$C_4$alkylene)-NR'-, NR'R"-Q3-;
when M is CR5, R is hydrogen, Q4-($C_1$-$C_4$alkylene)-O-, Q5-($C_1$-$C_4$alkylene)-NR'-, NR'R"-CO-($C_1$-$C_4$alkylene)-NR'-, NR'R"-($C_1$-$C_4$alkylene)-O-, NR'R"-Q6-;
Q1, Q2, Q3, Q4, Q5 and Q6 are independently 3-7-membered heterocycloalkyl or 3-7-membered heterocycloalkyl substituted by one or more Rq; when there are multiple substituents, they are the same or different;
Rq is independently halogen, hydroxyl or $C_1$-$C_4$alkyl;
R5 is independently cyano;
R' and R" are independently hydrogen or $C_1$-$C_6$alkyl;
R1 is hydrogen;
R2 and R3 are independently hydrogen;
R4 is halogen; for example, F;
m is 0,1 or 2; for example, 1;
W is independently C (halogen); For example, C(F);
W1 is independently C (halogen); For example, C(Cl);
W2 is CH;
when M is N, any one of Rc, Rd, Rg and Rh is hydrogen, or methyl, and the rest of Ra, Rb, Rc, Rd, Re, Rf, Rg and Rh are hydrogen;
When M is CR5, Ra, Rb, Rc, Rd, Re, Rf, Rg and Rh are hydrogen.

**9.** The compound of claim 1 or 5, or a pharmaceutically acceptable salt thereof, or an enantiomer, a diastereoisomer,

a tautomer, a torsional isomer, a solvate, a polymorph, or a prodrug thereof, wherein the compound has the following structure:

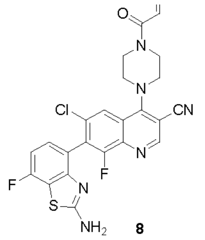 8 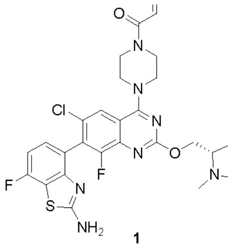 1 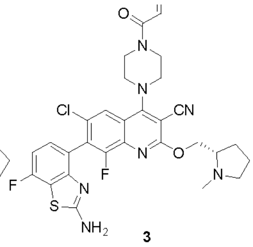 3 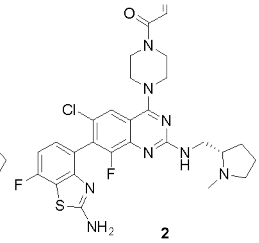 2 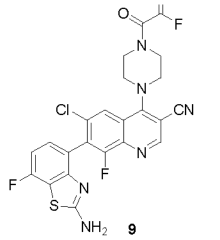 9

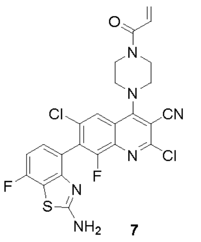 7 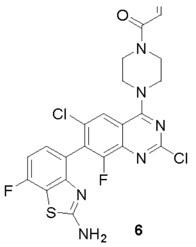 6 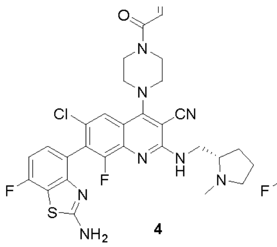 4 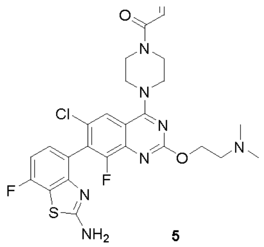 5 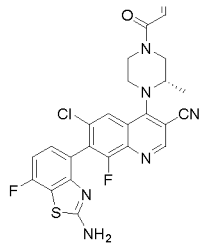

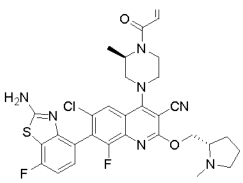 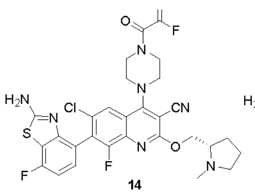 14 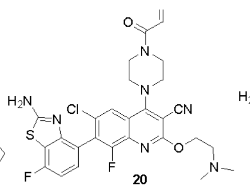 20 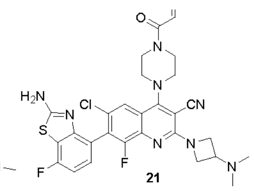 21 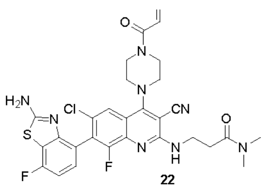 22

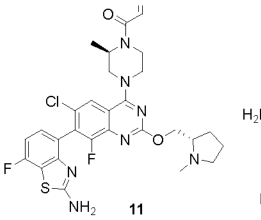 11 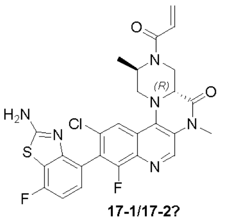 17-1/17-2? 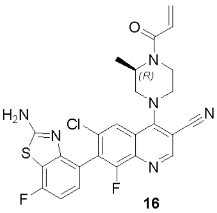 16 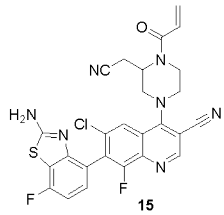 15 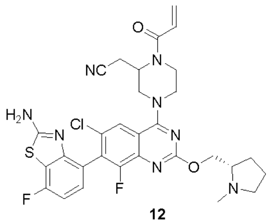 12

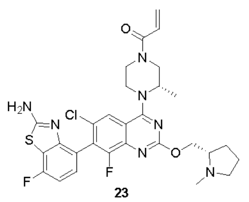 23 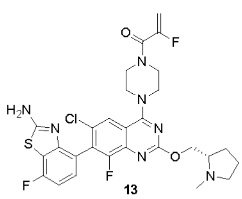 13 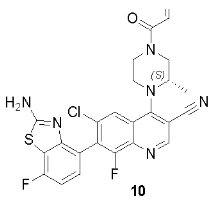 10 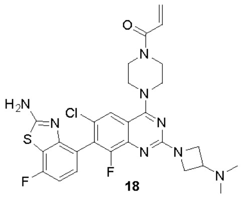 18 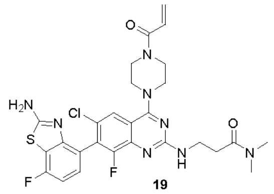 19

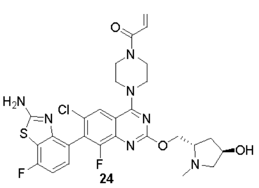 24 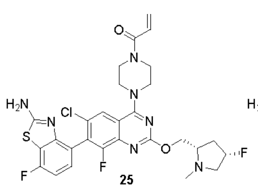 25 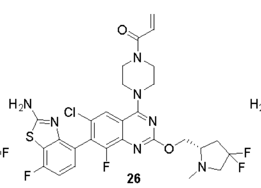 26 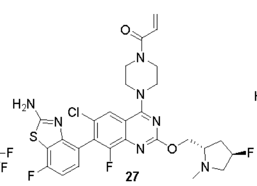 27 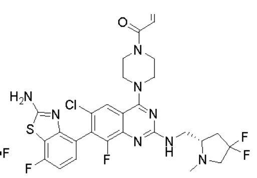

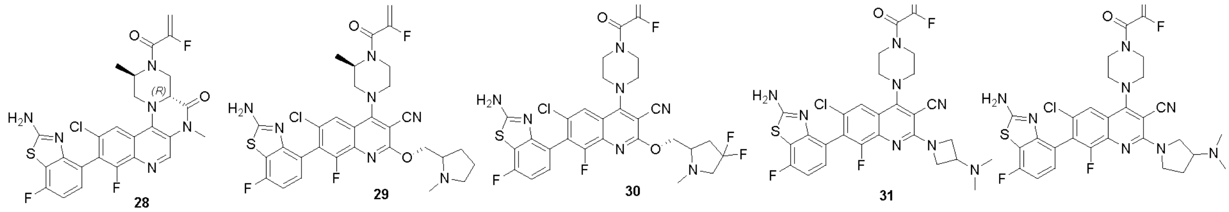

28 29 30 31

.

**10.** The compound according to claim 1 or 5, or a pharmaceutically acceptable salt thereof, or an enantiomer, a diastereoisomer, a tautomer, a torsional isomer, a solvate, a polymorph, or a prodrug thereof, wherein the compound is the following compound:

Theretention time of the compound

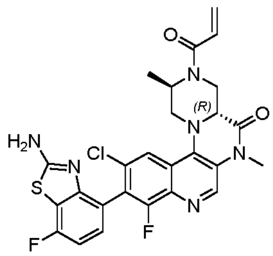

is 8.476min under the following conditions: preparative chromatographic resolution, chromatographic column:Sunfire C18 4.6*150mm, 5uM, elution gradient time: 13min, mobile phase: A:0.1% formic acid/water; B:0.1% formic acid/acetonitrile; flow rate:40 mL/min; wavelength:254nm;
theretention time of the compound

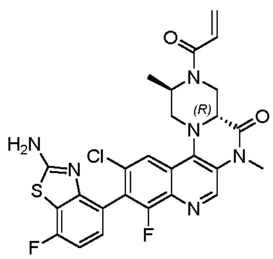

is 8.469 min under the following conditions: preparative chromatographic resolution, chromatographic column:Sunfire C18 4.6*150mm, 5uM, elution gradient time: 13min, mobile phase: A:0.1% formic acid/water; B:0.1% formic acid/acetonitrile; flow rate:40 mL/min; wavelength:254nm.

**11.** A compound of formula A, B or C,

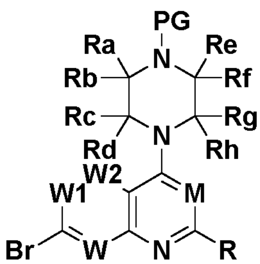

A

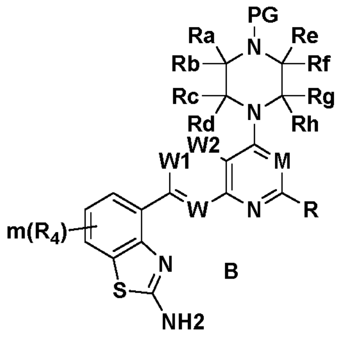

B

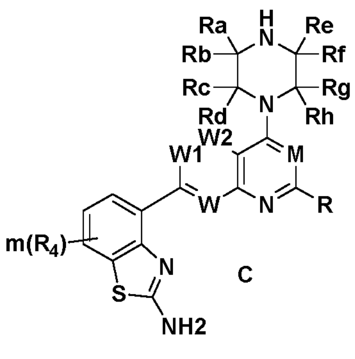

C

wherein, R, R4, m, Ra, Rb, Rc, Rd, Re, Rf, Rg, W, W1, W2 and M are as defined in any one of claims 1-10; for example, the compound of formula A, Band C are selected from the following compound;

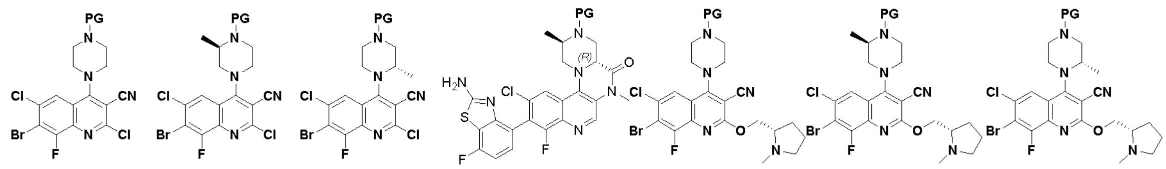

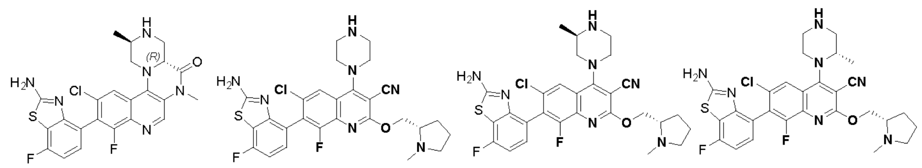

**12.** A pharmaceutical composition comprising an effective amount of thebenzothiazolyl biaryl compound of formula I according to any one of claims 1 to 10, or a pharmaceutically acceptable salt thereof, or an enantiomer, a diastereomer, a tautomer, a torsional isomer, a solvate, a polymorph or a prodrug thereof, and pharmaceutically acceptable carriers.

**13.** The use of the benzothiazolyl biaryl compound of formula I according to any one of claims 1 to 10, or a pharmaceutically acceptable salt thereof, or an enantiomer, a diastereomer, a tautomer, a torsional isomer, a solvate, a polymorph or a prodrug thereof inthe preparation of a Ras mutant protein inhibitor or a drug;

theRas mutant protein can be $KRAS^{G12C}$;
thedrug may be a drug for the treatment ofdiseases related to the activity or expression of the Ras mutant protein; or the drug may be a therapeutic drug for tumors;
the tumor is independently selected from non-small cell lung cancer, small cell lung cancer, lung adenocarcinoma, lung squamous cell carcinoma, breast cancer, prostate cancer, liver cancer, skin cancer, gastric cancer, intestinal cancer, cholangiocarcinoma, brain cancer, leukemia, lymphoma, fibroma, sarcoma, basal cell carcinoma, glioma, kidney cancer, melanoma, bone cancer, thyroid cancer, nasopharyngeal cancer, and pancreatic cancer.

**14.** A method for the prevention and/or treatment of diseases or tumors related to the activity or expression of Ras mutant protein, which comprises administering to apatient a therapeutically effective amountof thebenzothiazolyl biaryl compound of formula Iaccording to any one of claims 1-10, or a pharmaceutically acceptable salt thereof, or an enantiomer, a diastereoisomer, a tautomer, a torsional isomer, a solvate, a polymorph, or a prodrug thereof;
the tumor is independently selected from non-small cell lung cancer, small cell lung cancer, lung adenocarcinoma, lung squamous cell carcinoma, breast cancer, prostate cancer, liver cancer, skin cancer, gastric cancer, intestinal cancer, cholangiocarcinoma, brain cancer, leukemia, lymphoma, fibroma, sarcoma, basal cell carcinoma, glioma, kidney cancer, melanoma, bone cancer, thyroid cancer, nasopharyngeal cancer, and pancreatic cancer.

**15.** A medicament for the prevention and/or treatment of diseases or tumorsassociated withthe activity or expression of Ras mutant protein, comprising the benzothiazolyl biaryl compound of formula I according to any one of claims 1 to 10, or a pharmaceutically acceptable salt thereof, or an enantiomer, a diastereoisomer, a tautomer, a torsional isomer, a solvate, a polymorph, or a prodrug thereof.

**INTERNATIONAL SEARCH REPORT**

International application No.
**PCT/CN2020/127861**

**A. CLASSIFICATION OF SUBJECT MATTER**

C07D 417/14(2006.01)i; C07D 215/12(2006.01)i; C07D 215/02(2006.01)i; C07D 239/72(2006.01)i; C07D 277/62(2006.01)i; A61K 31/517(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D;A61K;A61P

Documentation searched other than minimum documentation to the extent that such documents are included in the fields searched

Electronic data base consulted during the international search (name of data base and, where practicable, search terms used)

WPI, EPODOC, CNPAT, CNKI, REGISTRY (STN), CAPLUS (STN); 凌达生物, 万惠新, 苯并噻唑, 喹啉, 喹唑啉, 哌嗪, KRAS, G12C, 抑制, 肿瘤, 癌, +benzothiazole+, +piperazine, +quinazolin, +quinoline, tumor, cancer, inhibit+

**C. DOCUMENTS CONSIDERED TO BE RELEVANT**

| Category* | Citation of document, with indication, where appropriate, of the relevant passages | Relevant to claim No. |
|---|---|---|
| X | CN 106488910 A (ARAXES PHARMA LLC) 08 March 2017 (2017-03-08) description, pages 7, 8, 40-108, 132, 133 and 138-141, and claims 1 and 4 | 1-9, 11-15 |
| X | TW 201938561 A (ASTRAZENECA AB) 01 October 2019 (2019-10-01) description, paragraphs [0006], [0007], [0088], [0101], [0124], [0126] and [0270] | 1-8, 10-15 |
| A | WO 2020081282 A1 (ELI LILLY AND COMPANY) 23 April 2020 (2020-04-23) description, pages 2, 3, 7 and 9 | 1-15 |
| A | CN 110869358 A (ARAXES PHARMA LLC) 06 March 2020 (2020-03-06) claims 1-45 | 1-15 |
| A | CN 110036010 A (ARAXES PHARMA LLC) 19 July 2019 (2019-07-19) claims 1-63 | 1-15 |
| PA | WO 2020146613 A1 (MIRATI THERAPEUTICS INC. et al.) 16 July 2020 (2020-07-16) description, pages 2-448 | 1-15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 January 2021** | **10 February 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/127861**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **14**
because they relate to subject matter not required to be searched by this Authority, namely:

[1] The subject matter of claim 14 is a method for the treatment of a disease as described in PCT Rule 39.1(iv). This search report is made on the basis of the use of the said compounds and related products in drugs for the treatment of related diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/127861**

| Patent document cited in search report | | | Publication date (day/month/year) | Patent family member(s) | | | Publication date (day/month/year) |
|---|---|---|---|---|---|---|---|
| CN | 106488910 | A | 08 March 2017 | SG | 11201602662Y | A | 30 May 2016 |
| | | | | EP | 3636639 | A1 | 15 April 2020 |
| | | | | AU | 2014331794 | B2 | 04 April 2019 |
| | | | | MX | 2016004360 | A | 19 August 2016 |
| | | | | WO | 2015054572 | A1 | 16 April 2015 |
| | | | | ZA | 201602245 | B | 25 September 2019 |
| | | | | JP | 6559123 | B2 | 14 August 2019 |
| | | | | NO | 20160646 | A1 | 19 April 2016 |
| | | | | EP | 3055290 | B1 | 02 October 2019 |
| | | | | PH | 12016500538 | B1 | 13 June 2016 |
| | | | | EA | 033689 | B1 | 18 November 2019 |
| | | | | CN | 106488910 | B | 31 July 2020 |
| | | | | AU | 2014331794 | A1 | 21 April 2016 |
| | | | | EA | 201690752 | A1 | 29 July 2016 |
| | | | | NI | 201600049 | A | 20 May 2016 |
| | | | | JP | 2016532656 | A | 20 October 2016 |
| | | | | AU | 2014331794 | C1 | 12 September 2019 |
| | | | | CA | 2926328 | A1 | 16 April 2015 |
| | | | | BR | 112016008016 | A2 | 12 September 2017 |
| | | | | UA | 119971 | C2 | 10 September 2019 |
| | | | | PH | 12016500538 | A1 | 13 June 2016 |
| | | | | EA | 033689 | B9 | 29 April 2020 |
| | | | | IL | 244699 | D0 | 21 April 2016 |
| | | | | KR | 20160076519 | A | 30 June 2016 |
| | | | | EP | 3055290 | A1 | 17 August 2016 |
| TW | 201938561 | A | 01 October 2019 | WO | 2019110751 | A1 | 13 June 2019 |
| | | | | US | 10597405 | B2 | 24 March 2020 |
| | | | | US | 2019177338 | A1 | 13 June 2019 |
| | | | | UY | 38001 | A | 31 May 2019 |
| WO | 2020081282 | A1 | 23 April 2020 | US | 2020115375 | A1 | 16 April 2020 |
| CN | 110869358 | A | 06 March 2020 | CA | 3063440 | A1 | 29 November 2018 |
| | | | | BR | 112019024674 | A2 | 16 June 2020 |
| | | | | EP | 3630745 | A2 | 08 April 2020 |
| | | | | US | 10745385 | B2 | 18 August 2020 |
| | | | | TW | 201900633 | A | 01 January 2019 |
| | | | | AU | 2018271990 | A1 | 12 December 2019 |
| | | | | WO | 2018218070 | A3 | 07 February 2019 |
| | | | | KR | 20200010306 | A | 30 January 2020 |
| | | | | WO | 2018218070 | A2 | 29 November 2018 |
| | | | | US | 2019062313 | A1 | 28 February 2019 |
| | | | | JP | 2020521742 | A | 27 July 2020 |
| CN | 110036010 | A | 19 July 2019 | US | 2018155348 | A1 | 07 June 2018 |
| | | | | US | 2020062761 | A1 | 27 February 2020 |
| | | | | US | 10723738 | B2 | 28 July 2020 |
| | | | | US | 10280172 | B2 | 07 May 2019 |
| | | | | EP | 3519402 | A1 | 07 August 2019 |
| | | | | JP | 2019529484 | A | 17 October 2019 |
| | | | | WO | 2018064510 | A1 | 05 April 2018 |
| WO | 2020146613 | A1 | 16 July 2020 | US | 2020331911 | A1 | 22 October 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010358615
- WO 2019110751 A1
- WO 2019014402 A1
- WO 201367597 A1
- WO 2020081282 A


### Non-patent literature cited in the description


- Hand book of Pharmaceutical Additives. Synapse Information Resources, Inc, 2002
- Remington's Pharmaceutical Sciences. Mack Publishing Company
- **CAREY ; SUNDBERG.** ADVANCED ORGANIC CHEMISTRY. Plenum Press, 2000, vol. A,B
- Chiral Separations, Methods and Protocols, Methods in Molecular Biology. 2004, vol. 243
- **A.M. STALCUP.** *Chiral Separations, annu. Rev. Anal. Chem.,* 2010, vol. 3, 341-63
- VOGEL'S ENCYCLOPEDIA OF PRACTICAL ORGANIC CHEMISTRY. Longman Scientific and Technical Ltd, 1991, 809-816
- *Heller, Acc. Chem. Res.,* 1990, vol. 23, 128
- *bioorg. med. chem. lett.,* 1994, vol. 4, 1985-1990
- **GREENWALD, R. B. et al.** *j. med. chem.,* 2000, vol. 43, 475
- Goodman and Gilman, The Pharmacological Basis of Therapeutic. Pergamon
- Remington's, Pharmaceutical Sciences. Mack Publishing Co,
- **GREENE, T. W ; P. G. M. WUTS.** Protective Groups in Organic Synthesis. Wiley, 1999